# EUROPEAN PATENT SPECIFICATION

(11) **EP 3 016 660 B1**
(45) Date of publication and mention of the grant of the patent: **10.06.2020**
(21) Application number: 14820357.3
(22) Date of filing: 01.07.2014
(51) Int. Cl.: A61K 31/565, A61K 31/56, A61P 3/04, A61P 3/00

(54) **SHIP INHIBITION TO COMBAT OBESITY**
SHIP-HEMMUNG GEGEN FETTLEIBIGKEIT
INHIBITION DE SHIP DANS LE CADRE DE LA LUTTE CONTRE L'OBÉSITÉ

(30) Priority: 01.07.2013 US 201361841738 P; 14.02.2014 US 201461940368 P; 17.06.2014 US 201462013361 P
(43) Date of publication of application: 11.05.2016
(60) Divisional application: 20167369.6
(73) Proprietor: The Research Foundation for the State University of New York, Syracuse, NY 13210 (US)
(72) Inventor: KERR, William G., Syracuse, NY 13210 (US); IYER, Sonia, Lexington, MA 02421 (US)
(74) Representative: Murgitroyd & Company
(86) International application number: PCT/US2014/045146
(87) International publication number: WO 2015/003003

(56) References cited:
- EP-A2- 0 133 995
- WO-A1-2008/068037
- WO-A2-2004/032880
- WO-A2-2009/032321
- WO-A2-2011/127465
- US-A- 5 710 143
- US-A1- 2004 205 831
- US-A1- 2008 255 076
- US-A1- 2012 178 725
- MATTHEW D BLUNT ET AL: "Targeting PI3K isoforms and SHIP in the immune system: new therapeutics for inflammation and leukemia", CURRENT OPINION IN PHARMACOLOGY, vol. 12, no. 4, 1 August 2012 (2012-08-01) , pages 444-451, XP55095312, ISSN: 1471-4892, DOI: 10.1016/j.coph.2012.02.015
- DIANE MATHIS: "Immunological Goings-on in Visceral Adipose Tissue", CELL METABOLISM, vol. 17, no. 6, 1 June 2013 (2013-06-01), pages 851-859, XP55331435, United States ISSN: 1550-4131, DOI: 10.1016/j.cmet.2013.05.008
- NEETU SRIVASTAVA ET AL: "A small-molecule inhibitor of SHIP1 reverses age- and diet-associated obesity and metabolic syndrome", JCI INSIGHT, vol. 1, no. 11, 21 July 2016 (2016-07-21), XP55331368, DOI: 10.1172/jci.insight.88544
- BROOKS, ROBERT ET AL.: 'SHIP1 inhibition increases immunoregulatory capacity and triggers apoptosis of hematopoietic cancer cells' THE JOURNAL OF IMMUNOLOGY vol. 184, no. 7, 2010, pages 3582 - 3589, XP055305470
- A Suwa ET AL: "Discovery and functional characterization of a novel small molecule inhibitor of the intracellular phosphatase, SHIP2", British Journal of Pharmacology, vol. 158, no. 3, 19 October 2009 (2009-10-19), pages 879-887, XP55093528, ISSN: 0007-1188, DOI: 10.1111/j.1476-5381.2009.00358.x

## Description

### CROSS-REFERENCE TO RELATED APPLICATIONS

### FIELD OF THE INVENTION

The present invention relates to the use SHIP inhibitors in various methods. More particularly, the present invention relates to the use of pan-SHIP1/2 inhibitors in various methods, including: (i) a method to treat obesity or reduce body fat of an obese subject; and (ii) a method to reduce glucose intolerance or insulin resistance.

### BACKGROUND OF THE INVENTION

Bone undergoes continuous remodeling in the body throughout life. This dynamic process involves a balance between bone-forming osteoblasts derived from multipotent mesenchymal stem cells (MSC) and osteoclasts that mediate bone resorption (Hadjidakis, D.J., and Androulakis, I. (2006). Bone remodeling. Ann N Y Acad Sci 1092, 385-396). Differentiation of uncommitted mesenchymal stem cells (MSC) into multiple lineages is tightly regulated by several transcriptional and regulatory factors. However, a detailed and integrated understanding of this process remains to be defined. In addition, underlying mechanisms that maintain MSC in an uncommitted state are poorly understood. Basic-helix-loop-helix (bHLH) transcription factors perform a wide array of cellular functions including stem cell commitment (Massari, M.E., and Murre, C. (2000). Helix-loop-helix proteins: regulators of transcription in eucaryotic organisms. Mol Cell Biol 20, 429-440).

Inhibitors of DNA binding-proteins or inhibitors of differentiation (Ids) belonging to the HLH family of proteins are nuclear factors that lack the basic amino acid region required for DNA-binding (Benezra, R., Davis, R.L., Lockshon, D., Turner, D.L., and Weintraub, H. (1990). The protein Id: a negative regulator of helix-loop-helix DNA binding proteins. Cell 61, 49-59; O'Toole, P.J., Inoue, T., Emerson, L., Morrison, I.E., Mackie, A.R., Cherry, R.J., and Norton, J.D. (2003), Id proteins negatively regulate basic helix-loop-helix transcription factor function by disrupting subnuclear compartmentalization. J Biol Chem 278, 45770-45776). Id proteins heterodimerize and sequester bHLH factors, and render them incapable of binding DNA of target genes, thereby, regulating their functions (Jogi, A., Persson, P., Grynfeld, A., Pahlman, S., and Axelson, H. (2002). Modulation of basic helix-loop-helix transcription complex formation by Id proteins during neuronal differentiation. J Biol Chem 277, 9118-9126; Lasorella, A., Uo, T., and Iavarone, A. (2001). Id proteins at the cross-road of development and cancer. Oncogene 20, 8326-8333; and Zebedee, Z., and Hara, E. (2001). Id proteins in cell cycle control and cellular senescence. Oncogene 20, 8317-8325). The function of Id proteins in maintaining sternness has recently been shown to require the deubiquitinase USP1 by preventing proteasomal degradation of Id2 (Williams, S.A., Maecker, H.L., French, D.M., Liu, J., Gregg, A., Silverstein, L.B., Cao, T.C., Carano, R.A., and Dixit, V.M. (2011). USP1 deubiquitinates ID proteins to preserve a mesenchymal stem cell program in osteosarcoma. Cell 146, 918-930).

The Src homology 2-domain-containing inositol 5' -phosphatase 1 (SHIP1) regulates cellular processes such as proliferation, differentiation and survival via the PI3K/AKT pathway at the plasma membrane. SHIP hydrolyzes the product of PI3K, PI(3,4,5)P3 to generate PI(3,4)P2, which, like PI(3,4,5)P3, can facilitate downstream activation of Akt (Brooks, R., Fuhler, G.M., Iyer, S., Smith, M.J., Park, M.Y., Paraiso, K.H., Engelman, R.W., and Kerr, W.G. (2010). SHIP1 inhibition increases immunoregulatory capacity and triggers apoptosis of hematopoietic cancer cells. J Immunol 184, 3582-3589; Franke, T.F., Kaplan, D.R., Cantley, L.C., and Toker, A. (1997). Direct regulation of the Akt proto-oncogene product by phosphatidylinositol-3,4-bisphosphate [see comments]. Science 275, 665-668; Fuhler, G.M., Brooks, R., Toms, B., Iyer, S., Gengo, E.A., Park, M.Y., Gumbleton, M., Viernes, D.R., Chisholm, J.D., and Kerr, W.G. (2012). Therapeutic potential of SH2 domain-containing inositol-5'-phosphatase 1 (SHIP1) and SHIP2 inhibition in cancer. Mol Med 18, 65-75; and Ma, K., Cheung, S.M., Marshall, A.J., and Duronio, V. (2008). PI(3,4,5)P3 and PI(3,4)P2 levels correlate with PKB/akt phosphorylation at Thr308 and Ser473, respectively; PI(3,4)P2 levels determine PKB activity. Cell Signal 20, 684-694).)

Previously it has been found that HSC from SHIP deficient mice demonstrate defective repopulating and self-renewal capacity upon transfer to SHIP-competent hosts (Desponts, C., Hazen, A.L., Paraiso, K.H., and Kerr, W.G. (2006). SHIP deficiency enhances HSC proliferation and survival but compromises homing and repopulation. Blood 107, 4338-4345; Helgason, C.D., Antonchuk, J., Bodner, C., and Humphries, R.K. (2003). Homeostasis and regeneration of the hematopoietic stem cell pool is altered in SHIP-deficient mice. Blood).

These findings suggested an intrinsic defect in HSC caused by SHIP deficiency. However, this defect was not observed when HSC were rendered SHIP-deficient in adult hosts where the BM milieu or niche remained SHIPcompetent. These findings suggested that defective HSC function in germline SHIP-deficient hosts might arise from disruption of niche cell components (Hazen, A.L., Smith, M.J., Desponts, C., Winter, 0., Moser, K., and Kerr, W.G. (2009). SHIP is required for a functional hematopoietic stem cell niche. Blood 113, 2924-2933). Consistent with this hypothesis, primary osteoblasts (OB) were found to express the SH2 domain containing 145 and 150kD isoforms encoded by the SHIP1 locus (Hazen et al., 2009). In addition,
bone marrow derived SHIP1-/- osteoblasts have less alkaline phosphatase (ALP) activity required for bone formation indicating impaired OB development and function (Hazen et al., 2009). SHIP-/mutant mice have previously been reported to be osteoporotic (Takeshita, S., Namba, N., Zhao, J.J., Jiang, Y., Genant, H.K., Silva, M.J., Brodt, M.D., Helgason, C.D., Kalesnikoff, J., Rauh, M.J., et al. (2002). SHIP-deficient mice are severely osteoporotic due to increased numbers of hyper-resorptive osteoclasts. Nat Med 8, 943-949). This pathology was attributed to hyper-resorptive activity by myeloid-derived osteoclasts (OC) (Takeshita et al., 2002) resulting from unopposed PI3K signaling at DAP12-associated receptors (Peng, Q., Malhotra, S., Torchia, J.A., Kerr, W.G., Coggeshall, K.M., and Humphrey, M.B. (2010). TREM2- and DAP12-dependent activation of PI3K requires DAP10 and is inhibited by SHIP1. Sci Signal 3, ra38).

There is a need to develop new methods of treating or preventing diseases or conditions such as obesity, diabetes, and high cholesterol. There is also a need for limiting bone development in a subject suffering from an osteopetrotic or sclerotic disease.

The present invention is directed to overcoming these and other deficiencies in the art.

### SUMMARY OF THE INVENTION

The present invention relates to the use SHIP inhibitors in various methods. More particularly, the present invention relates to the use of pan-SHIP1/2 inhibitors in various methods, as described herein.

In one aspect, the present invention relates to a pan-SHIP1/2 inhibitor for use in a method to treat obesity or to reduce body fat in an obese subject,
wherein the pan-SHIP1/2 inhibitor has the structure of: 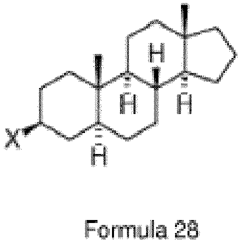 or is a
pharmaceutically acceptable salt thereof,
wherein X = NR₂, NRCONR₂, OR, SR, OCOR, OCONR₂, or NHCNHNH₂, preferably X = NH₂ or NH₃Cl, and wherein R = H, alkyl, cycloalkyl, aryl or benzyl, and
wherein the inhibitor reduces body fat in the obese subject.

In another aspect, the present invention relates to a pan-SHIP1/2 inhibitor for use in treatment to reduce glucose intolerance or to decrease insulin resistance in a subject,
wherein the pan-SHIP1/2 inhibitor has the structure of: 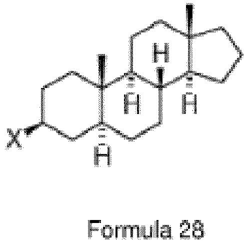 or is a
pharmaceutically acceptable salt thereof,
wherein X = NR₂, NRCONR₂, OR, SR, OCOR, OCONR₂, or NHCNHNH₂, preferably X = NH₂ or NH₃Cl, and wherein R = H, alkyl, cycloalkyl, aryl or benzyl, and
wherein the inhibitor reduces glucose intolerance or decreases insulin resistance when administered to the subject.

Described herein is a method to treat or prevent
diabetes in a subject. This method involves administering a SHIP1 inhibitor or a pan-SHIP1/2 inhibitor to a subject in an amount effective to treat or prevent diabetes in the subject.

Also described herein is a method to reduce glucose intolerance or insulin resistance in a subject. This method involves administering a SHIP1 inhibitor or a pan-SHIP1/2 inhibitor to a subject in an amount effective to reduce glucose intolerance or insulin resistance in the subject.

Also described herein is a method to lower cholesterol in a subject. This method involves administering a SHIP1 inhibitor or a pan-SHIP1/2 inhibitor to a subject in an amount effective to lower cholesterol in the subject.

### BRIEF DESCRIPTION OF THE DRAWINGS

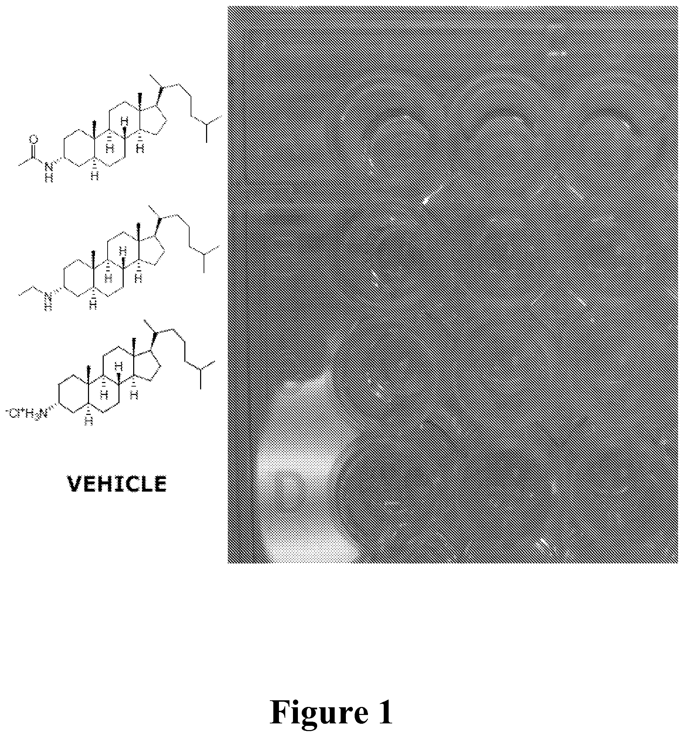

Figure 1 shows the results of a SHIP1 malachite phosphatase assay. Lack of green color indicates inhibition for the amide and the hydrochloride, while the ethylamine is a poor inhibitor

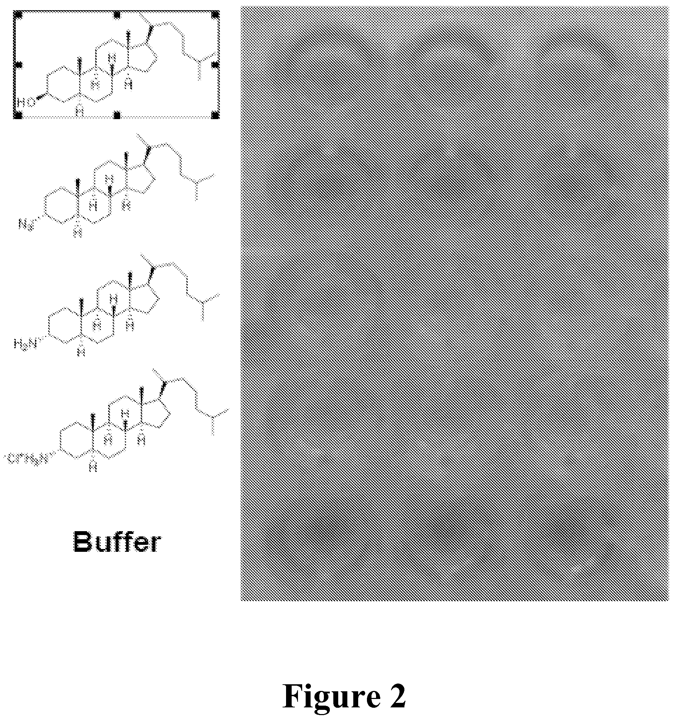

Figure 2 shows the results of a SHIP1 malachite phosphatase assay. Lack of green color indicates inhibition.

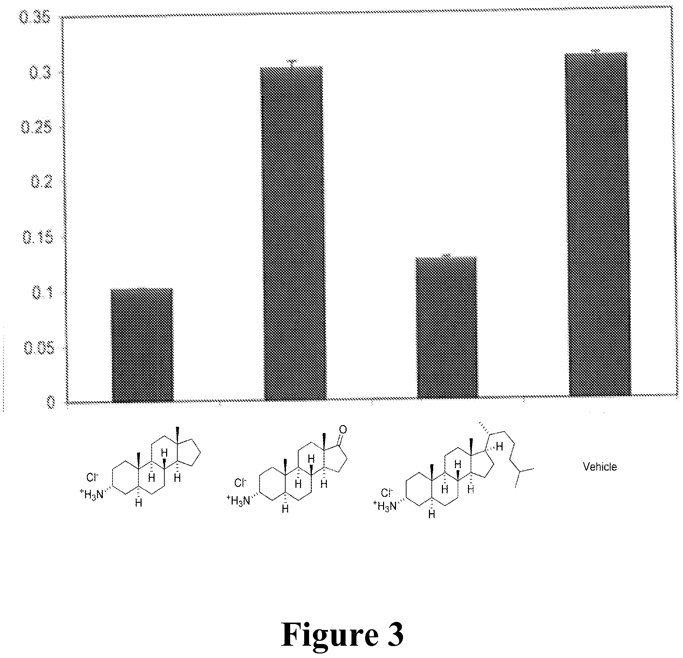

Figure 3 shows the results of a SHIP1 malachite phosphatase assay using a UV-Vis plate reader. Absorbance is recorded at 620nm, smaller numbers indicate a lack of green color and inhibition of the phosphatase.

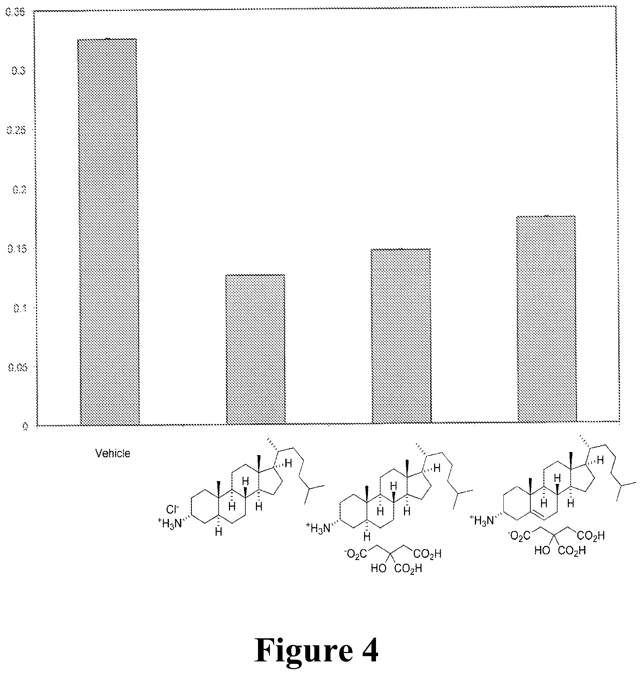

Figure 4 shows the results of a SHIP1 malachite phosphatase assay using a UV-Vis plate reader. Absorbance is recorded at 620nm, smaller numbers indicate a lack of green color and inhibition of the phosphatase.

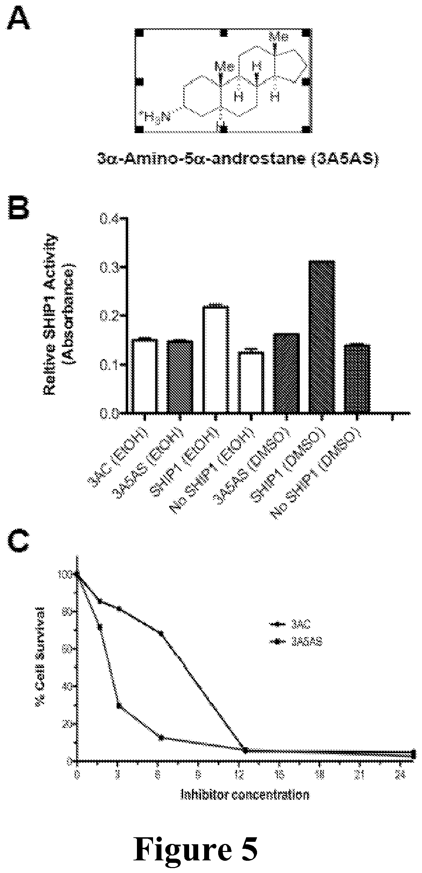

Figures 5A-5C illustrate results of potency studies regarding 3α-amino-5α-androstane (3A5AS). The 3AC derivative, 3A5AS (FIG. 5A), is equally potent for inhibition of recombinant SHIP1 activity *in vitro* as measured by a Malachite Green assay *(see* FIG 5B). However, 3A5AS is more potent for killing of blood cancer cells (C1498 leukemia cells) than the parent compound 3AC as measured in the MTT assay for cell viability *(see* FIG. 5C).

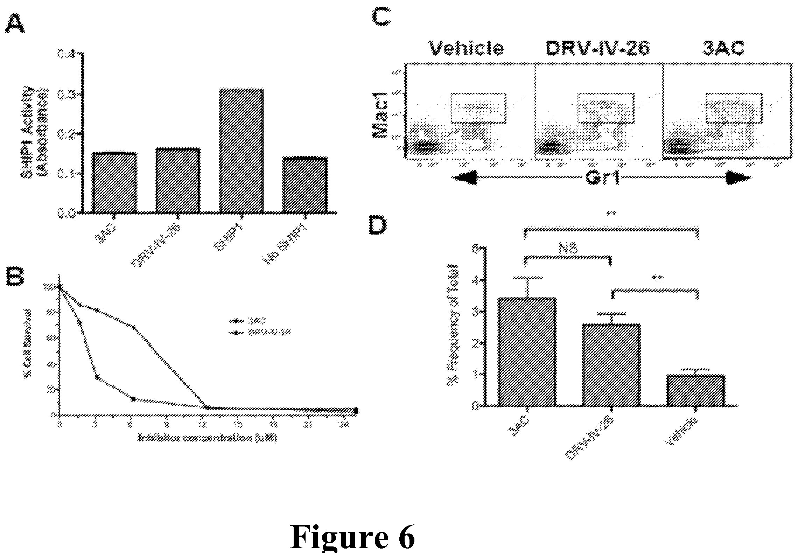

Figures 6A-6D illustrate results relating to SHIP1 inhibitory activity of a 3AC derivative. As shown by the results, a more soluble 3AC derivative retains SHIP1 inhibitory activity *in vitro* and *in vivo.* FIG. 6A: Malachite Green assay for SHIP1 activity against PI(3,4,5)P₃ substrate in the presence of 3AC or 3A5AS. The "No SHIP" column indicates background absorbance when the assay is carried out with PIP3 substrate in the absence of recombinant SHIP1. FIG. 6B: MTT assay of C1498 leukemia growth in the presence of 3AC and 3A5AS at the indicated concentrations. FIG. 6C: Examples of MIR cell induction in 3AC- and 3A5AS-treated mice as compared to a vehicle control. FIG. 6D: Bar graphs and statistical analysis of MIR cell numbers in the indicated treatment groups (**, p<0.01; NS, not significant). *[Noto bene:* DRV-IV-26 = 3A5AS].

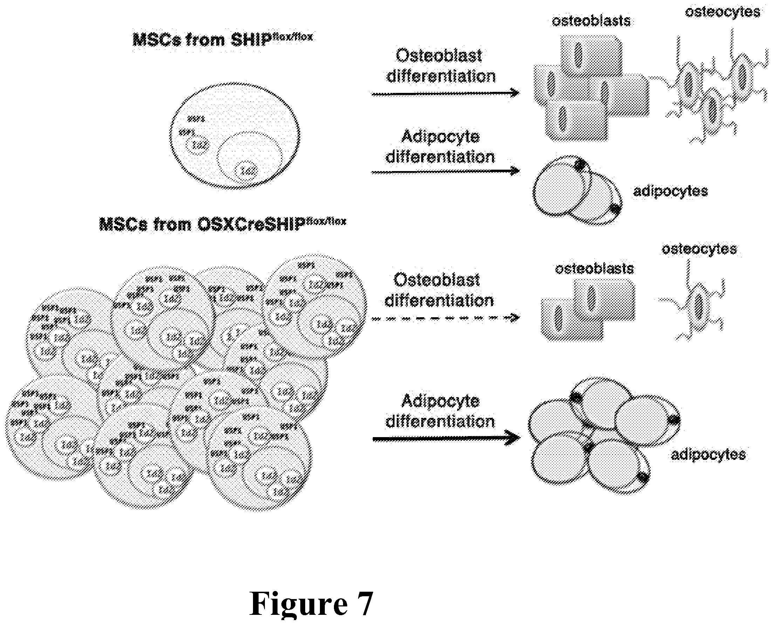

Figure 7 is a schematic summarizing how SHIP1 limits MSC proliferation and promotes osteolineage commitment by repressing the USP1/ID2 axis.

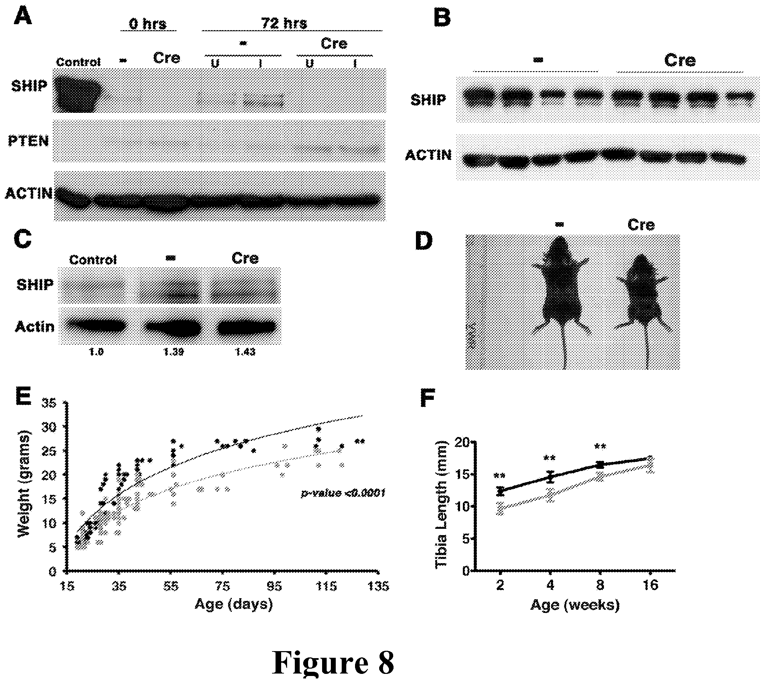

Figures 8A-8F: Osteoblast-specific ablation of SHIP1 expression retards growth and limb length. (A) BM derived MSC were cultured in conditions that induce osteoblast differentiation. SHIP1 and PTEN expression in OB cell lysates was assessed by immunoblotting at days 0 and 3. Actin serves as a loading control. Lanes containing SHIP^{flox/flox} (-) or OSXCreSHIP^{flox/flox} (Cre) cell lysates are as indicated. (B) SHIP1 levels in mononuclear cell lysates from blood were assessed by immunoblotting from 4 independent SHIP^{flox/flox} (-) and OSXCreSHIP^{flox/flox} (Cre) mice. (C) Macrophage progenitors in bone marrow were cultured with RANKL and M-CSF and SHIP1 levels in whole cell lysates (WCL) were assessed by immunoblotting at day 3 of differentiation. Actin quantification was performed using Image lab software 3.0.1 Beta 2, Bio-Rad Laboratories (Hercules, CA) (D) Representative image of 3 week old male SHIP^{flox/flox} (-) and OSXCreSHIP^{flox/flox} (Cre) (E) Weights of male SHIP^{flox/flox} (*black circles*) and OSXCreSHIP^{flox/flox} (*grey circles*) mice, each symbol represents an individual mouse, between 19 and 129 days post-partum, data analysis was performed using analysis of covariance (ANCOVA) followed by Bonferroni's multiple comparison post-hoc test with age or gender to evaluate the differences between groups, *** *P<* 0.0001. (F) Male tibia limb length at 2, 4, 8 and 16 weeks were measured for SHIP^{flox/flox} (*black circles*) and OSXCreSHIP^{flox/flox} (*grey square*), (*n*=5), ± SD. ***P*≤ 0.001 (Student's unpaired, two-tailed *t* test). NB: Significant differences in both weight and tibial length were also observed for female SHIP^{flox/flox} and OSXCreSHIP^{flox/flox} littermates.

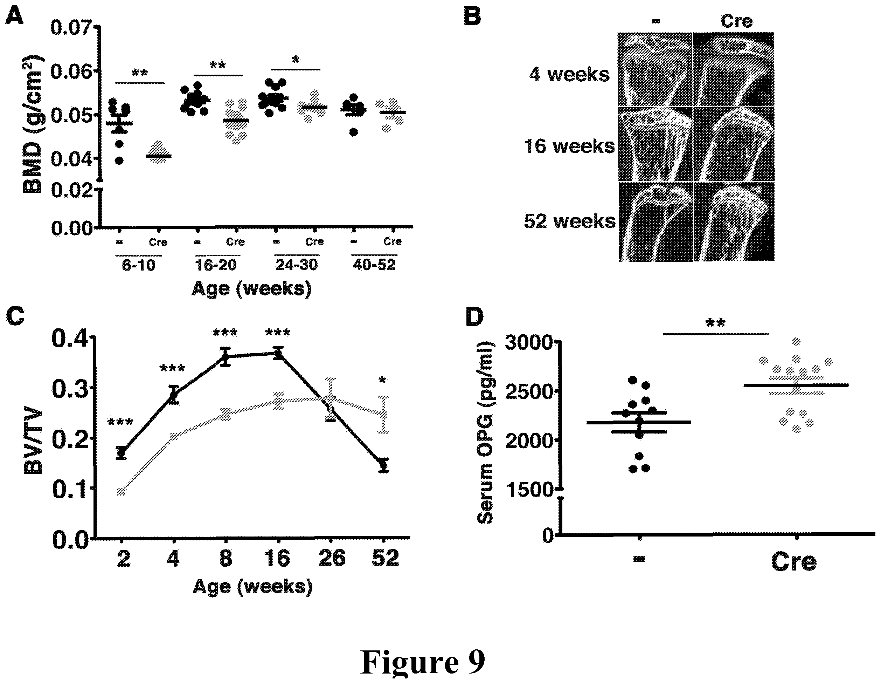

Figures 9A-9D: A SHIP-deficient osteoblast compartment causes osteoporosis. (A) Whole-body BMD by DEXA analysis of 6 to 52 week old SHIP^{flox/flox} (-) (*black circles)* and OSXCreSHIP^{flox/flox} (Cre) *(grey circles)* mice; [*(n≥5)*, *± SD. **P*≤ *0.001 and *P*≤ *0.01 Student's unpaired, two-tailed t test*]*.* (B) Sagittal sections through the proximal metaphysis taken from representative microCT scans of 4- 16- and 52-weeks SHIP^{flox/flox} (-) and OSXCreSHIP^{flox/flox} (Cre) mice. (C) Metaphyseal histomorphometric parameters, bone volume over tissue volume (BV/TV), measured at 2, 4, 8, 16, 26 and 52 weeks in SHIP^{flox/flox} (*black circles*) and OSXCreSHIP^{flox/flox} (*grey squares*) mice. [± *SD. *P*≤ *0.01, ***P*≤ *0.0001, Student's unpaired, two-tailed t test*]*.* (D) Serum OPG measured by ELISA was increased between 40 to 52 weeks OSXCreSHIP^{flox/flox} (Cre) *(grey circles)* in comparison to SHIP^{flox/flox}(-) (*black circles)* mice. [*n≥5 mice,* ± SD. ***P*≤ 0.001, Student's unpaired, two-tailed *t* test] [each symbol represents an individual mouse].

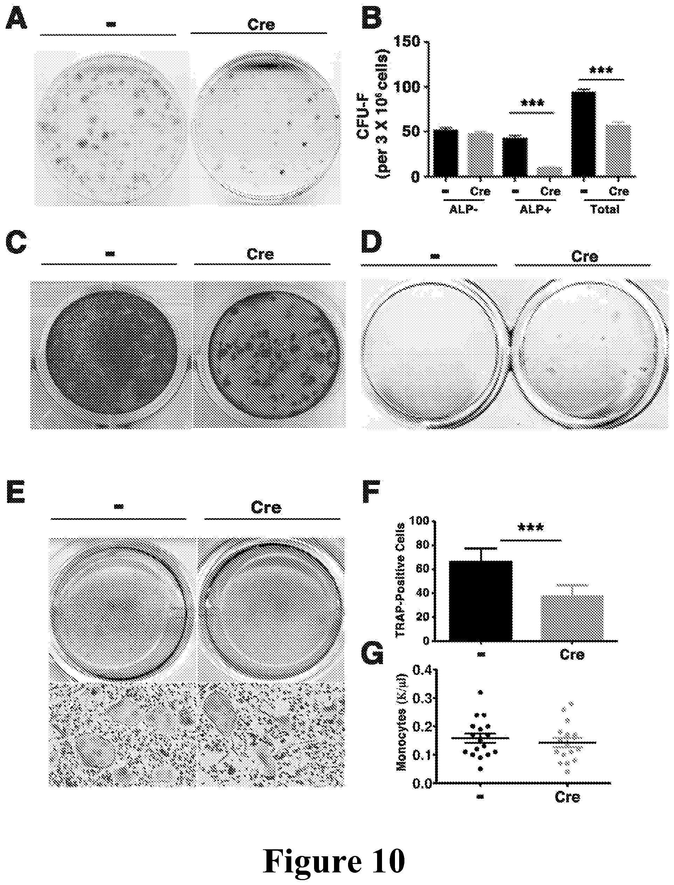

Figures 10A-10G: SHIP-deficiency causes defective development of osteolineage cells and enhanced adipogenesis and defective development of osteoclastogenesis. (A) Representative CFU-F plates of 16 week old SHIP^{flox/flox} (-) and OSXCreSHIP^{flox/flox} (Cre) from primary whole BM on left panel. (B) Corresponding quantitative plots (SHIP^{flox/flox}(-) *black bars* and OSXCreSHIP^{flox/flox} (Cre)*grey bars*) on right panel of CFU-F number (per 3X10⁶ cells) of ALP assay *(the mean colony numbers represent the means obtained from independent analyses of 5 mice of each gender and genotype with each mouse each analyzed in triplicate* ± SD ****P*≤ 0.0001 by a Student's unpaired, two-tailed *t* test). (C) Alizarin red staining showed decreased number of mineralized nodules after osteogenic induction of MSC from OSXCreSHIP^{flox/flox} mice (Cre) in comparison to SHIP^{flox/flox} (-) at 4 weeks of age, (*n*=*5*). (D) Oil Red-O staining showed increased lipid accumulation on day 21 in OSXCreSHIP^{flox/flox} (Cre) in comparison to SHIP^{flox/flox} (-) after adipocyte induction of MSC at 4-weeks of age, (*n*=*5*)*.* (E) TRAP staining of osteoclasts differentiated from BMMs in the presence of RANKL and M-CSF showed a 50% reduction in OC obtained from 8 weeks old BM of OSXCreSHIP^{flox/flox} (Cre) in comparison to BM of SHIP^{flox/flox} (-) mice. Top panels are from representative plates and the bottom panels are 10X magnifications from these plates. (F) The corresponding quantitation shown to the right SHIP^{flox/flox} (-) *black bars* and OSXCreSHIP^{flox/flox} (Cre), *grey bars.* [*n*=*6 mice,* ± SD. ****P*≤ 0.0001, Student's unpaired, two-tailed *t* test]. (G) No difference was observed in circulating monocytes measured at 24 to 52 weeks of age in OSXCreSHIP^{flox/flox} (Cre) in comparison to SHIP^{flox/flox} (-) controls.

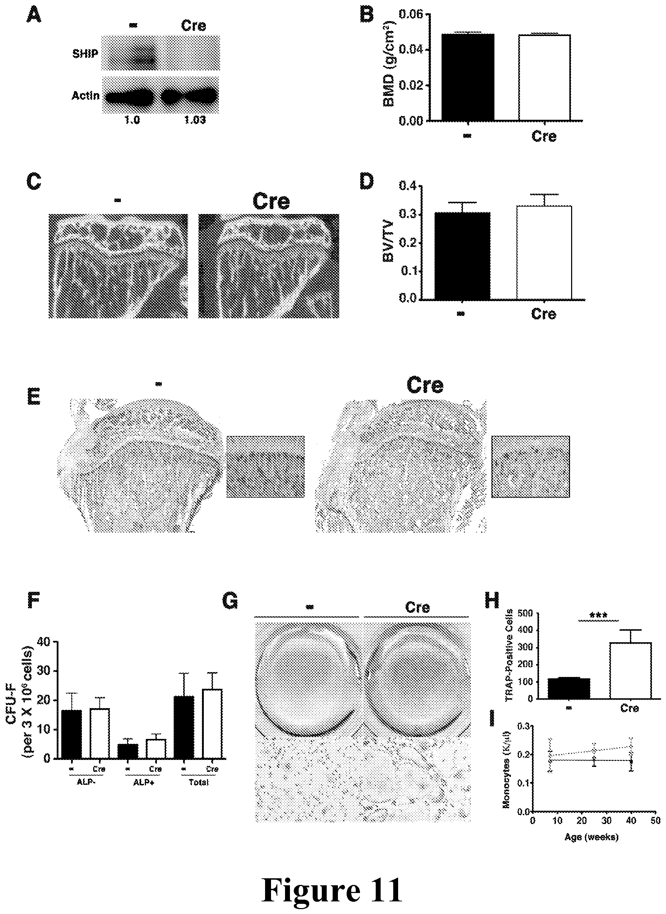

Figures 11A-11I: A SHIP 1-deficient OC compartment does not lead to osteoporosis. (A) Monocytes were differentiated with RANKL and M-CSF and SHIP1 levels in OC from SHIP^{flox/flox} (-) and LysMCreSHIP^{flox/flox} (Cre) mice were assessed by Western blot. Actin serves as a loading control and its relative quantification is indicated below. These results are representative of 4 mice of each genotype. No difference was seen in whole-body BMD by DEXA analysis of (B) 16 week SHIP^{flox/flox} (-) and LysMCreSHIP^{flox/flox} (Cre) mice and in sagittal sections through the proximal metaphysis taken derived from microCT scans (C). (D) Bv/Tv measurements at 16 weeks of SHIP^{flox/flox} (*black bar*) and LysMCreSHIP^{flox/flox} (*open bar*) showed no significant difference. (E) TRAP staining on frozen-sections of SHIP^{flox/flox} (-) and LysMCreSHIP^{flox/flox} (Cre) tibia showed no difference (4x and 20x magnification). (F) Quantitative plots of CFU-F numbers (per 3X10⁶ cells) from 16 week old SHIP^{flox/flox}(-) (*black bars*) and LysMCreSHIP^{flox/flox} (Cre) (*open bars*), mice (*n*=3) showed no difference. (G) TRAP staining of osteoclasts prepared from BMMC that were cultured with RANKL and M-CSF showed a ∼2.8 fold increase in OC numbers in 16 weeks LysMCreSHIP^{flox/flox} (Cre) vs. SHIP^{flox/flox} littermates (-). Top panels are representative plates and the bottom panels are 10Xmagnified images from these plates. (H) The corresponding bar graphs (SHIP^{flox/flox} (-) (*black bars*) and LysMCreSHIP^{flox/flox} (Cre) (*open bars*) to the right represent mean OC numbers as determined for cultures from 4 mice/genotype with BMMC from each mouse analyzed in duplicate ± SD, ****P≤* 0.0001, Student's unpaired, two-tailed *t* test. (I) No difference was observed in monocyte numbers in peripheral blood measured at 7, 25 and 40 weeks of age in LysMCreSHIP^{flox/flox} (Cre) vs. SHIP^{flox/flox} littermates (-).

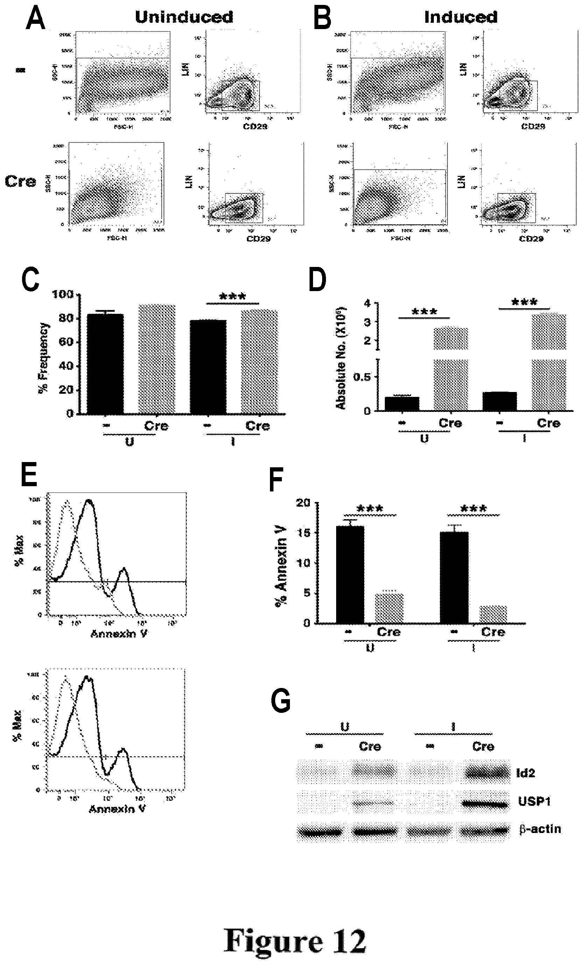

Figures 12A-12G: SHIP-deficient MSCs are more prolific and over-express the USP1/Id2 sternness genes. Bone marrow derived MSCs (BMMSC) from 4 week old OSXCreSHIP^{flox/flox} (Cre) or SHIP^{flox/flox} (-) mice were seeded at equal numbers and cultured under osteogenic conditions (I) for 6 days and processed as described. Uninduced cells (U) were used as controls for all experiments and were also initially seeded at equal cell numbers. (*See Material and Methods for details*)*.* Results shown are representative of three independent experiments for BM MSC from 4 weeks old mice and are also representative of BMMSC cultures prepared from 4 to 16 week old mice. (*Student's unpaired, two-tailed t test (± SEM. ***P≤ 0.0001).* Flow cytometry gates for (A) uninduced and (B) induced cultures (*osteogenic conditions*) to detect CD29⁺Lin⁻ MSC population in cultures prepared from OSXCreSHIP^{flox/flox}(Cre) and SHIP^{flox/flox}(-) mice. Cells were gated for: SSC-H vs FSC-H, lack of expression of a Lineage marker panel (Lin) and surface expression of CD29⁺ as indicated. (C) Significant increased frequency of OSXCreSHIP^{flox/flox} CD29⁺Lin⁻ MSC after osteogenic induction and (D) in the absolute numbers of OSXCreSHIP^{flox/flox} MSC in both uninduced (U) and induced (I) conditions in OSXCreSHIP^{flox/flox} (Cre) and SHIP^{flox/flox} (-) controls. (E) Annexin V staining CD29⁺Lin⁻ MSC in the uninduced (top) and induced (bottom) OSXCreSHIP^{flox/flox}(black line) and SHIP^{flox/flox}(grey line) cultures. (F) Quantitative analysis of apoptotic CD29⁺Lin⁻ cells based on Annexin V⁺ staining as indicated in (E). (G) Western blotting for expression of *Id2* and *USP1* with β-actin serving as loading control.

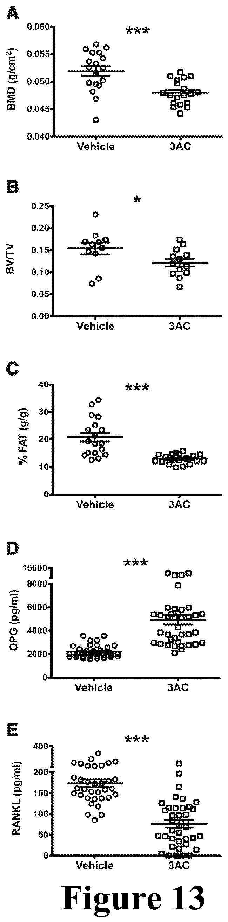

Figure 13A-13E: SHIP1 inhibitor (3AC) reduces bone mass. Male 6-12 month old C57BL6/J mice were injected intraperitoneally with vehicle or 3AC, a SHIP1 inhibitor, at 25 mg/kg three times per week for 4, 8 and 16 weeks. Significant decrease was observed in (A) whole-body BMD and (C) Percent body fat by DEXA analysis in 3AC treated group (*open square*) vs. vehicle group (*open circle*)[±*SEM* ****P*≤ 0.0001 *Student's unpaired, two-tailed t test].* (B) Metaphyseal histomorphometric parameter, bone volume over tissue volume (Bv/Tv) was significantly decreased after 3AC treatment in the 3AC group (*open sqaures*) in comparison to vehicle group (*open circles*)[*±SEM *P*≤ *0.05 Student's unpaired, two-tailed t test].* (D) Serum OPG measured by ELISA was increased, whereas (E) serum RANKL was decreased in 3AC treated group (*open square*) vs. vehicle group (*open circle*)[± SEM ****P≤ 0.0001,* Student's unpaired, two-tailed *t* test] [Each symbol represents an individual mouse].

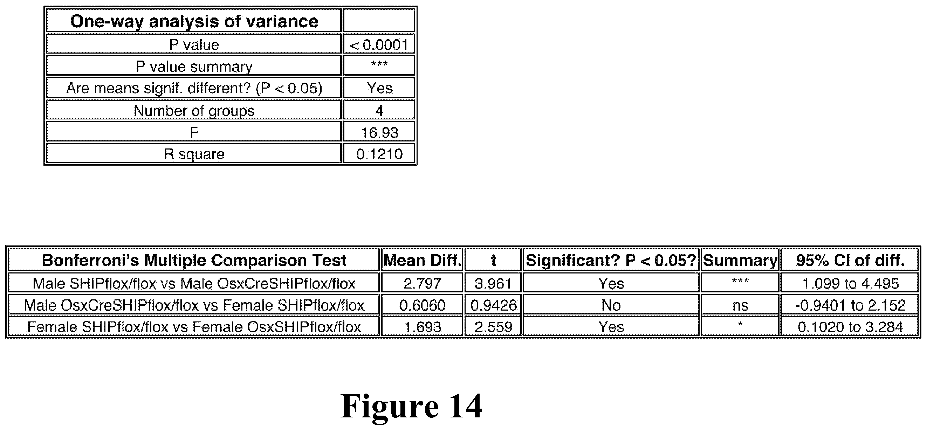

Figure 14: Analysis of covariance (ANCOVA) followed by post-hoc Bonferroni's Multiple Comparison Test to analyze differences in growth retardation in OSXCreSHIP^{flox/flox} in comparison to SHIP^{flox/flox}.

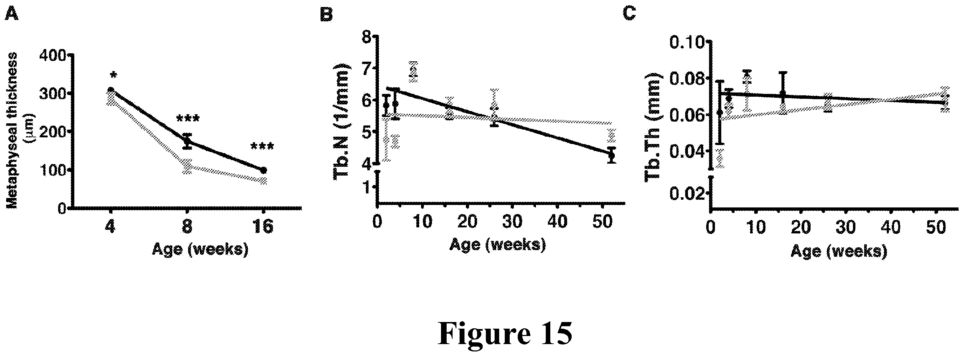

Figures 15A-15C: (A) Metaphyseal thickness (µm) of 4, 8 and 16 weeks old SHIP^{flox/flox} (*black circles*) and OSXCreSHIP^{flox/flox} (*grey squares*) mice. [± SD. **P*≤ 0.01 and *** *P≤* 0.0001, *Student's unpaired, two-tailed t test*]*.* (B) Trabecular number and (C) thickness were analyzed by linear regression in 2-, 4-, 8-, 16-, 26- and 52 week old SHIP^{flox/flox} (*black circles*) and OSXCreSHIP^{flox/flox} (*grey circles*) mice.

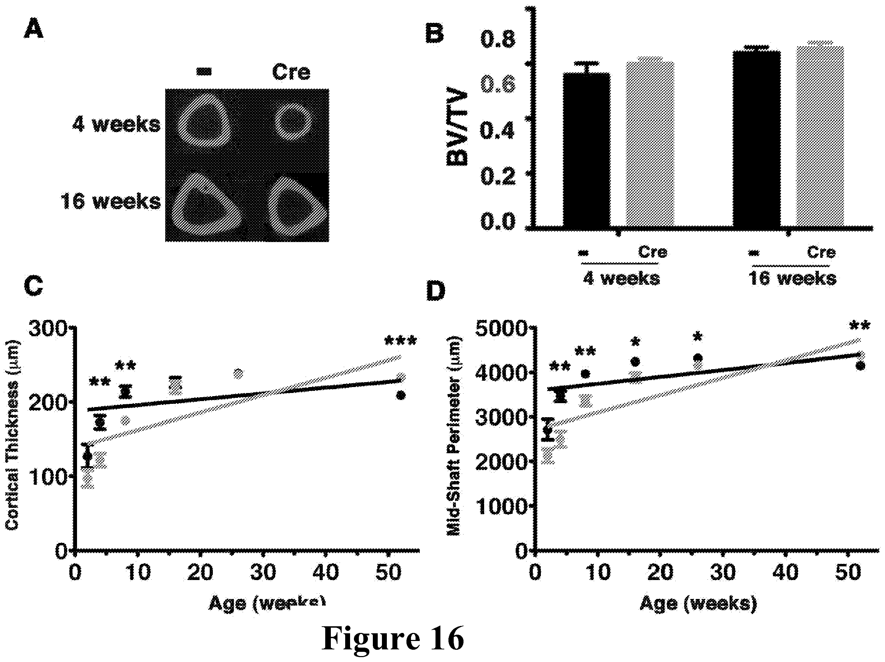

Figures 16A-16D: Cortical bone morphology as evidenced by MicroCT at the tibial mid-shaft. (A) Microradiographs of transverse sections of the tibial midshaft of OSXCreSHIP^{flox/flox} (Cre) and SHIP^{flox/flox} (-) at 4 and 16 weeks of life, showing representative cortical thickness and perimeter for representative SHIP^{flox/flox} and OSXCreSHIP^{flox/flox} mice as indicated. (B) Mean midshaft Bv/Tv measures for SHIP^{flox/flox}(-)(*black bars*) and OSXCreSHIP^{flox/flox} (Cre)(*grey bars*), mice as indicated. (C) Cortical thickness and (D) Midshaft perimeter measures for tibias from 2, 4, 8, 16, 26 and 52 weeks old SHIP^{flox/flox} (*black circle*) and OSXCreSHIP^{flox/flox} (*grey circle*) mice were analyzed by comparing slopes linear regression analysis and Student's unpaired, two-tailed *t* test (± SD. **P*≤ 0.01, ***P*≤ 0.001 and ****P*≤ 0.001). The p-value of linear regression analysis of cortical thickness was 0.0058 and cortical perimeter 0.00276.

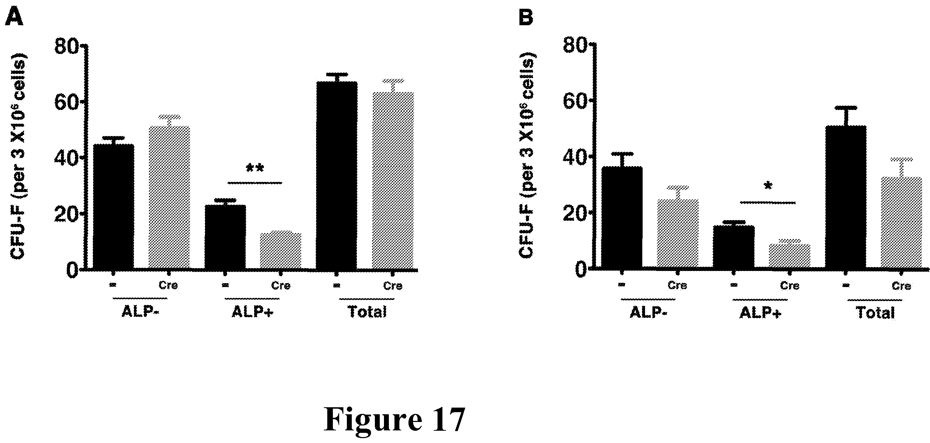

Figures 17A-17B: Quantitative plots of CFU-F number (per 3X10⁶ cells) for (A) 26-weeks and (B) 52-weeks old SHIP^{flox/flox} (-) *black bars* and OSXCreSHIP^{flox/flox} (Cre) *grey bars,* mice *(the mean colony numbers represent the means obtained from independent analyses of 5 mice of each gender and genotype with each mouse each analyzed in triplicate* ± SD **P*≤ 0.05 and ***P*≤ 0.01 by a Student's unpaired, two-tailed *t* test).

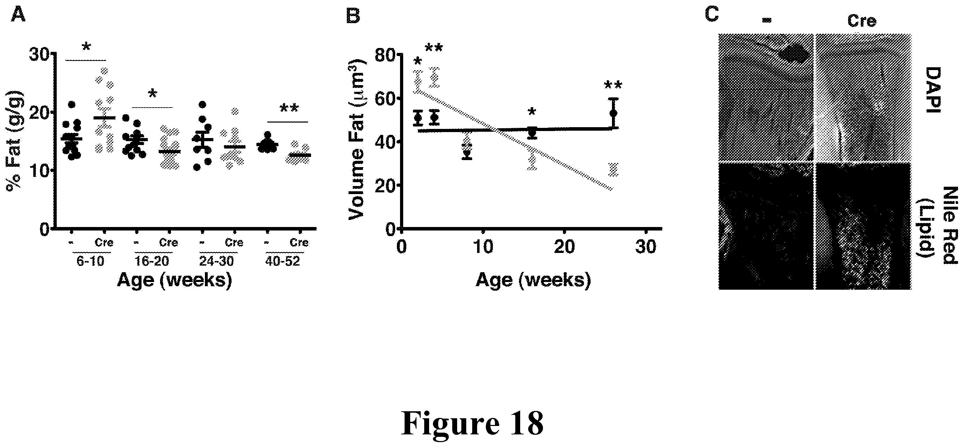

Figures 18A-18C: The loss of SHIP1 results in increased adipogenesis and adiposity. (A) Body fat was increased between 6 to 10 weeks and was decreased at 16 to 20 weeks and 40 to 52 weeks in OSXCreSHIP^{flox/flox} (Cre)(*grey circles*) in comparison to SHIP^{flox/flox} (-) (*black circles*) controls. (B) An analysis of microCT images using image segmentation demonstrated that bone marrow (*metaphyseal*) fat increased 16% and 35% at 2 and 4 weeks, respectively, and was decreased 32% at 16 weeks in OSXCreSHIP^{flox/flox} (Cre) (*grey circles*) tibia vs. SHIP^{flox/flox}(*black circles*) controls. A linear regression analysis demonstrated that there is no age-associated change in adiposity in the SHIP^{flox/flox} mice; whereas the adiposity in OSXCreSHIP^{flox/flox} mice was shown to linearly decrease with age. The *p*-value for linear regression analysis was <0.0001. Student's unpaired; two-tailed *t* test analysis at individual time points was also conducted. [± SD. **P*≤ 0.01 and ***P*≤ 0.001]. (C) Increased Nile Red (*green*) fluorescent staining was observed throughout the bone marrow of 4-weeks OSXCreSHIP^{flox/flox} tibias (Cre); whereas, very little Nile Red staining was observed in the BM of SHIP^{flox/flox} tibias (-); with most staining restricted to the perimeter of the marrow space. DAPI staining (top panels) was employed to observe general morphology (4x magnification).

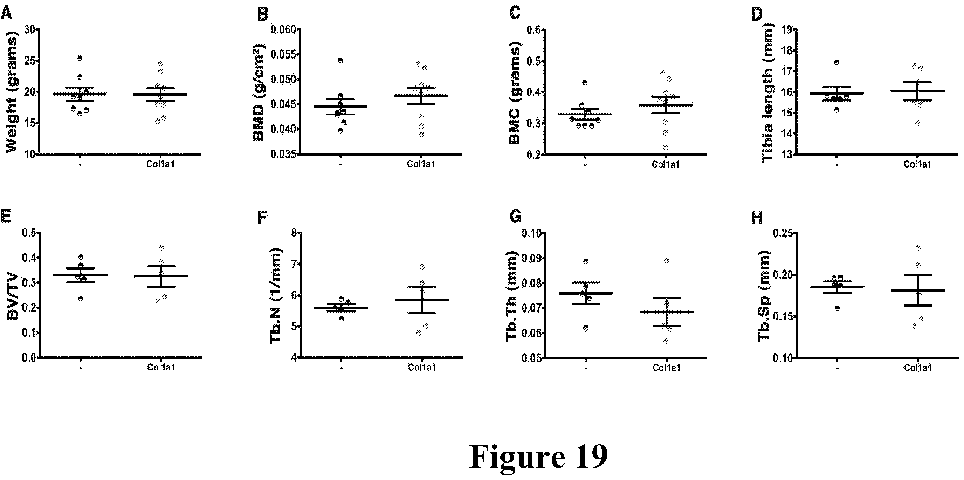

Figures 19A-19H: SHIP1 expression in mature osteoblasts does not limit bone apposition. The loss of SHIP1 in mature osteoblasts in 4-6 weeks old Col1a1CreSHIP^{flox/flox} mice (Col1a1) resulted no difference was seen in growth (A) body weight and (D) tibia length in comparison to SHIP^{flox/flox} controls (-). Whole-body DEXA analysis showed no difference in (B) Bone mineral density (BMD) and (C) Bone mineral content (BMC). Metaphyseal histomorphometric parameter, (E) Bv/Tv, (F) Trabecular number (G) Trabecular thickness and (H) Trabecular space.

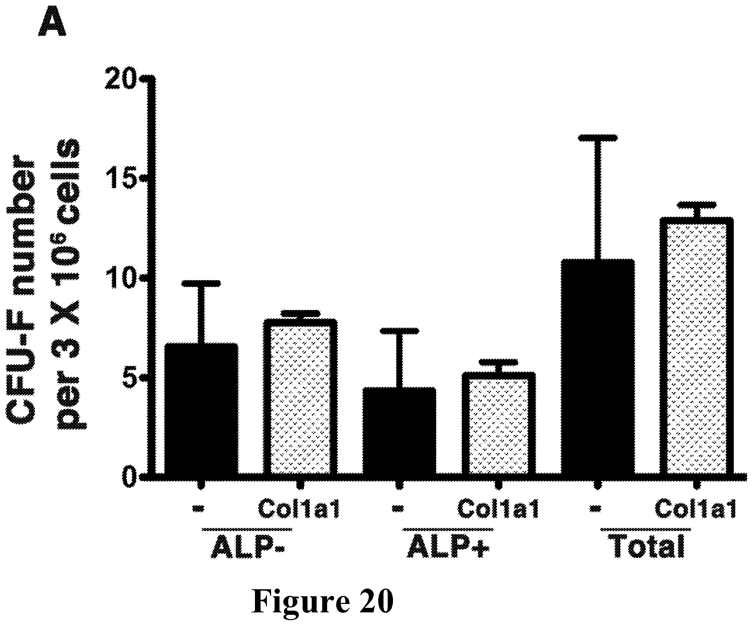

Figure 20: Quantitative plots of CFU-F numbers (per 3X10⁶ cells) from 4-6 weeks old SHIP^{flox/flox} (-)(*black bars*) and Col1a1CreSHIP^{flox/flox} mice (Col1a1)(*dotted bars*), mice (*n*=*3*) showed no difference.

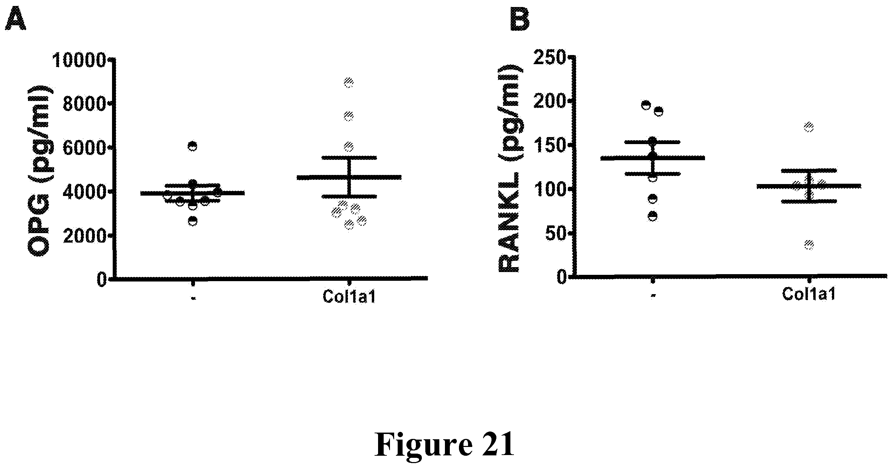

Figures 21A-21B: No difference was observed in serum (A) OPG and (B) RANKL levels in 4-6 weeks old Col1a1CreSHIP^{flox/flox} mice (Col1a1) in comparison to SHIP^{flox/flox} mice controls (-).

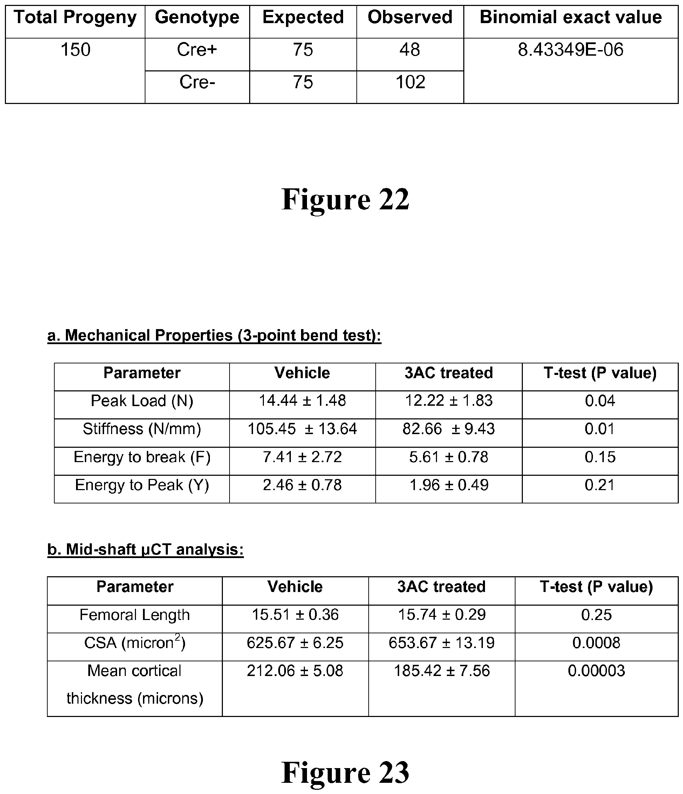

Figure 22: Observational table charting binomial exact analysis on OSXCreSHIP^{flox/flox} (Cre⁺) and SHIP^{flox/flox} (Cre⁻) progeny birth frequencies derived from pairings of OSXCreSHIP^{flox/flox} and SHIP^{flox/flox} partners.
Figures 23A-23B: SHIP1 inhibitor (3AC) post-treatment mid-shaft analysis. (A) Mechanical Properties (3-point bend test) and (B) Mid-shaft cortical µCT analysis.

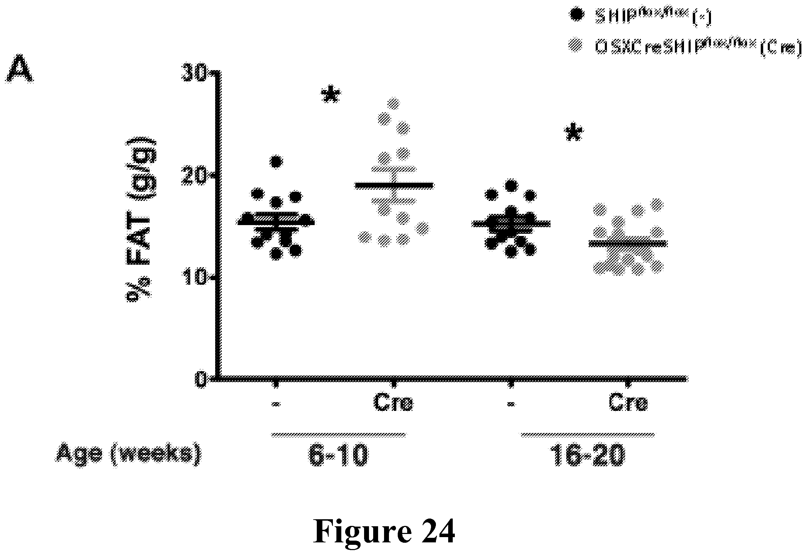

Figure 24: Expression of SHIP1 in mesenchymal stem cell and osteolineage progenitors regulates adipogenesis. Percent of body fat was increased between 6 to 10 weeks and was decreased at 16 to 20 in OSX CreSHIP^{flox/flox} (Cre) (grey circles) in comparison to SHIP^{flox/flox} (-) (black circles) controls.

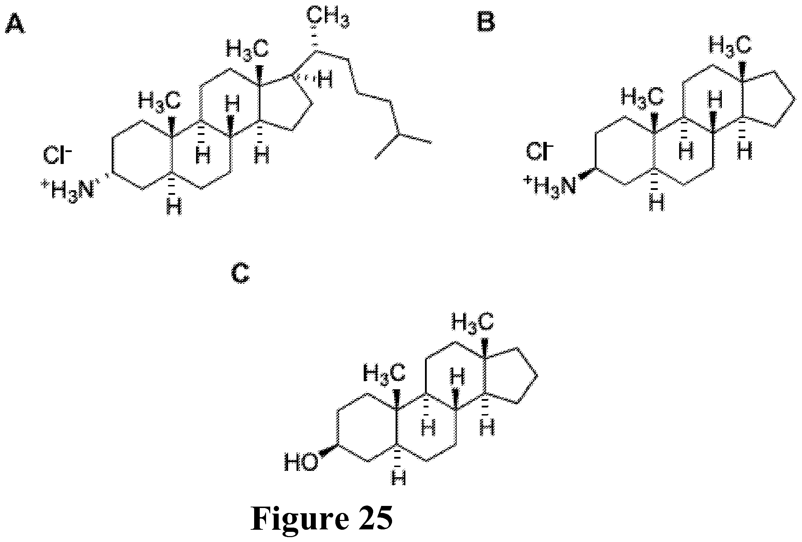

Figures 25A-25C: Chemical designation and structures of 3ac k118 and K189. (A) 3AC (K100), 3a-aminocholestane hydrochloride. (B) K118, 3b-amino-5a-androstane hydrochloride. (C) K189, 5b-hydroxy-5a-androstane.

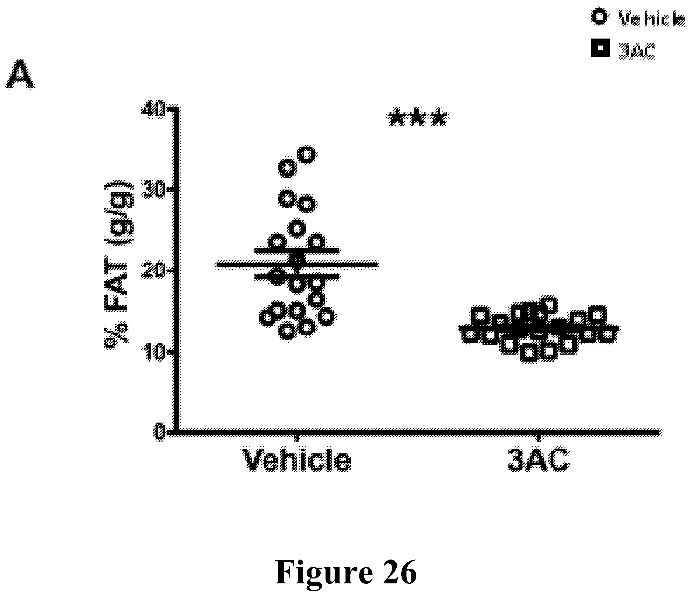

Figure 26: SHIPi by 3AC treatment reduces body fat content in aging mice. Male 6-12 month old C57BL6/J mice were injected with vehicle or 3AC, at 25 mg/kg three times per week for 4, 8, and 16 weeks. Significant decrease was observed in, Percent body fat by DEXA analysis in 3AC treated group (*open square*) vs. vehicle group (*open circle*)*.*

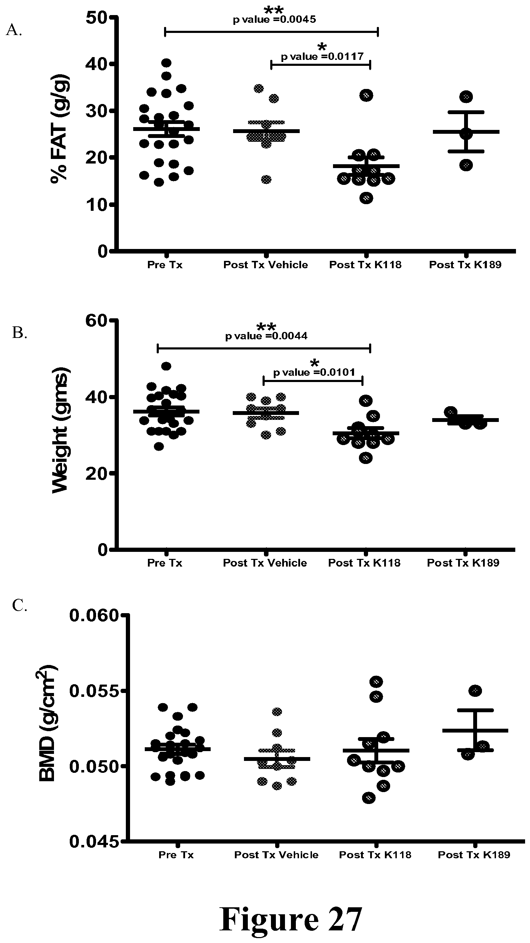

Figures 27A-27C: K118 reduces age-associated weight and body fat, but does not reduce BMD. (A) Percent body fat of vehicle, K118 and K189 treated (Tx) mice before and after treatment. (B) Weight of vehicle, K118 and K189 treated (Tx) mice before (pre Tx) and after treatment (post Tx). (C) BMD of vehicle, K118 and K189 treated (Tx) mice before and after treatment.

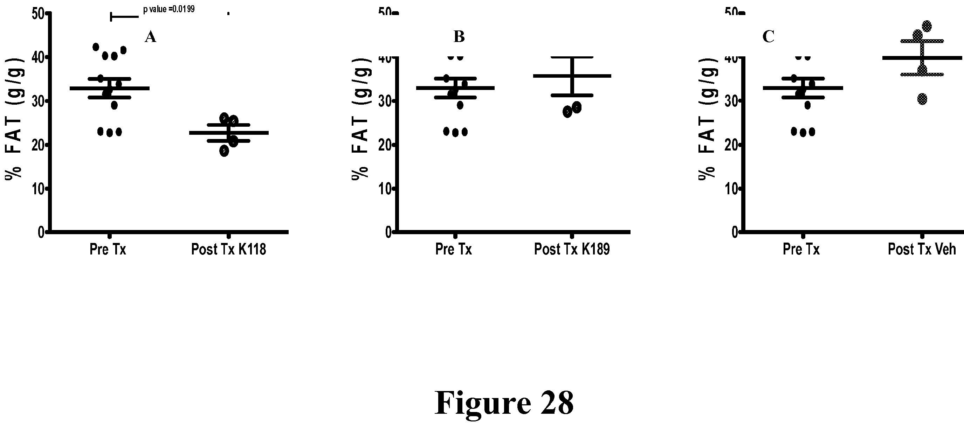

Figures 28A-28C: K118 reverses diet-induced obesity in young adult mice. (A) Percent body fat of K118 treated (Tx) mice before (pre Tx) and after treatment (post Tx). (B) Percent body fat of K189 treated (Tx) mice before and after treatment. (C) Percent body fat of Vehicle treated (Tx) mice before and after treatment.

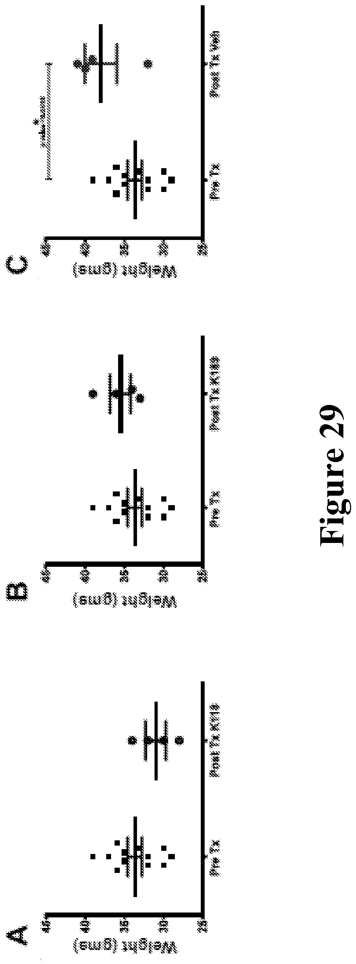

Figures 29A-29C: K118 prevents diet-induced weight gain in young adult mice. (A) Weight of K118 treated (Tx) mice before (pre Tx) and after treatment (post Tx). (B) Weight of K1189 treated (Tx) mice before and after treatment. (C) Weight of Vehicle treated (Tx) mice before and after treatment.

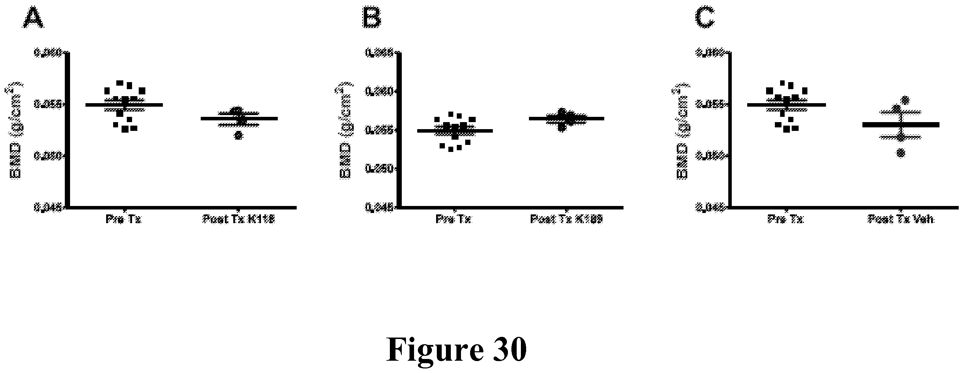

Figures 30A-30C: K118 does not cause a significant reduction in BMD in young adult mice on a high fat diet. (A) BMD of K118 treated (Tx) mice before (pre Tx) and after treatment (post Tx). (B) BMD of K1189 treated (Tx) mice before and after treatment. (C) BMD of Vehicle treated (Tx) mice before and after treatment.

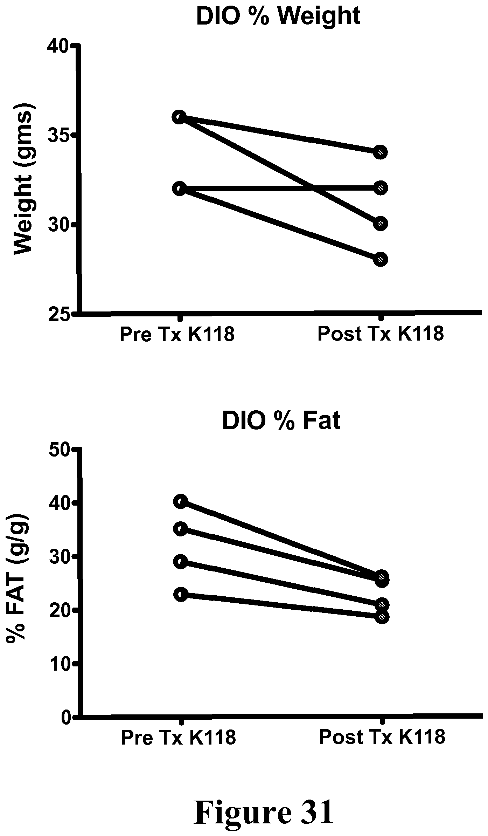

Figures 31A-31B: K118 reverses diet-induced weight and obesity in individual young adult mice. Analysis of individual mouse, (A) Weight and (B) Percent body fat in the K118 study before and after treatment (Tx).

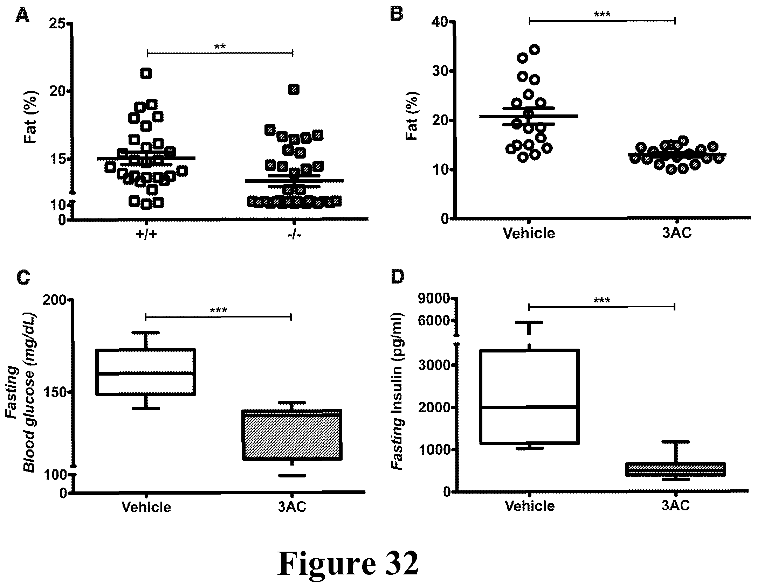

Figures 32A-32D: Age-associated loss in fat in SHIP-deficient MS/PC and 3AC treated mice. (A) Percent total body fat in 16-48 week OSXCreSHIP^{flox/flox} (-/-) and SHIP^{flox/flox} (+/+) controls (n>30). (B) Percent total body fat in 3AC treated and vehicle mice (n≥15). Fasting (C) Blood glucose levels (mg/dL) and (D) Insulin (pg/ml) in 3AC treated mice post treatment. All results are expressed as mean ±*SEM, Student's unpaired, two-tailed t test *p* ≤ *0.05*

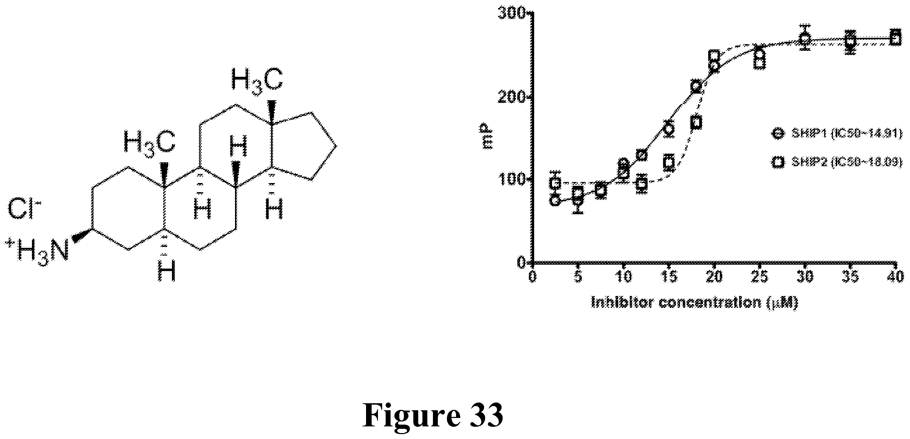

Figure 33: Structure of pan SHIP1/2 inhibitor, K118 and its SHIP1 inhibitory activity as measured by a fluorescent polarization assay designed for 5'-inositol phosphatases (Echelon Biosciences). Structure of K118. SHIP1 and SHIP2 inhibitory activity measured FP assay.

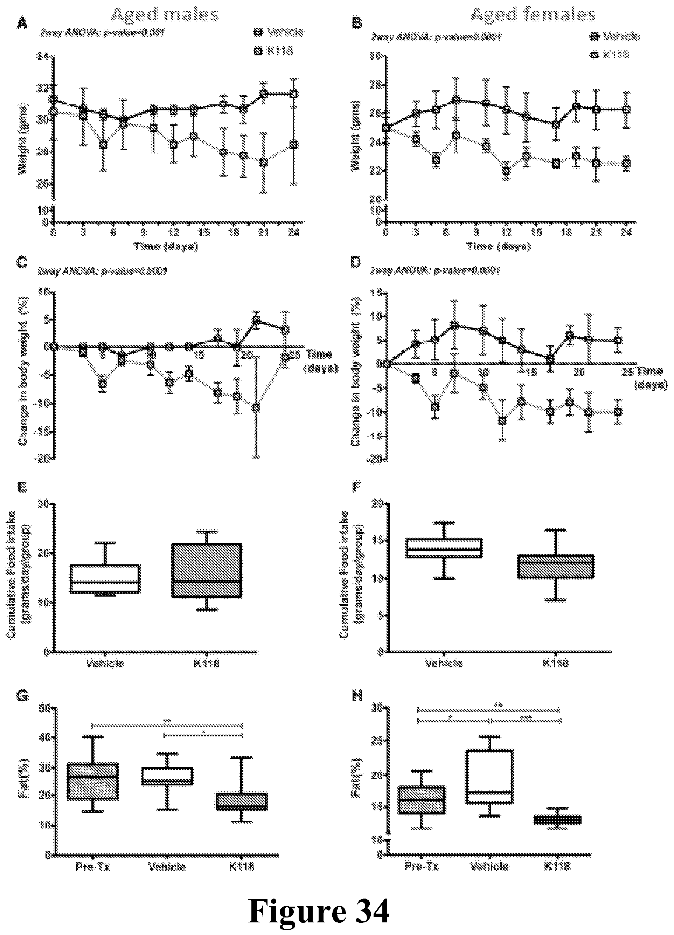

Figures 34A-34H: Phenotypic analysis of K118 treated aged male and female mice. (A) and (B): Analysis of body-weight of Aged male and Aged female mice from the beginning to the end of the vehicle or K118 treatment. (C) and (D): Change in body-weight (%) in Aged male and Aged female mice from the beginning to the end of the vehicle or K118 treatment. (G) and (H): Percent total body fat of mice before (Pre-Tx) and after K118 and vehicle treatment Aged male and Aged female. (E) and (F): No change in food intake in either vehicle or K118 treated Aged male and Aged females. All results are expressed as mean ±*SEM, Student's unpaired, two-tailed t test *p* ≤ *0.05,* (n≥10).

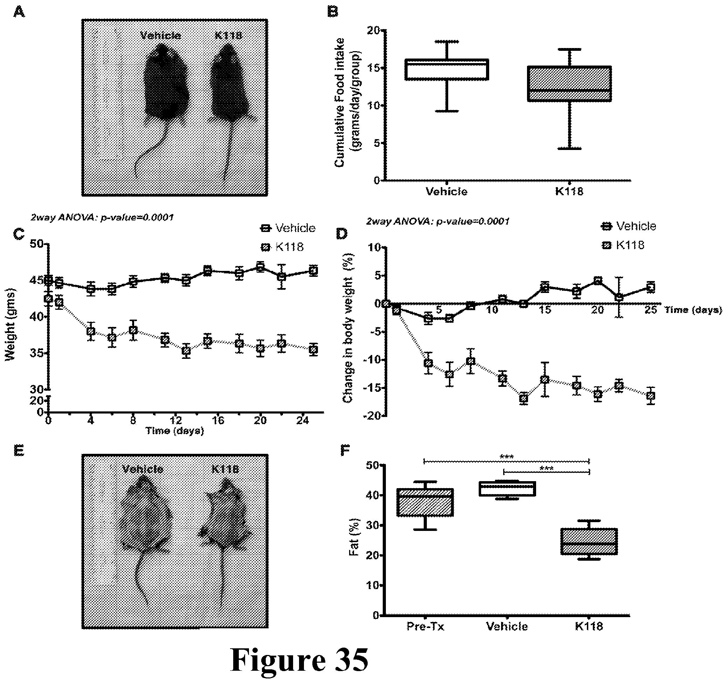

Figures 35A-35F: Phenotypic analysis of K118 treated DIO-mice. (C): Analysis of body-weight of DIO-C57B6 mice from the beginning to the end of the vehicle or K118 treatment. (D) Change in body-weight (%) in DIO-C57B6 mice from the beginning to the end of the vehicle or K118 treatment. (A) and (E): Representative pictures DIO-C57B6 mice after either vehicle or K118 treatment. (B): No change in food intake in either vehicle or K118 treated DIO-C57B6 mice. (F): Representative gross comparison of fat depots in DIO-C57B6 mice after either vehicle or K118 treatment. Percent total body fat of DIO-C57B6 mice before (Pre-Tx) and after K118 and vehicle treatment. All results are expressed as mean ±*SEM, Student's unpaired, two-tailed t test *p* ≤ *0.05,* (n≥10).

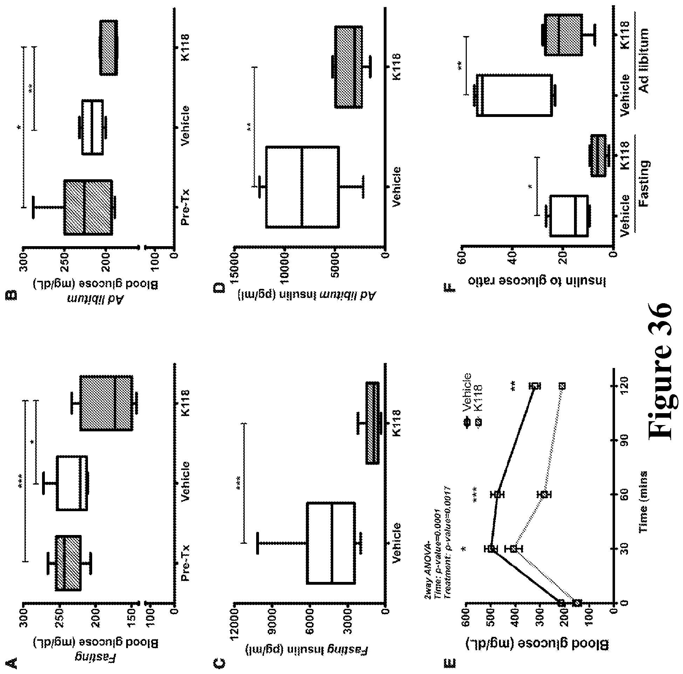

Figures 36A-36F: K118 treatment in DIO-C57B6 mice improves their impaired glucose homeostasis. (A) and (B): Fasting and Ad libitum blood glucose levels (mg/dL) in pretreated mice (Pre-Tx), vehicle and K118 treated DIO-C57B6 mice, (n=10). (C) and (D): Fasting and Ad libitum serum insulin levels (pg/ml) in pretreated mice (Pre-Tx), vehicle and K118 treated DIO-C57B6 mice, (n=10). (F): Fasting and Ad libitum serum insulin levels to blood glucose in vehicle and K118 treated DIO-C57B6 mice, (n=10). (E): Intraperitoneal glucose tolerance test on vehicle and K118 treated DIO-C57B6 mice, (n=6). All results are expressed as mean ±*SEM, Student's unpaired, two-tailed t test *p* ≤ *0.05.*

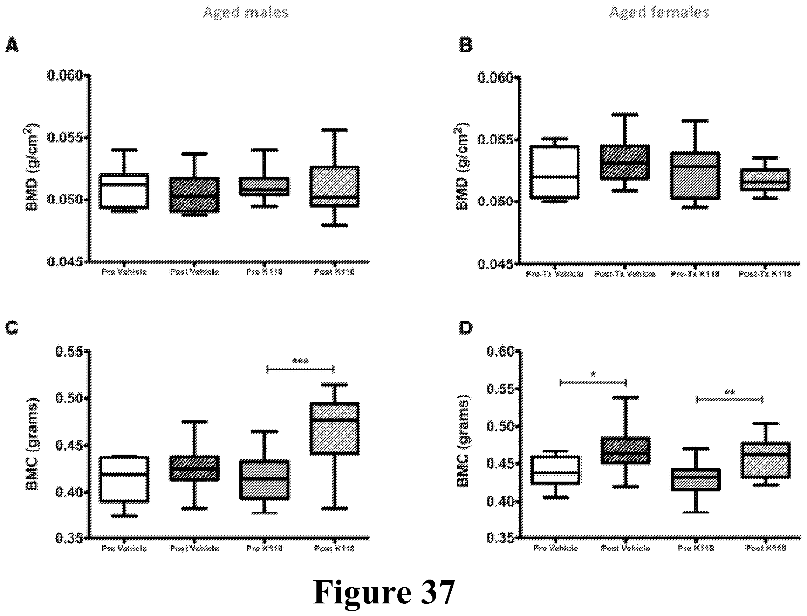

Figures 37A-37D: K118 treated aged male and female mice does not affect BMD and BMC. Bone mineral density (A) Aged male and (B) Aged female mice of pre and post vehicle and K118 treatment. Bone mineral content (C) Aged male and (D) Aged female mice of pre and post vehicle and K118 treatment. All results are expressed as mean ±*SEM, Student's unpaired, two-tailed t test *p ≤ 0.05,* (n=10).

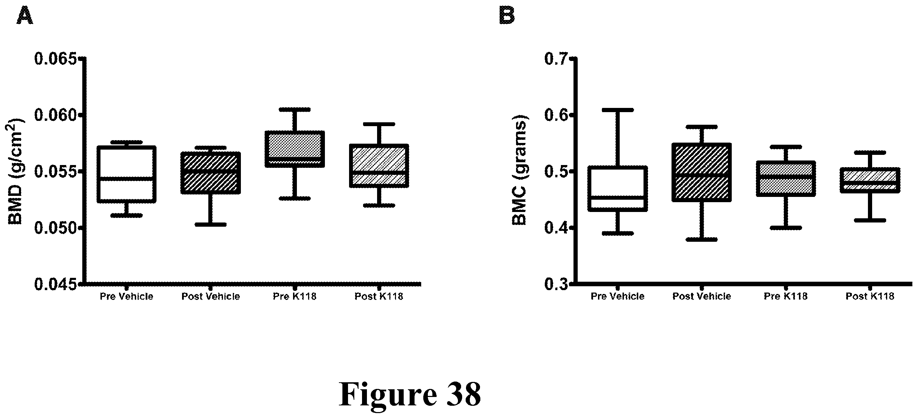

Figures 38A-38B: K118 treatment in DIO-C57B6 mice does not affect BMD and BMC. (A) Bone mineral density and (B) Bone mineral content of pre and post treatment vehicle and K118-DIO-C57B6 mice. All results are expressed as mean *±SEM, Student's unpaired, two-tailed t test *p* ≤ *0.05,* (n=9).

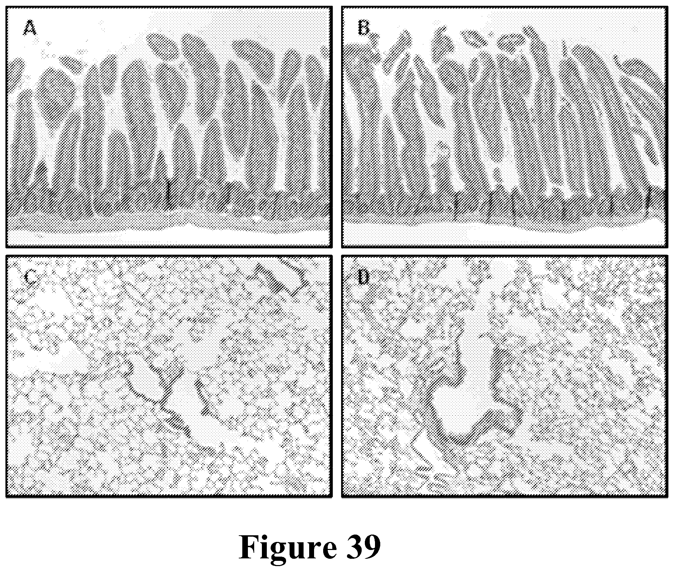

Figures 39A-39D: Histological appearance of the ileum (A-B) and lung (C-D) of C57BL/6J mice administered K118 (A, C) or vehicle (B, D), showing no significant lesions, including absence of Crohn's disease-like ileitis and absence of eosinophillic crystalline pneumonia, characteristic of germ line SHIP^{-/-} mice.

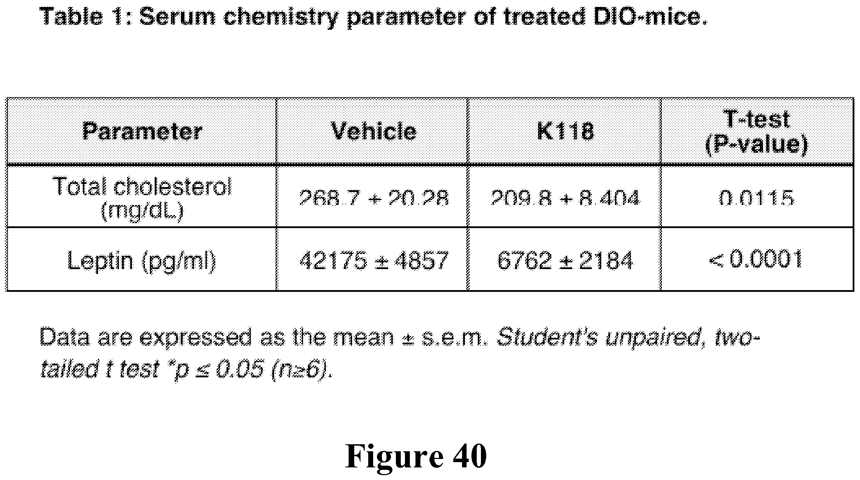

Figure 40: Leptin and total cholesterol in K118 DIO mice. Data are expressed as the mean ± s.e.m. Student's unpaired, two-tailed t test *p ≤ 0.05 (n=6 for cholesterol and n=10 for Leptin).

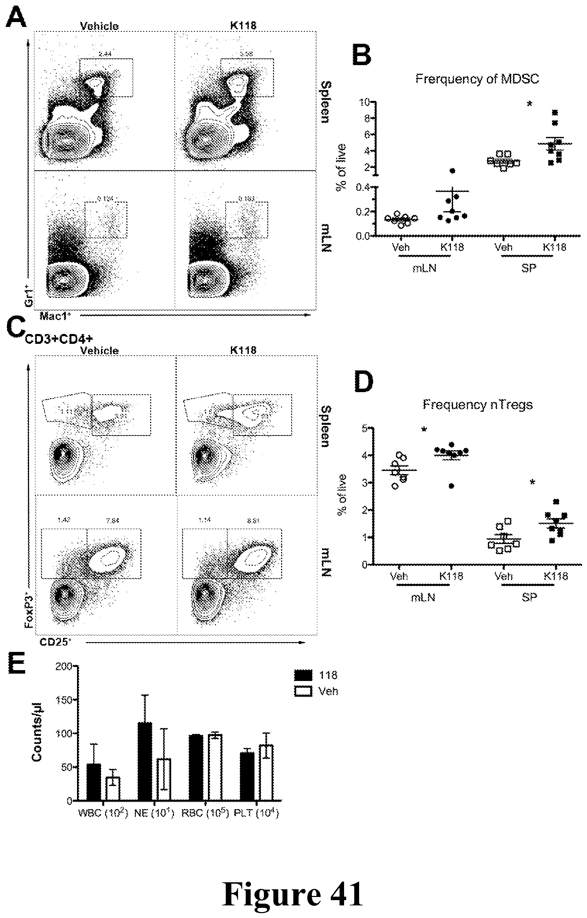

Figures 41A-41E: Increase in immunoregulatory cells following oral administration of K118 in mice. (A) Representative flow cytometry plots for Spleen and mLN of Vehicle and K118 treated mice, stained for Gr1 and Mac1 following gating on live cells. (B) Frequency of MDSC in mLN and Spleen. (C) Representative flow cytometry plots for Spleen and mLN of Vehicle and K118 treated mice, stained for CD25 and FoxP3 following gating on live cells CD3+CD4+ cells. (D) Frequency of nTregs in Spleen and mLN. (E) Increase in Neutrophils (NE) in K118 p=0.0555 (Student t-test).

### DETAILED DESCRIPTION OF THE INVENTION

The present invention generally relates to new uses of pan-SHIP1/2 inhibitors. Described are pharmaceutical compositions that comprise various SHIP inhibitors described herein, including, without limitation, the SHIP1 and/or pan-SHIP1/2 inhibitors as disclosed herein.

In one aspect, the present invention relates to a pan-SHIP1/2 inhibitor for use in a method to treat obesity or to reduce body fat in an obese subject, as claimed in claim 1.

In another aspect, the present invention relates to a pan-SHIP1/2 inhibitor for use in treatment to reduce glucose intolerance or decrease insulin resistance in a subject, as claimed in claim 2.

Also described herein is a method to limit bone development in a subject suffering from an osteopetrotic or sclerotic disease. This method involves administering to a subject suffering from an osteopetrotic or sclerotic disease a SHIP1 inhibitor or a pan-SHIP1/2 inhibitor in an amount effective to limit bone development in the subject.

Also described herein is a method to treat or prevent diabetes in a subject. This method involves administering a SHIP1 inhibitor or a pan-SHIP1/2 inhibitor to a subject in an amount effective to treat or prevent diabetes in the subject.

Also described is a method to lower cholesterol in a subject. This method involves administering a SHIP1 inhibitor or a pan-SHIP1/2 inhibitor to a subject in an amount effective to lower cholesterol in the subject.

According to various options, the substance suitable for use in the methods described herein can be a nucleic acid, such as a genetic construct or other genetic means directing expression of an antagonist of SHIP function. Nucleic acid molecules suitable for the described method include anti-sense polynucleotides, other polynucleotides that bind to SHIP mRNA, recombinant retroviral vector, or a combination thereof. A genetic construct which may be used comprises a gene delivery vehicle, a recombinant retroviral vector, or a combination thereof. In one option, the substance that inhibits SHIP function may be a nucleic acid that hybridizes to a SHIP mRNA.

In other options, the substances suitable for the described method may also include peptidomimetic inhibitors of SHIP function, ribozymes, and an RNA aptamer, or a combination thereof.

Pharmaceutical agents or genetic therapies that reduce or eliminate SHIP activity and function encompass, but are not limited to the following: 1) small molecule inhibitors (preferably having a molecular weight of less than 10,000) of SHIP enzymatic activity (i.e. suicide substrates; competitive or non-competitive inhibitors of SHIP activity; RNA aptamers; or PIP 3, 4, or 5 analogs), 2) anti-sense oligonucleotides, 3) peptidomimetics, 4) ribozymes, 5) means for interfering with transcription and/or translation of SHIP RNA, or 6) genetic therapy comprising transfection with a dominant negative SHIP mutant. These agents and/or genetic therapies can exert their effects by preventing the recruitment of SHIP to complexes with other signal transduction components or to the plasma membrane where SHIP can access its inositol phospholipid substrates.

Within the present disclosure, the following terms are to be understood as follows:

An "isolated polypeptide" or "isolated polynucleotide" as used herein refers to a polypeptide or polynucleotide, respectively, produced in vivo or in vitro in an environment manipulated by humans using state of the art techniques of molecular biology, biochemistry and gene therapy. For example, an isolated polypeptide can be produced in a cell free system by automated peptide or polypeptide synthesis, in heterologous host cells transformed with the nucleic acid sequence encoding the polypeptide and regulatory sequences for expression in the host cells, and in an animal into which the coding sequence of the polypeptide has been introduced for expression in the animal. A polypeptide or polynucleotide is "isolated" for purposes herein to the extent that it is not present in its natural state inside a cell as a product of nature. For example, such isolated polypeptides or polynucleotides can be 10% pure, 20% pure, or a higher degree of purity.

The term "inositol polyphosphate 5-phosphatase" as used herein refers to a family of phosphatases each of which removes the 5 phosphate from inositol- and phosphatidylinositol-polyphosphates. The family of proteins is determined by the substrate specificity of these enzymes and by amino acid sequence homology. A description of some of the aspects of the family is provided in Jefferson and Majerus, J Biol Chem 270: 9370-77 (1995). The term "activated T cell" and "activated B cell" refers to T and B cells that have been stimulated, for example, with cytokines or growth factors, or which have had their antigen receptors cross-linked using antibodies, all of which events stimulate gene expression, cell proliferation or other responses in T and B cells.

The term "tyrosine phosphorylated" as used herein refers to the addition of a phosphate group at a tyrosine residue. Generally, tyrosine phosphorylation of polypeptides is associated with activation or inactivation of signaling pathways. Tyrosine phosphorylation is also associated with activation or inhibition of signaling molecules. Tyrosine phosphorylation of a polypeptide of the invention can occur in response to, for example, B or T cell activation. In some cases, binding to other polypeptides occurs before, after, or during the tyrosine phosphorylation of a polypeptide.

The term "apparent molecular weight" as used herein refers to the molecular weight of the protein or polypeptide as it migrates on a polyacrylamide gel under reducing or non-reducing conditions. The "apparent" molecular weight may be accounted for by glycosylations or other moieties that alter the molecular weight of the polypeptide alone.

The term "SHIP" as used herein refers to SH2-containing inositol-5-phosphatase. SHIP may have an apparent molecular weight of about 145 kDa and is expressed in at least hemopoietic cells. It contains an amino-terminal src-homology domain (SH2), a central 5'-phosphoinositol phosphatase domain, two phosphotyrosine binding consensus sequences, and a proline-rich region at the carboxyl tail.

The term a "means for inhibiting SHIP function" comprises genetic and non-genetic means for inhibiting SHIP function, and includes substances that inhibit SHIP functions.

Among the genetic construct inhibiting SHIP function are various "gene delivery vehicles" known to those of skill in the art, that facilitate delivery to a cell of, for example, a coding sequence for expression of a polypeptide, such as a SHIP inhibitor, an anti-sense oligonucleotide, an RNA aptamer capable of inhibiting SHIP enzymatic activity, an RNA aptamer capable of inhibiting a ribozyme, or another genetic construct of inhibiting SHIP activity known to those of skill in the art.

Among the non-genetic means inhibiting SHIP function are pharmaceutical agent, pharmaceutically acceptable salts thereof that are preferably administered in a pharmaceutically acceptable carrier.

According to certain options, substances suitable for use in the described methods can be a nucleic acid, such as a genetic construct or other genetic means directing expression of an antagonist of SHIP function. Nucleic acid molecules suitable for the method include anti-sense polynucleotides, other polynucleotides that bind to SHIP mRNA, recombinant retroviral vector, or a combination thereof. A genetic construct of the invention which may be used comprises a gene delivery vehicle, a recombinant retroviral vector, or a combination thereof. In an option, the substance that inhibits SHIP function is a nucleic acid that hybridizes to a SHIP mRNA.

Substances may also include peptidomimetic inhibitors of SHIP function, ribozymes, and an RNA aptamer, or a combination thereof.

Suitable substances for the described methods may also be a low molecular weight substance having a molecular weight of less than about 10,000 that inhibits SHIP activity.

The cell to which said component or substance is delivered can be within a mammal, as in in vivo gene therapy, or can be removed from a mammal for transfection, or administration of a pharmaceutical agent, and can be subsequently returned to the mammal, as, for example, in ex vivo therapy or ex vivo gene therapy. The delivery vehicle can be any component or vehicle capable of accomplishing the delivery of a gene or substance to a cell, for example, a liposome, a particle, naked DNA, or a vector. A gene delivery vehicle is a recombinant vehicle, such as a recombinant viral vector, a nucleic acid vector (such as plasmid), a naked nucleic acid molecule such as a gene, a nucleic acid molecule complexed to a polycationic molecule capable of neutralizing the negative charge on the nucleic acid molecule and condensing the nucleic acid molecule into a compact molecule, a nucleic acid associated with a liposome (Wang, et al., PNAS 84:7851, 1987), and certain eukaryotic cells such as a producer cell, that are capable of delivering a nucleic acid molecule having one or more desirable properties to host cells in an organism. The desirable properties include the ability to express a desired substance, such as a protein, enzyme, or antibody, and/or the ability to provide a biological activity, which is where the nucleic acid molecule carried by the gene delivery vehicle is itself the active agent without requiring the expression of a desired substance. One example of such biological activity is gene therapy where the delivered nucleic acid molecule incorporates into a specified gene so as to inactivate the gene and "turn off' the product the gene was making, or to alter the translation or stability of the mRNA of the specified gene product. Gene delivery vehicle refers to an assembly which is capable of directing the expression of the sequence(s) or gene(s) of interest or of turning off the gene of interest. The gene delivery vehicle will generally include promoter elements and may include a signal that directs polyadenylation. In addition, the gene delivery vehicle can include a sequence which is operably linked to the sequence(s) or gene(s) of interest and, when transcribed, acts as a translation initiation sequence. The gene delivery vehicle may also include a selectable marker such as Neo, SV.sup.2 Neo, TK, hygromycin, phleomycin, histidinol, or DHFR, as well as one or more restriction sites and a translation termination sequence. Gene delivery vehicles as used within the present invention refers to recombinant vehicles, such as viral vectors (Jolly, Cancer Gen. Therapy 1:5164, 1994), nucleic acid vectors, naked DNA, oligonucleotides, cosmids, bacteria, and certain eukaryotic cells (including producer cells; see U.S. Ser. No. 08/240,030 and U.S. Ser. No. 07/800,921), that are capable of eliciting an immune response within an animal. Representative examples of such gene delivery vehicles include poliovirus (Evans et al., Nature 339:385-388, 1989; and Sabin, J. Biol. Standardization 1:115-118, 1973); rhinovirus; pox viruses, such as canary pox virus or vaccinia virus (Fisher-Hoch et al., PNAS 86:317-321, 1989; Flexner et al., Ann. N.Y. Acad. Sci. 569:86-103, 1989; Flexner et al., Vaccine 8:17-21, 1990; U.S. Pat. Nos. 4,603,112, 4,769,330, and 5,017,487; WO 89/01973); SV40 (Mulligan et al., Nature 277:108-114, 1979); retrovirus (U.S. Pat. No. 4,777,127, GB 2,200,651, EP 0,345,242, and WO 91/02805); influenza virus (Luytjes et al., Cell 59:1107-1113, 1989; McMicheal et al., N. Eng. J. Med. 309:13-17, 1983; and Yap et al., Nature 273:238-239, 1978); adenovirus (Berkner, Biotechniques 6:616-627, 1988; Rosenfeld et al., Science 252:431-434, 1991; WO 93/9191; Kolls et al., PNAS 91:215-219, 1994; Kass-Eisler et al., PNAS 90:11498-11502, 1993; Guzman et al., Circulation 88:2838-2848, 1993; Guzman et al., Cir. Res. 73:1202-1207, 1993; Zabner et al., Cell 75:207-216, 1993; Li et al., Hum. Gene. Ther. 4:403-409, 1993; Caillaud et al., Eur. J. Neurosci. 5:1287-1291, 1993; Vincent et al., Nat. Genet. 5:130-134, 1993; Jaffe et al., Nat. Genet. 1:372-378, 1992; and Levrero et al., Gene 101:195-202, 1991); parvovirus such as adeno-associated virus (Samulski et al., J. Vir. 63:3822-3828, 1989; Mendelson et al., Virol. 166:154-165, 1988; PA 7/222,684); herpes (Kit, Adv. Exp. Med. Biol. 215:219-236, 1989); SV40; HIV (Poznansky, J. Virol. 65:532-536, 1991); measles (EP 0 440,219); astrovirus (Munroe, S. S. et al., J. Vir. 67:3611-3614, 1993); Semlild Forest Virus, and coronavirus, as well as other viral systems (e.g., EP 0,440,219; WO 92/06693; U.S. Pat. No. 5,166,057). In addition, viral carriers may be homologous, non-pathogenic(defective), replication competent viruses (e.g., Overbaugh et al., Science 239:906-910, 1988) that nevertheless induce cellular immune responses, including cytotoxic T-cell lymphocytes (CTL).

The term "ex vivo administration" refers to transfecting or administering a substance to a cell, for example a cell from a population of cells that are exhibiting aberrant SHIP activity, after the cell is removed from the mammal. After transfection or administration of the substance, the cell is then replaced in the mammal. Ex vivo administration can be accomplished by removing cells from a mammal, optionally selecting cells to transform, rendering the selected cells incapable of replication, transforming or treating the selected cells with a polynucleotide or other means for modulating SHIP activity, and placing the transformed or treated cells back into the mammal.

"Administration" or "administering" as used herein refers to the process of delivering to a mammal a therapeutic agent, or a combination of therapeutic agents. The process of administration can be varied, depending on the therapeutic agent, or agents, and the desired effect. Administration can be accomplished by any means appropriate for the therapeutic agent, for example, by parenteral, mucosal, pulmonary, topical, catheter-based, or oral means of delivery. Parenteral delivery can include, for example, subcutaneous, intravenous, intramuscular, intraarterial, and injection into the tissue of an organ. Mucosal delivery can include, for example, intranasal delivery. Pulmonary delivery can include inhalation of the agent. Catheter-based delivery can include delivery by iontophoretic catheter-based delivery. Oral delivery can include delivery of an enteric coated pill, or administration of a liquid by mouth. Administration will generally also include delivery with a pharmaceutically acceptable carrier, such as, for example, a buffer, a polypeptide, a peptide, a polysaccharide conjugate, a liposome and/or a lipid. Gene therapy protocol is considered an administration in which the therapeutic agent is a polynucleotide capable of accomplishing a therapeutic goal when expressed as a transcript or a polypeptide in the mammal.

A "nucleic acid" or a "polynucleotide," as used herein, refers to either RNA or DNA molecule that encodes a specific amino acid sequence or its complementary strand. Nucleic acid molecules may also be non-coding sequences, for example, a ribozyme, an antisense oligonucleotide, or an untranslated portion of a gene. A "coding sequence" as used herein, refers to either RNA or DNA that encodes a specific amino acid sequence, or its complementary strand. A polynucleotide may include, for example, an antisense oligonucleotide, or a ribozyme, and can also include such items as a 3' or 5' untranslated region of a gene, or an intron of a gene, or other region of a gene that does not make up the coding region of the gene. The DNA or RNA may be single stranded or double stranded. Synthetic nucleic acids or synthetic polynucleotides can be chemically synthesized nucleic acid sequences, and can also be modified with chemical moieties to render the molecule resistant to degradation. Synthetic nucleic acids can be ribozymes or antisense molecules, for example. Modifications to synthetic nucleic acid molecules include nucleic acid monomers or derivative or modifications thereof, including chemical moieties, such as, for example, phosphothioate modification. A polynucleotide derivative can include, for example, such polynucleotides as branched DNA (bDNA). A polynucleotide can be a synthetic or recombinant polynucleotide, and can be generated, for example, by polymerase chain reaction (PCR) amplification, or recombinant expression of complementary DNA or RNA, or by chemical synthesis.

The term "an expression control sequence" or a "regulatory sequence" refers to a sequence that is conventionally used to effect expression of a gene that encodes a polypeptide and include one or more components that affect expression, including transcription and translation signals. Such a sequence includes, for example, one or more of the following: a promoter sequence, an enhancer sequence, an upstream activation sequence, a downstream termination sequence, a polyadenylation sequence, an optimal 5' leader sequence to optimize initiation of translation in mammalian cells, a Kozak sequence, which identifies optimal residues around initiator AUG for mammalian cells. The expression control sequence that is appropriate for expression of the present polypeptide differs depending upon the host system in which the polypeptide is to be expressed. For example, in prokaryotes, such a control sequence can include one or more of a promoter sequence, a Shine-Dalgarno sequence, a ribosomal binding site, and a transcription termination sequence. In eukaryotes, for example, such a sequence can include a promoter sequence, and a transcription termination sequence. If any necessary component of an expression control sequence is lacking in the nucleic acid molecule of the present invention, such a component can be supplied by the expression vector to effect expression. Expression control sequences suitable for use herein may be derived from a prokaryotic source, an eukaryotic source, a virus or viral vector or from a linear or circular plasmid. Further details regarding expression control sequences are provided below. An example of a regulatory sequence is the human immunodeficiency virus ("HIV-1") promoter that is located in the U3 and R region of the HIV-1 long terminal repeat ("LTR"). Alternatively, the regulatory sequence herein can be a synthetic sequence, for example, one made by combining the UAS of one gene with the remainder of a requisite promoter from another gene, such as the GADP/ADH2 hybrid promoter.

"Hybridization" refers to the association of two nucleic acid sequences to one another by specific hydrogen bonding. Typically, one sequence can be fixed to a solid support and the other is free in solution. The two sequences are placed in contact with one another under conditions that favor hydrogen bonding. Factors that affect this binding bonding include: the type and volume of solvent; reaction temperature; time of hybridization; agitation; agents to block the non-specific attachment of the liquid phase sequence to the solid support (Denhardt's reagent or BLOTTO); concentration of the sequences; use of substances to increase the rate of association of sequences (dextran sulfate or polyethylene glycol); and, the stringency of the washing conditions following hybridization. See Sambrook et al. MOLECULAR CLONING; A LABORATORY MANUAL, SECOND EDITION (1989), Volume 2, chapter 9, pages 9.47 to 9.57. "Stringency" refers to conditions in a hybridization reaction that favor association of very similar sequences over sequences that differ. For example, the combination of temperature and salt concentration should be chosen that is approximately 12.degree. to 20.degree. C. below the calculated Tm of the hybrid under study.

The term "naked DNA" refers to polynucleotide DNA for administration to a mammal for expression in the mammal or to inhibit SHIP activity. The polynucleotide can be, for example, a coding sequence, and the polynucleotide DNA can be directly or indirectly connected to an expression control sequence that can facilitate the expression of the coding sequence once the DNA is inside a cell. Alternatively, the DNA can direct production of RNA or a polypeptide that inhibits SHIP activity.

"Recombinant retroviral vector" refers to an assembly which is capable of directing the expression of a sequence(s) or gene(s) of interest. Preferably, the retroviral vector construct should include a 5' LTR, a tRNA binding site, a packaging signal, one or more heterologous sequences, an origin of second strand DNA synthesis and a 3' LTR. A wide variety of heterologous sequences may be included within the vector construct, including for example, sequences which encode a protein (e.g., cytotoxic protein, disease-associated antigen, immune accessory molecule, or replacement protein), or which are useful in and of themselves (e.g., as ribozymes or antisense sequences). Alternatively, the heterologous sequence may merely be a "stuffer" or "filler" sequence of a size sufficient to allow production of retroviral particles containing the RNA genome. Preferably, the heterologous sequence is at least 1, 2, 3, 4, 5, 6, 7 or 8 Kb in length. The retroviral vector construct may also include transcriptional promoter/enhancer or locus defining element(s), or other elements which control gene expression by means such as alternate splicing, nuclear RNA export, post-translational modification of messenger, or post-transcriptional modification of protein. Optionally, the retroviral vector construct may also include selectable markers that confer resistance of recombinant retroviral vector, transduced or transfected, cells to TK, hygromycin, phleomycin, histidinol, or DHFR, as well as one or more specific restriction sites and a translation termination sequence.

A "therapeutically effective amount" is that amount that will generate the desired therapeutic outcome. For example, if the therapeutic effect desired is reduction or suppression of rejection of a transplant, the therapeutically effective amount is that amount that facilitates reduction or suppression of rejection of a transplant. A therapeutically effective amount can be an amount administered in a dosage protocol that includes days or weeks of administration.

The term "pharmaceutically acceptable carrier" refers to a carrier for administration of a therapeutic agent, such as, for example, a polypeptide, polynucleotide, small molecule (preferably a molecule having a molecular weight of less than about 10,000), peptoid, or peptide, refers to any pharmaceutically acceptable carrier that does not itself induce the production of antibodies harmful to the individual receiving the composition, and which may be administered without undue toxicity.

"Vector construct" refers to an assembly which is capable of directing the expression of the sequence(s) or gene(s) of interest. The vector construct can include transcriptional promoter/enhancer or locus defining element(s), or other elements which control gene expression by other means such as alternate splicing, nuclear RNA export, post-translational modification of messenger, or post-transcriptional modification of protein. In addition, the vector construct must include a sequence which, when transcribed, is operably linked to the sequence(s) or gene(s) of interest and acts as a translation initiation sequence. Optionally, the vector construct may also include a signal which directs polyadenylation, a selectable marker such as Neo, TK, hygromycin, phleomycin, histidinol, or DHFR, as well as one or more restriction sites and a translation termination sequence. In addition, if the vector construct is placed into a retrovirus, the vector construct must include a packaging signal, long terminal repeats (LTRs), and positive and negative strand primer binding sites appropriate to the retrovirus used (if these are not already present).

"Tissue-specific promoter" refers to transcriptional promoter/enhancer or locus defining elements, or other elements which control gene expression as discussed above, which are preferentially active in a limited number of tissue types. Representative examples of such tissue-specific promoters include the PEP-CK promoter, HER2/neu promoter, casein promoter, IgG promoter, Chorionic Embryonic Antigen promoter, elastase promoter, porphobilinogen deaminase promoter, insulin promoter, growth hormone factor promoter, tyrosine hydroxylase promoter, albumin promoter, alphafetoprotein promoter, acetyl-choline receptor promoter, alcohol dehydrogenase promoter, a or P globin promoters, T-cell receptor promoter, or the osteocalcin promoter.

"Mammalian cell" as used herein refers to a subset of eukaryotic cells useful as host cells, and includes human cells, and animal cells such as those from dogs, cats, cattle, horses, rabbits, mice, goats, pigs, etc. The cells used can be genetically unaltered or can be genetically altered, for example, by transformation with appropriate expression vectors, marker genes, and the like. Mammalian cells suitable for the method described herein are any mammalian cell capable of expressing the genes of interest, or any mammalian cells that can express a cDNA library, cRNA library, genomic DNA library or any protein or polypeptide useful in the method of the invention. Mammalian cells also include cells from cell lines such as those immortalized cell lines available from the American Type Culture Collection (ATCC). Such cell lines include, for example, rat pheochromocytoma cells (PC12 cells), embryonal carcinoma cells (P19 cells), Chinese hamster ovary (CHO) cells, HeLa cells, baby hamster kidney (BHK) cells, monkey kidney cells (COS), human hepatocellular carcinoma cells (e.g., Hep G2), human embryonic kidney cells, mouse sertoli cells, canine kidney cells, buffalo rat liver cells, human lung cells, human liver cells, mouse mammary tumor cells, as well as others. Also included are hematopoetic stem cells, neuronal stem cells such as neuronal sphere cells, and pluripotent or embryonic stem cells (ES cells).

The term "antagonist" as used herein refers to a molecule that blocks signaling, such as for example a molecule that can bind a receptor, but which does not cause a signal to be transduced by the receptor to the cell. In the case of inositol polyphosphatase 5'-phosphatases an antagonist might block signaling by binding, for example, at an SH2 domain on the molecule, or by binding, for example, so as to inhibit its phosphatase activity. In general, an antagonist of a polypeptide is an inhibitor of any biological activity of the polypeptide. A given inhibitor or agonist may target and inhibit one biological activity, while not affecting another non-target activity of the molecule.

A suitable SHIP1 inhibitor for use in certain methods described herein can include, without limitation, the following SHIP inhibitor compound:

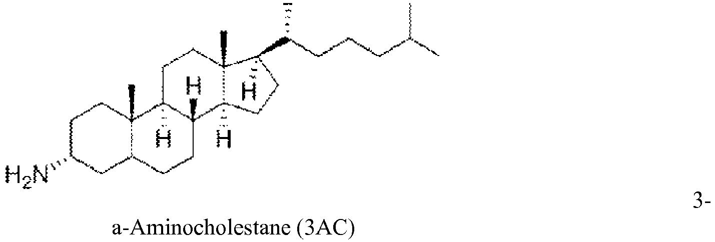

Other suitable SHIP1 inhibitors for use in methods described herein can include, without limitation, the SHIP inhibitor compounds of the formula (I), and pharmaceutically acceptable salts thereof, where formula (I) is as follows: 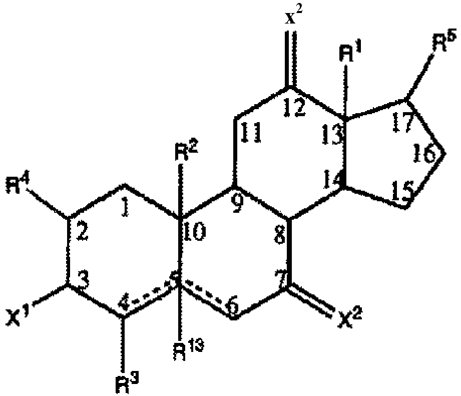 wherein:
----- at the 4,5 and 5,6 positions represents a single or double bond, with the proviso that the sum of double bonds present at the 4,5 and 5,6 positions is 0 or 1.
R¹ is a straight chain C₁-C₄ alkyl or C₁-C₄ haloalkyl. In one example, R¹ is methyl.
R² is hydrogen, methyl, or halomethyl. In one embodiment, R² is methyl.
R³ and R¹³ (when present), are individually selected from hydrogen, substituted or unsubstituted amino, C₁-C₄ alkyl, C₁-C₄ haloalkyl, and C₁-C₄ alkenyl. In one example, both R³ and R¹³ are hydrogen.
R⁴ is hydrogen, hydroxy, substituted or unsubstituted amino, alkyl, or benzyl. In one example, R⁴ is hydrogen.
R⁵ represents a divalent oxo atom, hydrogen, or an alkyl group. In one example, R⁵ represents an alkyl group. In one embodiment, the alkyl group is 1, 5-dimethylhexyl.
X¹ may be selected from the group consisting of hydrogen, hydroxy, mercapto, alkoxy, aryloxy, alkylthio, and arylthio. The alkoxy, aryloxy, alkylthio, and arylthio moieties may be further substituted.
X¹ may also be selected from the group consisting of alkylcarbonamido, arylcarbonamido, aminocarbonamido, hydrazinocarbonamido, alkylsulfonamido, arylsulfonamido, aminosulfonamido, and hydrazinosulfonamido, all of which may be further substituted.
X¹ may also be selected from the group consisting of (C₁-C₄ alkyl)carbonyloxy, (C₁-C₄ alkoxy)carbonyloxy, arylcarbonyloxy, aryloxycarbonyloxy, and aminocarbonyloxy, all of which may be further substituted.
X¹ may further be selected from the group consisting of a substituted or unsubstituted amino and secondary and tertiary amino groups that include at least one C₁-C₄ alkyl, C₅-C₆ cycloalkyl, aryl, or heterocyclic substituent, or combinations thereof. In one example, the secondary or tertiary amino group contains at least one C₁-C₄ alkyl moiety, which may be further substituted.
X¹ may further be an aminoalkyl group, amino(CH₂)ₙ, where "amino" is an unsubstituted or a substituted secondary or tertiary amino as defined above, and n is an integer from 1 to 4.
X¹ may further represent a divalent oxygen moiety, =O, or a divalent N-hydroxyamino moiety, =NOH.
X¹ may further be an amino group, except when: R¹ and R² are each methyl; X², R³, R⁴, and R¹³ are each hydrogen; and R⁵ represents one hydrogen atom together with an alkyl group, where the alkyl group is 1, 5-dimethylhexyl alkyl group.
Each X² is independently defined to represent a divalent oxo or two hydrogen atoms. In one embodiment, each X² represents two hydrogen atoms.

The compounds for use in the present invention, as will be appreciated by one skilled in the art, possess several potential chiral carbon atoms. As a consequence of these chiral centers, the compounds for use in the present invention may occur as racemates, racemic mixtures, individual diastereomers and substantially pure isomers. All asymmetric forms, individual isomers, and combinations thereof, are within the scope of the present invention.

Throughout this specification, the terms and substituents retain their definitions. Below are particular definitions of terms used herein.

The term "alkyl" by itself or as part of another substituent means, unless otherwise stated, a straight or branched chain hydrocarbon radical and includes straight or branch chain groups such as methyl, ethyl, n-propyl, isopropyl, n-butyl, t-butyl, isobutyl, sec-butyl, and higher homologs and isomers such as n-pentyl, n-hexyl, 2-methylpentyl, 1,5-dimethylhexyl, 1-methyl-4-isopropyl, hexyl and the like. Preferred alkyl groups are those of C₂₀ or below (i.e., C₁₋₂₀). A divalent radical derived from an alkane is exemplified by -CH₂CH₂CH₂CH₂-. A divalent radical derived from an alkene is exemplified by -CH=CH-CH₂-. An example of a non-limiting subset of alkyl is alkyl groups of from 1 to 10 carbon atoms (C₁₋₁₀ alkyl) (e.g., 1, 2, 3, 4, 5, 6, 7, 8, 9, or 10 carbon atoms).

The term "alkenyl", employed alone or in combination with other terms, means a straight chain or branched monounsaturated hydrocarbon group having the stated number of carbon atoms, such as, for example, vinyl, propenyl (allyl), crotyl, isopentenyl, and the various butenyl isomers.

Alkyl and alkenyl groups may include substitutents selected from the group consisting of halo, hydroxy, cyano, mercapto, -S(C₁-C₄ alkyl), amino, substituted amino, acetamido, carboxy, trifluoromethyl, C₁-C₄ alkoxy, (C₁-C₄ alkoxy)carbonyl and aminocarbonyl.

The term "cycloalkyl" means an unsubstituted or substituted monovalent saturated cyclic hydrocarbon radical having the stated number of carbon atoms, including, various isomers of cyclopentyl and cyclohexyl. The term "cycloalkenyl" means an unsubstituted or substituted monovalent monounsaturated cyclic hydrocarbon radical having the stated number of carbon atoms, including, various isomers of cyclopentenyl and cyclohexenyl. The term "cycloalkadienyl" means a monovalent diunsaturated cyclic radical having the stated number of carbon atoms, including, the various isomers of cyclopentadienyl and cyclohexadienyl. The substituents can be one or two of the same or different substituents selected from halo, hydroxy, cyano, mercapto,-S(C₁-C₄ alkyl), amino, substituted amino, acetamido, carboxy, trifluoromethyl, C₁-C₄ alkoxy, (C₁-C₄ alkoxy)carbonyl and aminocarbonyl.

The dotted lines between the 4,5 and 5,6 positions represent the presence or absence of an additional bond; that is, an unsaturation. Only one unsaturation can be present at any one time. The R¹³ shown in Formula (I) will, of course, be absent when an unsaturation is present.

The term "aryl" means an unsubstituted or substituted monovalent phenyl group. The substituents may be independently selected from halo, -OH, -SH, -S(C₁-C₄)alkyl), C₁-C₅ alkyl, C₁-C₅ alkoxy, carboxy, (C₁-C₄ alkoxy)carbonyl, aminocarbonyl, C₁-C₄ alkylaminocarbonyl, amino, acetamido, C₁-C₄ alkylamino, di(C₁-C₄ alkyl)amino or a group -(CH₂)_{q}-R where q is 1, 2, 3, or 4 and R is hydroxy, C₁-C₄ alkoxy, carboxy, C₁-C₄ alkoxycarbonyl, amino, aminocarbonyl, C₁-C₄ alkylamino or di(C₁-C₄ alkyl)amino.

The term "benzyl" means a monovalent group in which a phenyl moiety is substituted by a methylene group. The benzyl group may include further substituents on the phenyl moiety.

The term "amino" means a group -NH₂. The term, "substituted amino" means an amino group where one or both amino hydrogens are independently replaced by a C₁-C₄ alkyl, C₂-C₄ alkenyl, C₅-C₆ cycloalkyl, C₅-C₆ cycloalkenyl, aryl, benzyl, or a group -(CH₂)_{q}-R where q is 1, 2, 3, or 4 and R is hydroxy, C₁-C₄ alkoxy, carboxy, C₁-C₄ alkoxycarbonyl, amino, aminocarbonyl, C₁-C₄ alkylamino or di(C₁-C₄ alkyl)amino.

The term "alkylcarbonamido" means a group (C₄-C₄ alkyl)C(O)N(R)-, where R represents H or C₁-C₄ alkyl. More specifically, the term "acetamido" means a group CH₃C(O)NH-. The term "arylcarbonamido" means a group (aryl)C(O)N(R)-, where R represents H or C₁-C₄ alkyl. The term "aminocarbonamido" means a group R'R"NC(O)N(R)-, where R represents H or C₁-C₄ alkyl, and R' and R" independently represent H, C₁-C₄ alkyl, C₅-C₆ cycloalkyl, aryl, or heterocyclic.

The term "alkylsulfonamido" means a group (C₁-C₄ alkyl)SO₂N(R)-, where R represents H or C₁-C₄ alkyl. The term "arylsulfonamido" means a group (aryl)SO₂N(R)-, where R represents H or C₁-C₄ alkyl. The term "aminosulfonamido" means a group R'R"NHSO₂N(R)-, where R represents H or C₁-C₄ alkyl, and R' and R" independently represent H, C₁-C₄ alkyl, C₅-C₆ cycloalkyl, aryl, or heterocyclic.

The term "alkylcarbonyloxy" means a group (C₁-C₄ alkyl)C(O)O-. The term "alkoxycarbonyloxy" means a group (C₁-C₄ alkyl)OC(O)O-. The term "arylcarbonyloxy" means a group (aryl)C(O)O-. The term "aryloxycarbonyloxy" means a group (aryl)OC(O)O-. The term "aminocarbonyloxy" means a group R'R"NC(O)O-, where R' and R" independently represent H, C₁-C₄ alkyl, C₅-C₆ cycloalkyl, aryl, or heterocyclic.

The term "halo" means chloro, bromo, fluoro or iodo. The term "mercapto" means a group -SH.

The term "heterocycle" means an unsubstituted or substituted stable 5- or 6-membered monocyclic heterocyclic ring that consists of carbon atoms and from one to three heteroatoms selected from the group consisting of N, O and S, and wherein the nitrogen and sulfur heteroatoms may optionally be oxidized, and the nitrogen heteroatom may optionally be quaternized. The heterocyclic ring may be attached, unless otherwise stated, at any heteroatom or carbon atom that affords a stable structure. The heterocycle may be unsubstituted or substituted with one or two substituents.

The compound of formula (I) may be a compound of a formula as set forth below: 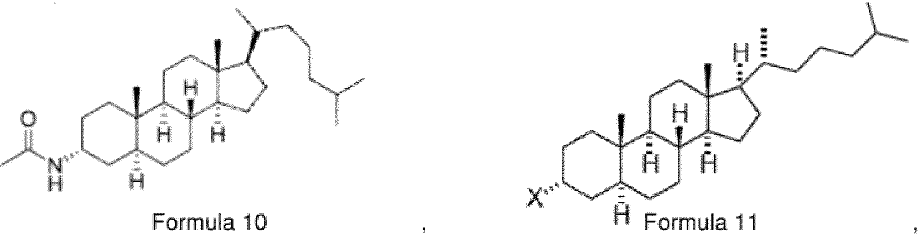 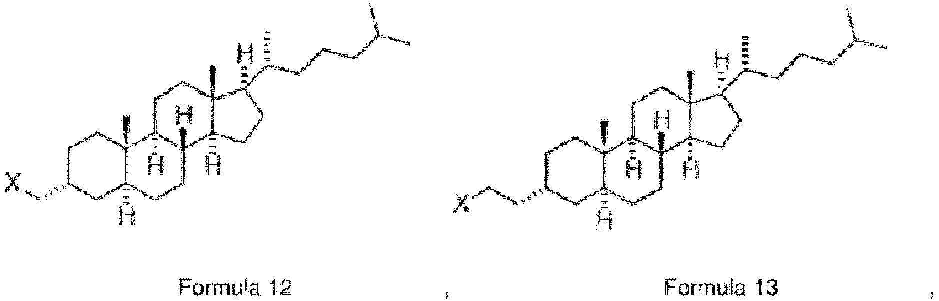 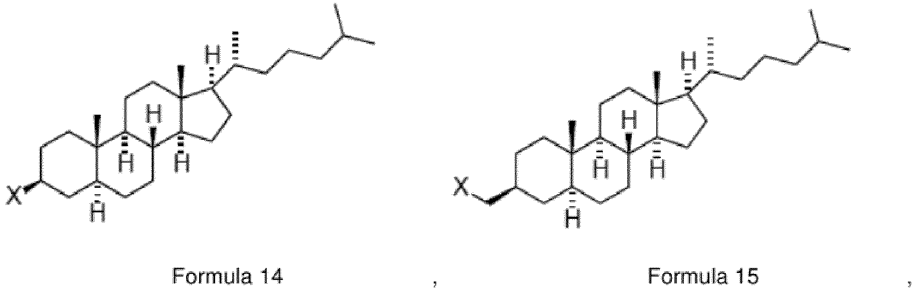 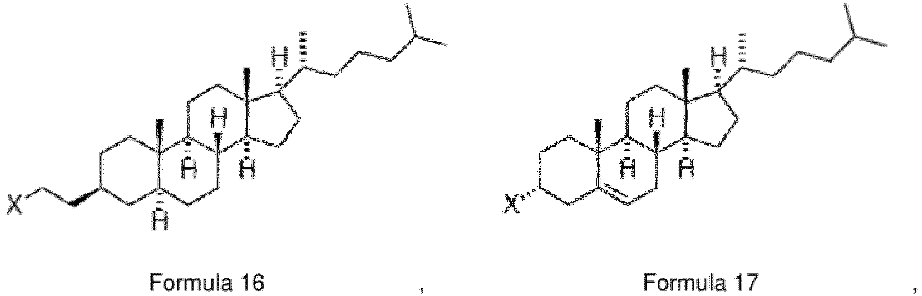 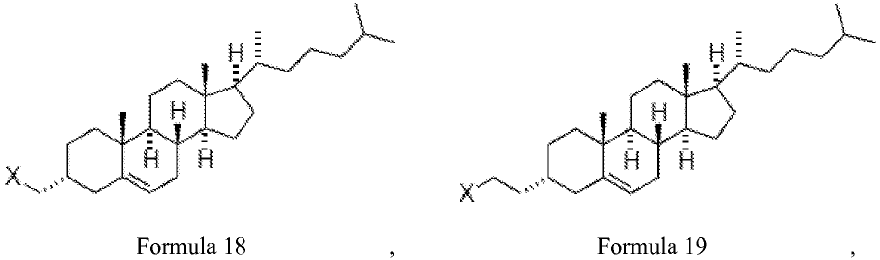 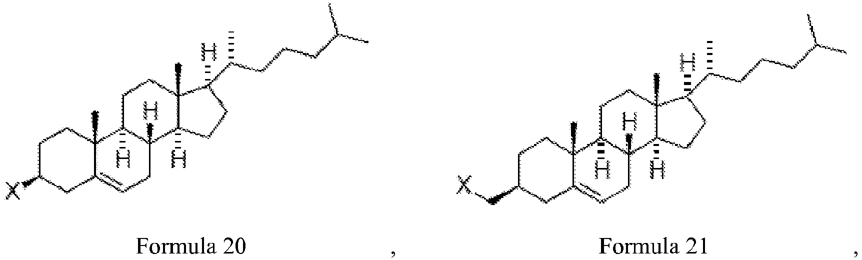 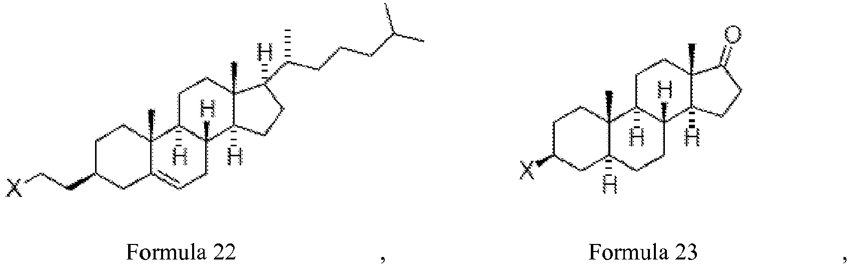 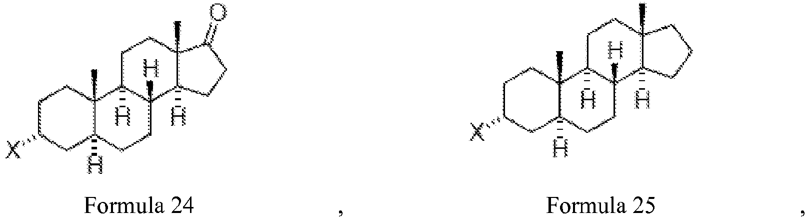 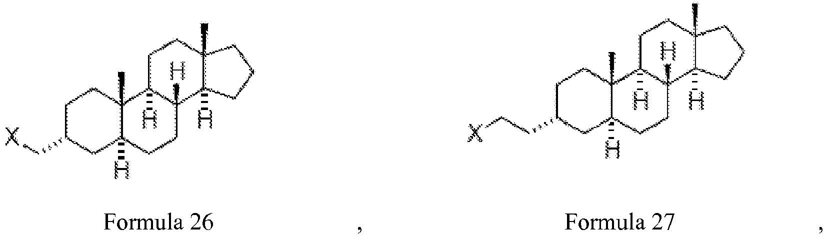 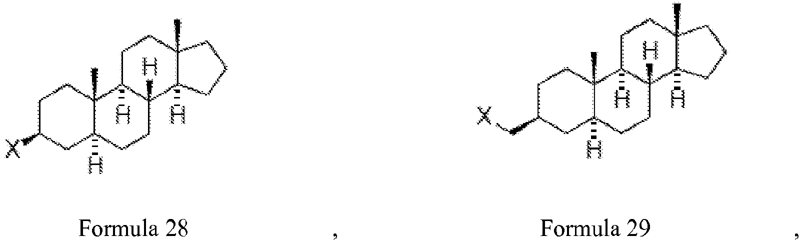 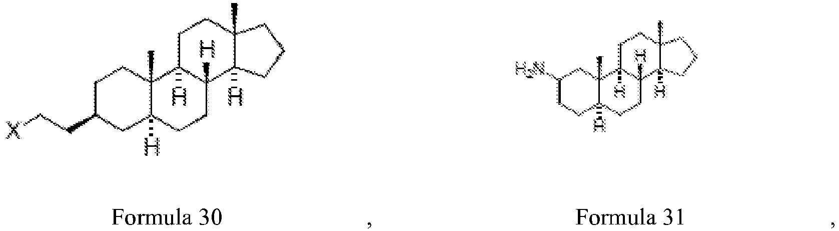 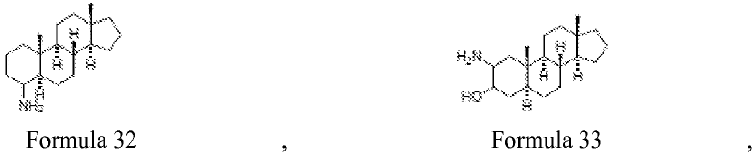 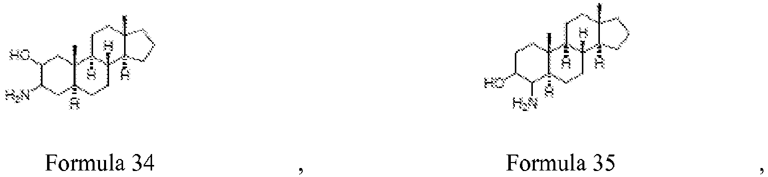 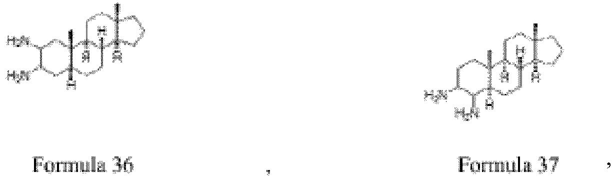 and pharmaceutically acceptable salts thereof, wherein X = NR₂, NRCOR, NHCONR₂, OR, SR, OCOR, OCONR₂, or NHCNHNH₂, and wherein R = H, alkyl, cycloalkyl, aryl, or benzyl.

The compound of Formula (I) or pharmaceutically acceptable salt thereof may be a compound of Formula (IA) or a pharmaceutically acceptable salt thereof:
wherein 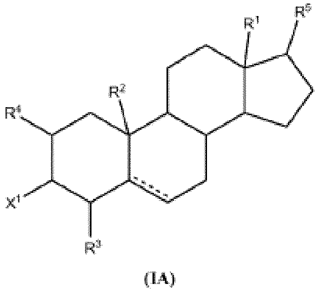
   ----- represents a single or double bond (in particular embodiments, ----- represents a single bond);
   R¹ and R² are individually selected from hydrogen and C₁-₃ alkyl (e.g., methyl);
   R³ is selected from hydrogen and amino;
   R⁴ is selected from hydrogen, amino, and hydroxy;
   R⁵ is selected from hydrogen, a divalent oxo atom, and C₁₋₁₀ alkyl (e.g., C₁-C₈ alkyl, such as, for example, C₈ alkyl, e.g., 1, 5-dimethylhexyl); and
   X¹ is selected from hydrogen, amino, and hydroxy. In some embodiments, X¹ is selected from hydrogen and amino. In a particular embodiment, X¹ is amino.

The compound of Formula (IA) or salt thereof may be selected from a compound of Formula (IB) or (IC) below, or a pharmaceutically acceptable salt thereof:

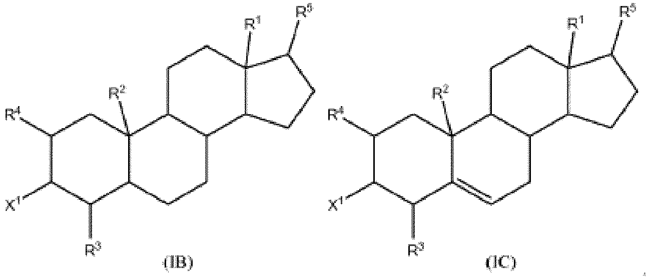

The compound of Formula (IA) or salt thereof may be selected from a compound of Formula (ID)-(IO) below, or a pharmaceutically acceptable salt thereof:

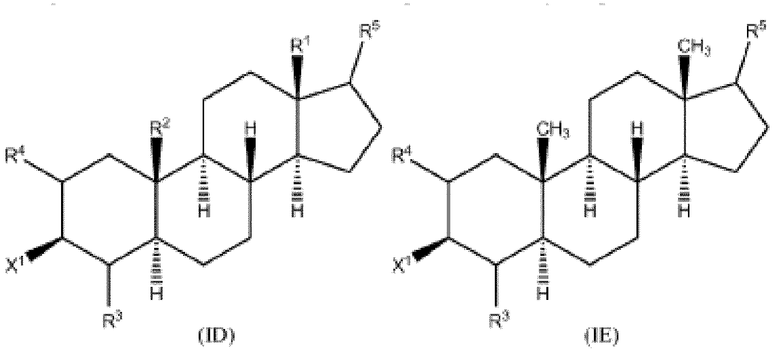

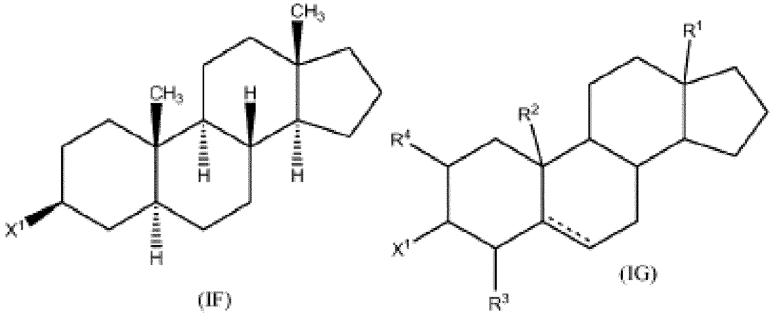

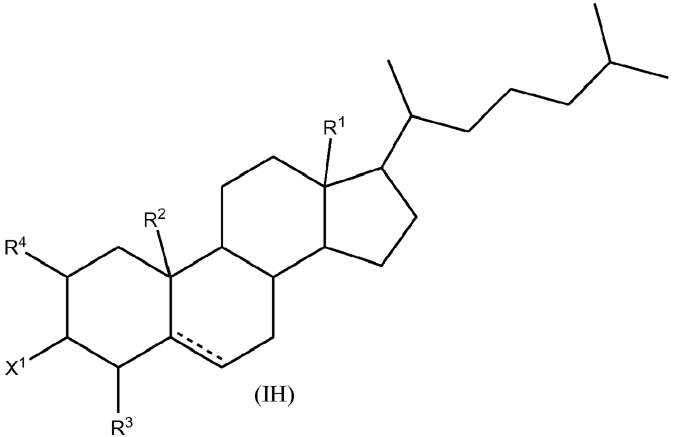

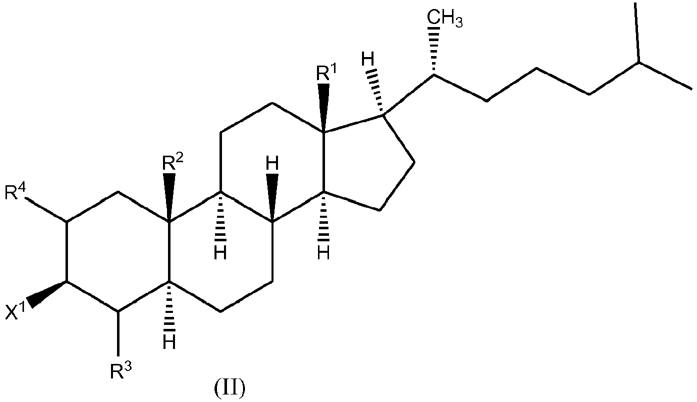

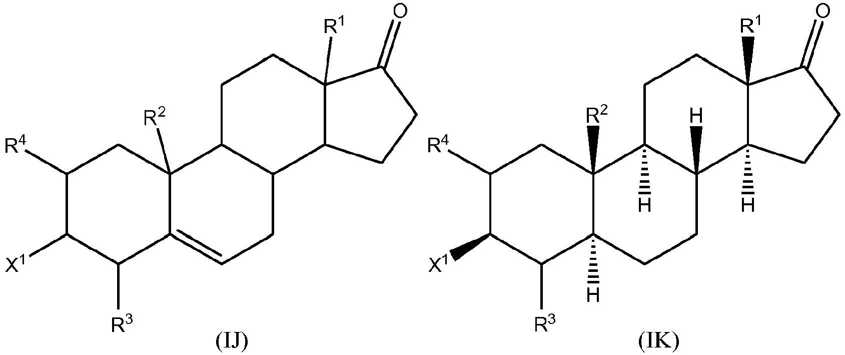

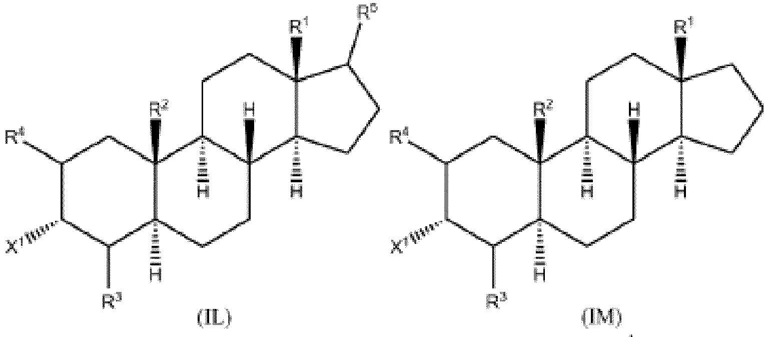

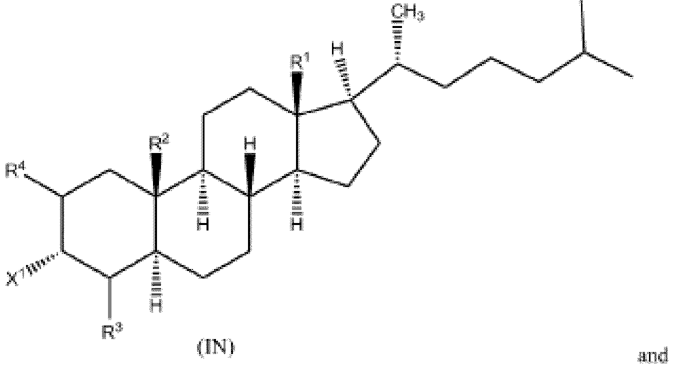

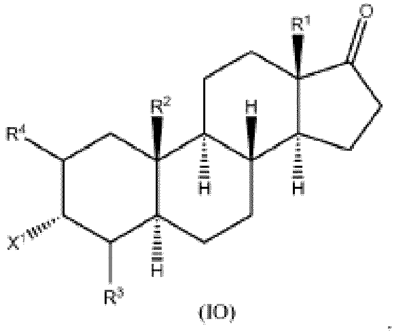

Described is a method comprising administering a pharmaceutically acceptable salt of a compound according to any one of Formulas (I) or (IA)-(IO). Optionally, the pharmaceutically acceptable salt is a hydrochloride salt. Optionally, the pharmaceutically acceptable salt is a salt of a compound wherein X¹ is amino (for example, a hydrochloride salt of such a compound, e.g., the pharmaceutically acceptable salt may be a compound having NH₃Cl at the X¹ position).

Optionally, the method comprises administering a compound of Formula (I) (or any sub-genus thereof) as described herein, or a pharmaceutically acceptable salt thereof, with the proviso that if X¹ is hydroxy, then R⁴ is a hydrogen, substituted or unsubstituted amino, C₁-C₄ alkyl, or benzyl.

Optionally, the method comprises administering a compound of Formula (I) (or any sub-genus thereof) as described herein, or a pharmaceutically acceptable salt thereof, with the proviso that if X¹ is hydroxy, at least one of R³ and R⁴ is other than hydrogen.

Optionally, the method comprises administering a compound of Formula (I) (or any sub-genus thereof) as described herein, or a pharmaceutically acceptable salt thereof, with the proviso that if X¹ is hydroxy, R⁵ is not an alkyl group.

Optionally, the method comprises administering a compound selected from one of the following:

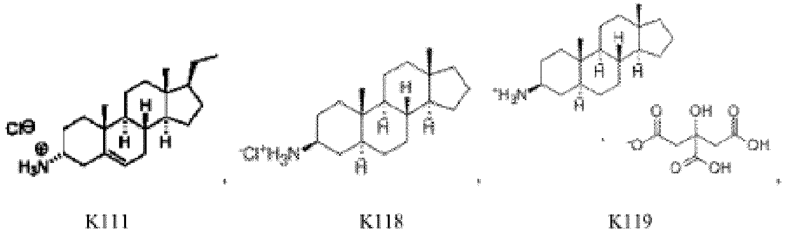

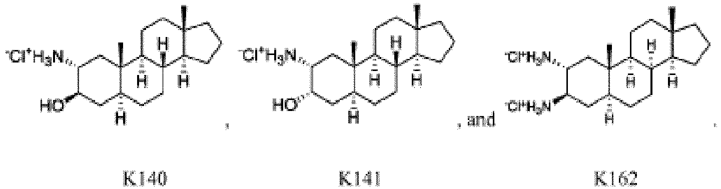

The "SHIP inhibitor compounds" of the disclosure are also referred to herein as "SHIP inhibitors," "SHIP1 inhibitors," "SHIP1 inhibitor compounds," "pan-SHIP1/2 inhibitors," and the like. In one option, the SHIP inhibitor compounds are selective inhibitors of SHIP1.

As used herein, suitable pan-SHIP1/2 inhibitors for use in the methods described herein can include, without limitation, the pan-SHIP1/2 inhibitor compounds as follows:

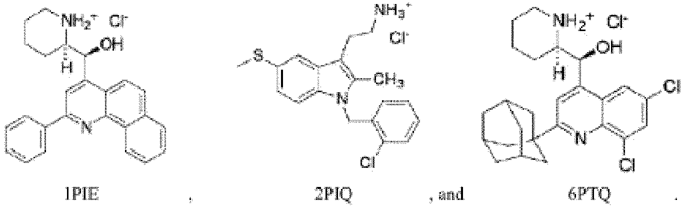

Various aspects and embodiments of the present invention as they relate to the pan-SHIP1/2 inhibitors are shown in Figures 1-6 and are further described in the Examples and the associated figures and tables provided herewith in connection with the Examples.

As used herein, in other options, suitable SHIP I inhibitors for use in the methods described herein can include, without limitation, small interfering RNAs (siRNAs) or microRNAs (miRNAs) that are effective to inhibit SHIP1 via RNA interference (RNAi) (post transcriptional gene silencing).

RNAi technology provides an efficient means for blocking expression of a specific gene. RNAi technology takes advantage of the cell's natural machinery, facilitated by short interfering RNA molecules, to effectively knock down expression of a gene of interest. There are several ways to induce RNAi, synthetic molecules, siRNA, miRNA, RNAi vectors, and *in vitro* dicing.

RNAi can be used to inhibit the SHIP1 genes, such as by creating siRNAs or miRNAs having the appropriate sequence and delivering them to the cells in which inhibition of the SHIP1 gene is desired. A key area of research in the use of RNAi for clinical applications is the development of a safe delivery method, which to date has involved mainly viral vector systems similar to those suggested for gene therapy. Once developed, these delivery methods can be used for the purposes of the present invention. RNAi inducing agents can also be delivered using bacteria, retroviruses, DNA viruses, lipidoids and amphoteric liposomes.

General rules for selecting siRNA targets on mRNA sequences include, for example, the following (www.rnaiweb.com/RNAi/siRNA_Design/): (i) Targets should be located 50-100 nt downstream of the start codon (ATG); (ii) Search for sequence motif AA(N₁₉)TT or NA(N₂₁), or NAR(N₁₇)YNN, where N is any nucleotide, R is purine (A, G) and Y is pyrimidine (C, U); (iii) Target sequences should have a G+C content between 35-60%; (iv) Avoid stretches of 4 or more nucleotide repeats; (v) Avoid 5'URT and 3'UTR, although siRNAs targeting UTRs have been shown to successfully induce gene silencing; and (vi) Avoid sequences that share a certain degree of homology with other related or unrelated genes.

Selecting targets for miRNA: In animals, the tendency of miRNAs to bind their mRNA targets with imperfect sequence homology poses considerable challenges with target prediction. In animals, target sites are often only partially complementary to their miRNAs and are mostly located in the 3'UTR of target genes. Several computational approaches have been developed to facilitate experimental design and predicting miRNA targets. In general, computational target prediction identifies potential binding sites according to base-pairing rules and cross species conservation conditions.

The dosage form of the SHIP inhibitor described herein may be a liquid solution ready for use or intended for dilution with a preservation solution. Alternatively, the dosage form may be lyophilized or power filled prior to reconstitution with a preservation solution. The lyophilized substance may contain, if suitable, conventional excipients.

Other than in the operating examples, or unless otherwise expressly specified, all of the numerical ranges, amounts, values and percentages such as those for amounts of materials, times and temperatures of reaction, ratios of amounts, values for molecular weight (whether number average molecular weight ("Mₙ") or weight average molecular weight ("M_{w}"), and others in the following portion of the specification may be read as if prefaced by the word "about" even though the term "about" may not expressly appear with the value, amount or range. Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the disclosure are approximations, the numerical values set forth in the specific examples are reported as precisely as possible. Any numerical value, however, inherently contain certain errors necessarily resulting from the standard deviation found in their respective testing measurements. Furthermore, when numerical ranges of varying scope are set forth herein, it is contemplated that any combination of these values inclusive of the recited values may be used.

As used herein, the term "pretreating" (or "pretreatment") is intended to mean that a first treatment is administered prior to, or in conjunction with, a second treatment. In other words, the pretreatment may be performed before another, later treatment, thus allowing the pretreatment time to take effect. Alternatively, the pretreatment may be performed or administered simultaneously with a second treatment without a temporal delay. Advantageously, a pretreatment is administered prior to a second treatment.

The term "administration" and variants thereof (e.g., "administering" a compound) in reference to a compound for use in the invention can also mean introducing the compound into the system of the animal in need of treatment. When a compound for use in the invention is provided in combination with one or more other active agents (e.g., a cytotoxic agent, etc.), "administration" and its variants are each understood to include concurrent and sequential introduction of the compound and other agents.

As used herein, the term "composition" is intended to encompass a product comprising the specified ingredients in the specified amounts, as well as any product which results, directly or indirectly, from combination of the specified ingredients in the specified amounts.

The term "therapeutically effective amount" as used herein can also means that amount of active compound or pharmaceutical agent that elicits the biological or medicinal response in a tissue, system, animal or human that is being sought by a researcher, veterinarian, medical doctor or other clinician.

A "subject in need of treatment" is a mammal with a bone-loss condition.

A "pharmaceutically acceptable" component is one that is suitable for use with humans and/or animals without undue adverse side effects (such as toxicity, irritation, and allergic response) commensurate with a reasonable benefit/risk ratio.

A "safe and effective amount" refers to the quantity of a component that is sufficient to yield a desired therapeutic response without undue adverse side effects (such as toxicity, irritation, or allergic response) commensurate with a reasonable benefit/risk ratio when used in the manner of this invention.

A "pharmaceutically acceptable carrier" can also refer to a carrier, such as a solvent, suspending agent or vehicle, for delivering the compound or compounds in question to the animal or human. The carrier may be liquid or solid and is selected with the planned manner of administration in mind. Liposomes are also a pharmaceutical carrier. As used herein, "carrier" includes any and all solvents, dispersion media, vehicles, coatings, diluents, antibacterial and
antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated.

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this invention.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods. See, generally, Sambrook et al., Molecular Cloning: A Laboratory Manual, 2d ed. (1989) Cold Spring Harbor Laboratory Press, Cold Spring Harbor, N.Y. and Ausubel et al., Short Protocols in Molecular Biology (1999) 4th Ed, John Wiley & Sons, Inc.; as well as Guthrie et al., Guide to Yeast Genetics and Molecular Biology, Methods in Enzymology, Vol. 194, Academic Press, Inc., (1991), PCR Protocols: A Guide to Methods and Applications (Innis, et al. 1990. Academic Press, San Diego, Calit), McPherson et al., PCR Volume 1, Oxford University Press, (1991), Culture of Animal Cells: A Manual of Basic Technique, 2nd Ed. (R. I. Freshney. 1987. Liss, Inc. New York, N.Y.), and Gene Transfer and Expression Protocols, pp. 109-128, ed. E. J. Murray, The Humana Press Inc., Clifton, N.J.).

### 3β-Amino-5α-Androstane Hydrochloride (K118)

As used herein, in one embodiment, a suitable SHIP inhibitor for use in the methods of the present invention can include, without limitation, the following SHIP inhibitor compound of Formula 28, wherein X = NH₂ or NH₃Cl:

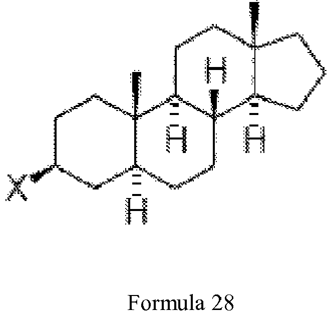

More particularly, the compound described herein as "K118" refers to the SHIP inhibitor compound of Formula 28 wherein X is NH₃Cl. K118 is also referred to herein as 3β-amino-5a-androstane hydrochloride. Aspects of K118 are further described in Example 20, Example 21, and the figures associated with the relevant Examples. For example, as described in Example 21, K118 can be effective as a SHIP inhibitor to prevent or reduce obesity without negatively impacting bone density. K118 is a water-soluble derivative of 3AC and has comparable SHIP1 inhibitory activity. Because K118 is water-soluble, it can be used for pharmacological targeting of SHIP1. K118 can also be described as being a pan-SHIP1/2 type of inhibitor.

Various analogs of K118 can include, without limitation, the compounds identified herein as Formula 11, Formula 14, Formula 17, Formula 20, Formula 23, Formula 24, Formula 25, Formula 31, Formula 32, Formula 33, Formula 34, Formula 35, Formula 36, and Formula 37, wherein X = NH₂ or NH₃ Cl.

Provided below are more particular terms and aspects regarding various embodiments for the use of K118 as a therapeutic composition, although the use of K118 is not meant to be limited by the terms and aspects described below. Further, as used herein, reference to K118 is also meant to relate to the derivatives, analogs, and any variations of K118.

An "effective amount" of K118, and pharmaceutically acceptable salts or derivatives thereof, may be in a dosing range of from 0.05 mg/kg to 150 mg/kg and particularly in a dosing range of from 0.1 mg/kg to 100 mg/kg. More particularly, the dosing range can be from 0.08 mg/kg to 140 mg/kg, from 0.1 mg/kg to 130 mg/kg, from 0.1 mg/kg to 120 mg/kg, from 0.1 mg/kg to 110 mg/kg, from 0.1 mg/kg to 110 mg/kg, from 0.5 mg/kg to 100 mg/kg,
from 1 mg/kg to 100 mg/kg, from 10 mg/kg to 80 mg/kg, from 20 mg/kg to 70 mg/kg, from 20 mg/kg to 60 mg/kg, from 20 mg/kg to 50 mg/kg, from 20 mg/kg to 40 mg/kg, and from 20 mg/kg to 30 mg/kg.

A "pharmaceutically acceptable derivative" means any non-toxic salt, ester, salt of an ester or other derivative of a compound of this invention that, upon administration to a recipient, is capable of providing, either directly or indirectly, a compound of this invention or an inhibitory active metabolite or residue thereof.

In one embodiment of the present invention, K118 is for administration at a dose from 0.05 mg/kg to 150 mg/kg or more particularly at a dose from 0.1 mg/kg to 100 mg/kg once a day, every other day, three times a week, twice a week, once a week, etc. In another embodiment, K118 is for administration at a dose from 0.08 mg/kg to 140 mg/kg, from 0.1 mg/kg to 130 mg/kg, from 0.1 mg/kg to 120 mg/kg, from 0.1 mg/kg to 110 mg/kg, from 0.1 mg/kg to 110 mg/kg, from 0.5 mg/kg to 100 mg/kg, from 1 mg/kg to 100 mg/kg, from 10 mg/kg to 80 mg/kg, from 20 mg/kg to 70 mg/kg, from 20 mg/kg to 60 mg/kg, from 20 mg/kg to 50 mg/kg, from 20 mg/kg to 40 mg/kg, and from 20 mg/kg to 30 mg/kg once a day, every other day, three times a week, twice a week, once a week, etc.

The term "pharmaceutically acceptable" means that a compound or combination of compounds is sufficiently compatible with the other ingredients of a formulation, and not deleterious to the patient up to those levels acceptable by the industry standards.

Therefore, K118 may be formulated into various pharmaceutical forms for administration purposes. As appropriate compositions there may be cited all compositions usually employed for systemically administering drugs. To prepare the pharmaceutical compositions suitable for use in this invention, an effective amount of K118 as the active ingredient is combined in intimate admixture with a pharmaceutically acceptable carrier, which carrier may take a wide variety of forms depending on the manner of preparation desired for administration. These pharmaceutical compositions are desirable in unitary dosage form suitable, particularly, for administration orally, rectally, transdermally, intradermally, topically, by inhalation, nasally, buccally, vaginally, via an implanted reservoir or by parenteral routes. The term "parenteral" as used herein includes subcutaneous, intravenous, intramuscular, intra-articular, intra-synovial, intrasternal, intrathecal, intrahepatic, intralesional and intracranial injection or infusion techniques.

The term "pharmaceutically acceptable carrier" refers to a non-toxic carrier, adjuvant, or vehicle that does not destroy the pharmacological activity of the compound with which it is formulated. Pharmaceutically acceptable carriers, adjuvants or vehicles that may be used in the compositions of this disclosure include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

In one embodiment of the present invention, K118 is for administration orally. K118 can be administered by the oral route in solid dosage forms, such as tablets, capsules, and powders, or in liquid dosage forms, such as elixirs, syrups, suspensions, pharmaceutically acceptable emulsions, microemulsions, solutions, suspensions, syrups and elixirs. The pharmaceutical compositions for use in this invention can also be administered parenterally, in sterile liquid dosage forms.

In preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, lubricants, binders, disintegrating agents and the like in the case of powders, pills, capsules, and tablets. Liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents. Because of their ease in administration, tablets and capsules represent the most advantageous oral dosage unit forms, in which case solid pharmaceutical carriers are obviously employed.

K118 may as well be administered in oral dosage forms such as the ones described in U.S. Patent No. 7,182,958, as a free drug in admixture with a diluent, a lubricant, a hydrophilic binder selected from the group consisting of a cellulose derivative, povidone, and a mixture thereof, a disintegrant selected from the group consisting of crospovidone, croscarmellose sodium, and a mixture thereof, and, optionally, microcrystalline cellulose and/or a wetting agent. Optionally, the formulation additionally comprises a second diluent.

K118 may as well be administered as a coprecipitate preparation with a polymer, as disclosed in U.S. Patent No. 5,985,326, wherein the polymer is for example hydroxy-propyl methylcellulose phthalate. This coprecipitate preparation is prepared, then milled, mixed with excipients, and compressed into tablets for oral administration.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polyethylene glycols and the like.

Provided compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of a compound for use in this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, and eye drops are also contemplated as being for use within the scope of this invention. Additionally, the present invention contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

Also, in certain embodiments, free K118 drug is preferred in particulate form, and wherein at least 90% of the particles have a particle size of less than about 40 microns, and preferably less than 30 microns. Highly preferred particulate forms of the compound (I) have at least 90% of the particles less than 25 microns in size. Most preferred forms of the free compound (I) are those wherein 90% of the particles are less than 10 microns in size, as described and prepared in U.S. Patent No. 6,821,975.

Formulations for parenteral administration may be in the form of aqueous or nonaqueous isotonic sterile injection solutions or suspensions. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations which are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. These latter suitable additives may be anti-oxidants, preservatives, stabilizing agents, emulsifiers, salts for influencing the osmotic pressure, and/or buffer substances.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

Injectable formulations can be sterilized, for example, by filtration through a bacterial -retaining filter, or by incorporating sterilizing agents in the form of sterile solid compositions which can be dissolved or dispersed in sterile water or other sterile injectable medium prior to use.

In order to prolong the effect of a provided compound, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsule matrices of the compound in biodegradable polymers such as polylactide-polyglycolide. Depending upon the ratio of compound to polymer and the nature of the particular polymer employed, the rate of compound release can be controlled.

Examples of other biodegradable polymers include poly(orthoesters) and poly(anhydrides). Depot injectable formulations are also prepared by entrapping the compound in liposomes or microemulsions that are compatible with body tissues.

In one embodiment of the present invention, K118 is for administration transdermally. In one embodiment of the present invention, K118 is for administration topically.

As appropriate topical or transdermal compositions there may be cited for example gels, jellies, creams, pastes, emulsions, dispersions, ointments, films, sponges, foams, aerosols, powders, implants, patches. In the compositions suitable for topical cutaneous administration, the carrier optionally comprises a suitable wetting agent, optionally combined with suitable additives of any nature in minor proportions, which additives do not introduce a significant deleterious effect on the skin. Said additives may facilitate the administration to the skin and/or may be helpful for preparing the desired compositions. These compositions may be administered in various ways, e.g., as a cream or gel.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Pharmaceutically acceptable compositions described herein may also be administered by nasal aerosol or inhalation. Such compositions are prepared according to techniques well-known in the art of pharmaceutical formulation and may be prepared as solutions in saline, employing benzyl alcohol or other suitable preservatives, absorption promotors to enhance bioavailability, fluorocarbons, and/or other conventional solubilizing or dispersing agents.

Pharmaceutically acceptable compositions described herein may be formulated for oral administration. Such formulations may be administered with or without food. In some embodiments, pharmaceutically acceptable compositions of this disclosure are for administration without food. In other embodiments, pharmaceutically acceptable compositions of this disclosure are for administration with food.

The amount of provided compounds that may be combined with carrier materials to produce a composition in a single dosage form will vary depending upon the patient to be treated and the particular mode of administration. Provided compositions may be formulated such that a dosage of between 0.01 - 150 mg/kg body weight/day of the inhibitor can be administered to a patient receiving these compositions.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, suppositories, powder packets, wafers, injectable solutions or suspensions and the like, and segregated multiples thereof.

Notwithstanding the effective amounts and doses indicated above, still the dose of K118, its pharmaceutically acceptable salts and solvates thereof to be administered will depend on the individual case and, as customary, is to be adapted to the conditions of the individual case for an optimum effect, Thus it depends, of course, on the frequency of administration and on the potency and duration of action of the compound employed in each case for therapy or prophylaxis, but also on the nature and severity of the disease and symptoms, and on the sex, age, weight co-medication and individual responsiveness of the subject to be treated and on whether the therapy is acute or prophylactic. Doses may be adapted in function of weight and for paediatric applications. Daily doses may be administered q.d. or in multiple quantities such as b.i.d., t.i.d. or q.i.d. Alternatively, doses may be administered every other day, every three, every four, every five, every six, every seven days, every other week, every month.

The present disclosure discloses a pharmaceutical composition comprising a SHIP inhibitor compound, including, without limitation, a SHIP1 inhibitor and/or a pan-SHIP1/2 inhibitor compound as described herein, or a pharmaceutically acceptable salt thereof

### EXAMPLES

The following examples are intended to illustrate particular embodiments of the present invention, but are by no means intended to limit the scope of the present invention.

### Example 1

### Synthesis of 3α-Acetamido-5α-Cholestane

The 3α-acetamido-5α-cholestane for use in invention can be made using the following synthetic scheme:

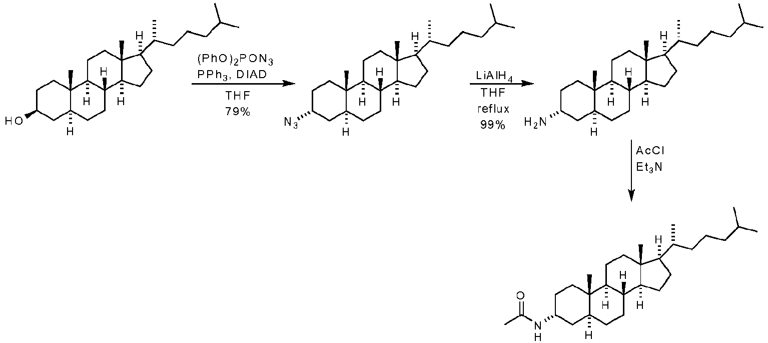

### Example 2

### Experimental Data Relating to 3α-Acetamido-5α-Cholestane

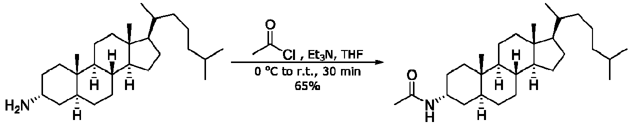

**3α-Acetamido-5α-cholestane.** The α-amine (0.29 g, 0.75 mmol) was dissolved THF (2.21 mL) in a round bottom flask. Et₃N (0.12 mL, 0.90 mmol) was added dropwise and the resulting solution was cooled at 0 °C. Acetyl chloride (0.06 mL, 0.83 mmol) was added dropwise into the cooled solution which resulted on the formation of white precipitate. The milky white solution was stirred continuously for 15 min at 0 °C before allowing the reaction mixture to warm up to room temperature. THF (5 mL) was added and the diluted solution was washed with HC1 (10 mL, 1 M), brine solution (10 mL), and H₂O (10 mL). The organic layer was collected, dried over Na₂SO₄, and concentrated under reduced pressure. Recrystallization of the solid residue using EtOH afforded amide (0.22 g, 65 %) as off white solid.
IR(KBr): 3265, 2931, 2864, 2848, 1667, 1337 cm⁻¹. m.p. = 215-216 °C ¹H NMR (300 MHz, CDCl₃): δ 5.71 (broad, 1H), 4.13 (broad, 1H), 1.99 (s, 3H), 1.96 (t, *J* = 3 Hz, 1H), 1.79 (m 1H), 1.60-1.65 (m, 2H), 1.45-1.60 (m, 7H), 1.31-1.36 (m, 6H), 1.27-1.28 (m, 1H), 1.03-1.04 (m, 2H), 0.96- 1.00 (m, 5H), 0.94-0.96 (m, 1H), 0.87 (s, *J* = 1.2 Hz, 3H), 0.85 (*d, J* = 1.2 Hz, 3H), 0.80 (s, 3H), 0.68-0.73 (m, 1H), 0.65 (s, 3H). ¹³C NMR (75 MHz, CDCl₃): δ 169.4, 56.7, 56.4, 54.7, 44.9, 42.7, 41.0, 40.2, 39.6, 36.3, 36.1, 35.9, 35.5, 33.3, 33.0, 32.1, 28.6, 28.4, 28.1, 26.1, 24.3, 24.0, 23.8, 23.0, 22.7, 20.9, 18.8, 12.2, 11.6

### Example 3

### Synthesis of the β-Amine Compound

The β-amine compound can be made using the following synthetic scheme:

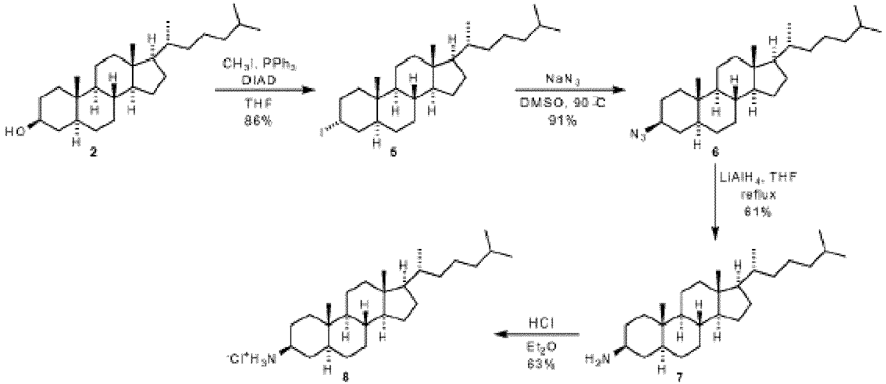

### Example 4

### Analogs

Various SHIP inhibitors are described below:

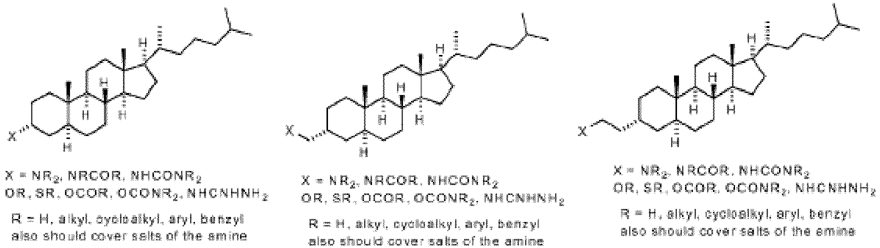

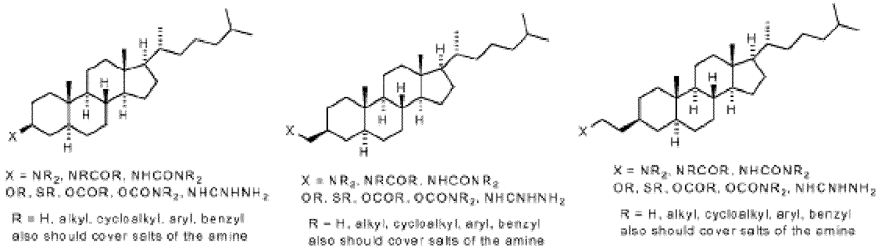

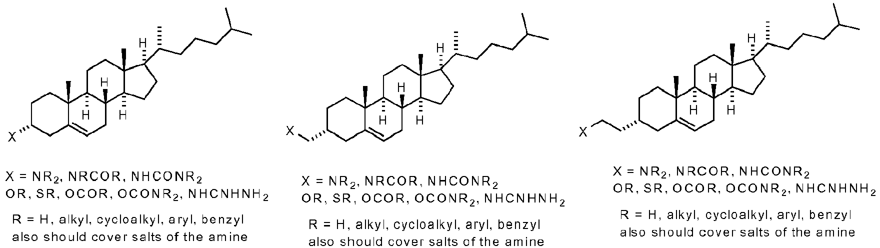

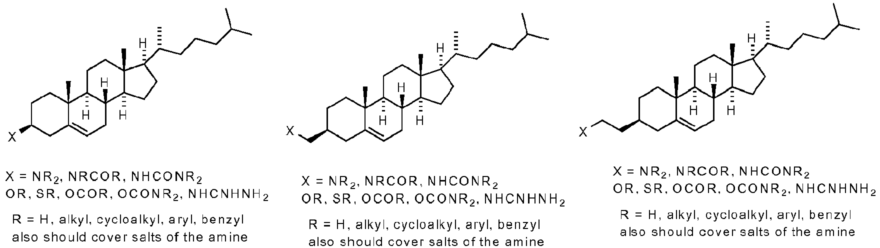

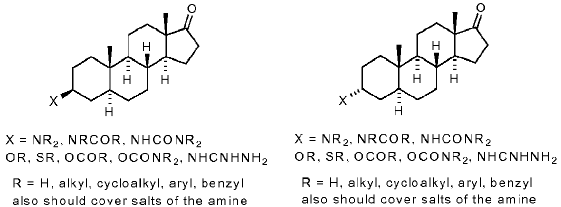

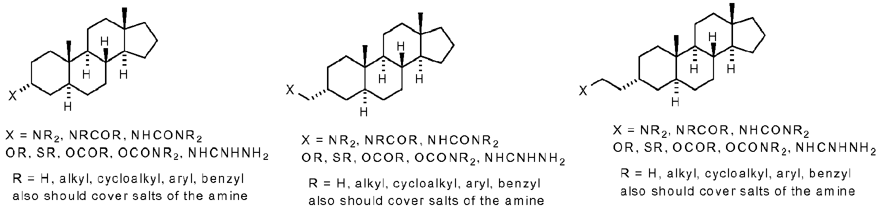

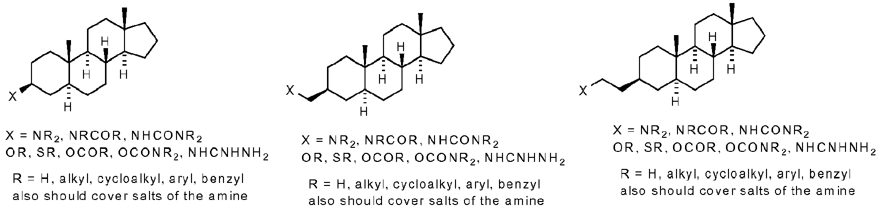

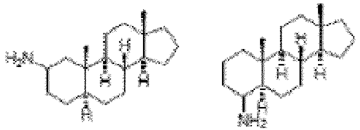

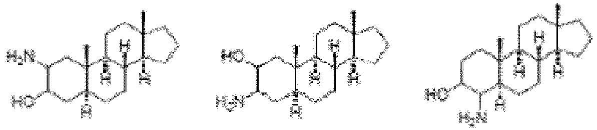

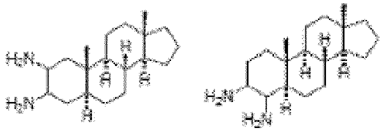

### Example 5

### Synthetic Schemes

Below are various schemes relating to analogs contemplated as being SHIP inhibitors, as described below:

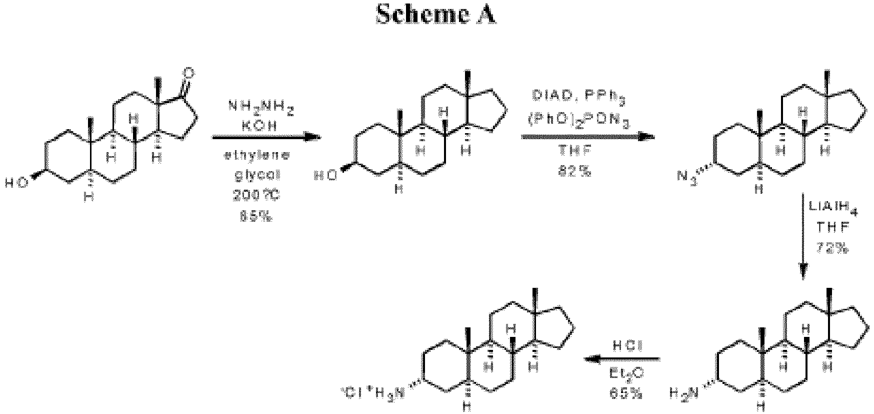

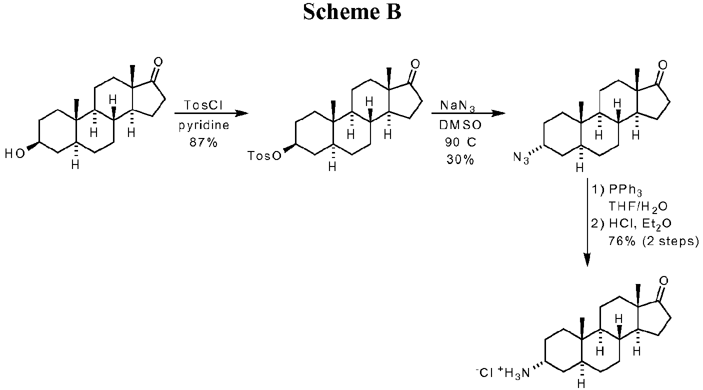

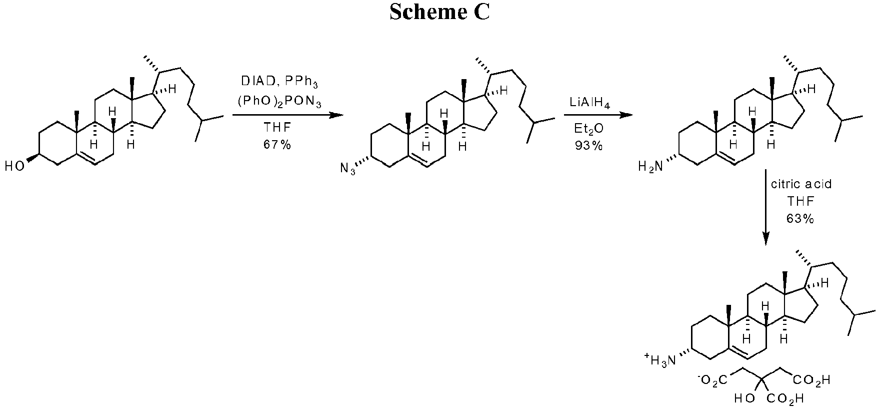

### Example 6

### 5α-Androstan-3β-ol

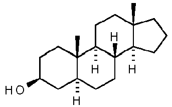

**5α-Androstan-3β-ol:** In a flame-dried flask, potassium hydroxide (1.58 g, 28.2 mmol) was dissolved in ethylene glycol (10 mL) by heating. The solution was cooled at room temperature before adding *trans*-androsterone (2.00 g, 6.89 mmol) and hydrazine hydrate (0.98 mL, 20.2 mmol). The solution was heated to reflux at 208 °C. After 23 h, the solution was cooled at room temperature before adding HCl (14.1 mL, 2M). It was extracted with CH₂Cl₂ (4 x 30 mL). The organic layer were collected, combined, dried over Na₂SO₄, and concentrated under reduced pressure. The resulting solid residue was recrystallized in MeOH to afford 5α-androstan-3β-ol (1.56 g, 82%). ¹H NMR (300 MHz, CDCl₃): d 3.58 (heptet, *J* = 4.9 Hz, 1H), 1.76-1.82 (m, 1H), 1.70-1.75 (m, 2H), 1.65-1.69 (m, 2H), 1.61-1.63 (m, 1H), 1.57-1.60 (m, 1H), 1.52-1.57 (m, 2H), 1.47-1.50 (m, 1H), 1.40-1.45 (m, 1H), 1.33-1.39 (m, 1H), 1.29-1.30 (m, 1H), 1.22-1.28 (m, 4H), 1.04-1.17 (m, 4H), 0.9-1.02 (m, 1H), 0.85-0.93 (m, 2H), 0.80 (s, 3H), 0.68 (s, 3H) 0.60-0.65 (m, 1H).

### Example 7

### 3α-Azido-5α-Androstane

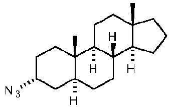

**3α-Azido-5α-Androstane:** In a 50 mL round bottom flask, 5α-androstan-3β-ol (1.12 g, 4.05 mmol) was dissolved in THF (20 mL). PPh₃ (1.06 g, 4.04 mmol) was added into the solution followed by DIAD (0.83 mL, 4.05 mmol). The resulting yellow solution was stirred continuously at room temperature for 10 min before adding (PhO)₂PON₃ (0.88 mL, 4.05 mmol). The solution was stirred continuously at room temperature. After 24 h, the reaction mixture was concentrated and the residue was recrystallized to afford 3α-azido-5α-androstane as a white solid (0.90 g, 74%). ¹H NMR (300 MHz, CDCl₃): δ 3.88 (p, *J* = 2.8 Hz, 1H), 1.71-1.72 (m, 1H), 1.67-1.70 (m, 3H), 1.59-1.64 (m, 2H), 1.57-1.53 (m, 3H), 1.45-1.52 (m, 3H), 1.36-1.42 (m, 2H), 1.26-1.31 (m, 1H), 1.18-1.24 (m, 3H), 1.14-1.17 (m, 2H), 1.13-1.10 (m, 1H), 0.85-1.03 (m, 2H), 0.79 (s, 3H), 0.72-0.77 (m, 1H), 0.69 (s, 3H).

### Example 8

### 3α-Amino-5α-Androstane

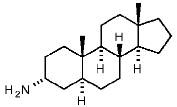

**3α-Amino-5α-Androstane:** In round bottom flask, LiAlH₄ (0.39 g, 9.83 mmol, 95%) was suspended in THF (10 mL). The suspension was cooled at 0 °C using ice/H₂O bath before adding a solution of α-azide (0.90 g, 2.98 mmol) in THF (5 mL). The solution was warmed to room temperature and refluxed at 80 °C for 4 h. The reaction was cooled to room temperature before diluting the solution with THF (15 mL). The diluted reaction mixture was cooled at 0 °C and quenched using a Fieser method. The reaction mixture was stirred continuously until it turned into a milky white solution. The solution was then filtered through celite and washed with THF. The filtrate was dried over Na₂SO₄ and concentrated under reduced pressure to afford 3a-amino-5a-androstane (0.59 g, 72%). IR (KBr): 2926, 2855, 1472, 1378, 1124, 753 cm⁻¹. ¹H NMR (300 MHz, CDCl₃): d 3.18 (broad, 1H), 1.71-1.73 (m, 2H), 1.65-1.69 (m, 3H), 1.61-1.63 (m, 1H), 1.59-1.60 (m, 1H), 1.55-1.57 (m, 2H), 1.50-1.53 (m, 1H), 1.40-1.45 (m, 3H), 1.30-1.32 (m, 1H), 1.23-1.29 (m, 3H), 1.18-1.21 (m, 3H), 1.14-1.18 (m, 2H), 1.07-1.10 (m, 2H), 0.89-1.99 (m, 2H), 0.78 (s, 3H), 0.69 (s, 3H).

### Example 9

### 3α-Amino-5α-androstane hydrochloride

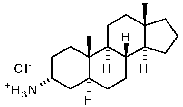

**3α-Amino-5α-androstane hydrochloride:** The **α**-amine **11** (0.20 g, 0.73 mmol) was dissolved in Et₂O (5 mL). A solution of HCl in Et₂O (0.73 mL, 2 M) was added dropwise which resulted to the formation of precipitate. The solution was filtered and the precipitate was collected, washed over Et₂O, and dried over vacuum to afford 3a-amino-5a-androstane hydrochloride (0.15 g, 65%) as a white solid. IR (KBr): 3320, 2945, 1619, 1495, 1443, 1379 cm⁻¹. ¹H NMR (300 MHz, CDCl₃): d 8.45 (broad, 3H), 3.60 (broad, 1H), 1.84 (broad, 2H), 1.62-1.69 (m, 8H), 1.51-1.58 (m, 4H), 1.37-1.44 (m, 1H), 1.23-1.29 (m, 2H), 1.09-1.20 (m, 4H), 0.92-1.07 (m, 3H), 0.79 (s, 3H), 0.69 (s, 3H).

### Example 10

### 3α-Acetamido-5α-Androstane

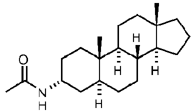

**3α-Acetamido-5α-Androstane:** The α-amine (0.20 g, 0.73 mmol) was dissolved THF (3 mL) in a round bottom flask. Et₃N (0.12 mL, 0.88 mmol) was added dropwise and the resulting solution was cooled at 0 °C. Acetyl chloride (0.05 mL, 0.80 mmol) was added dropwise into the cooled solution which resulted on the formation of white precipitate. The milky white solution was stirred continuously for 15 min at 0 °C before allowing the reaction mixture to warm up to room temperature. THF (5 mL) was added and the diluted solution was washed with HCl (10 mL, 1 M), brine solution (10 mL), and H₂O (10 mL). The organic layer was collected, dried over Na₂SO₄, and concentrated under reduced pressure. Recrystallization of the solid residue using EtOH afforded 3α-acetamido-5α-androstane (0.05 g, 22 %) as white solid. IR (KBr): 3264, 3077, 2933, 2834, 1637, 1558 cm⁻¹. ¹H NMR (300 MHz, CDCl₃): δ 5.70 (broad, 1H), 4.12 (m, 1H), 1.99 (s, 3H), 1.72-1.76 (m, 1H), 1.68-1.71 (m, 2H), 1.62-1.66 (m, 2H), 1.60-1.62 (m, 2H), 1.56-1.58 (m, 1H), 1.52-1.55 (m, 1H), 1.48-1.51 (m, 1H), 1.42-1.46 (m, 1H), 1.36-1.39 (m, 1H), 1.29-1.34 (m, 2H), 1.23-1.27 (m, 1H), 1.21 (d, *J* = 3.0 Hz, 1H), 1.18-1.19 (m, 1H), 1.12-1.17 (m, 2H), 1.08-1.11(m, 1H), 1.00-1.06 (m, 1H), 0.92-0.97 (m, 1H), 0.84-0.90 (m, 1H), 0.81 (s, 3H), 0.71-0.77 (m, 1H), 0.69 (s, 3H).

### Example 11

### 3β-Tosyloxy-5α-Androstan-17-one

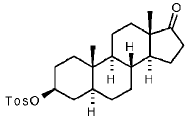

**3β-Tosyloxy-5α-Androstan-17-one:** In a 25 mL round bottom flask, *trans-*androsterone (1.00 g, 3.44 mmol) and p-toluenesulfonyl chloride (1.51 g, 7.91 mmol) was dissolved in in pyridine (4.30 mL). The reaction mixture was stirred continuously at room temperature. After 24 h, the reaction mixture was quenched by adding H₂O (10 mL) and it was extracted with CH₂Cl₂ (3 x 20 mL). All organic layers were collected, combined together and washed over HCl (3 x 20 mL, 2 M), brine solution (3 x 20 mL), and H₂O (3 x 20 mL), dried over Na₂SO₄, and concentrated under reduced pressure afforded 3β-tosyloxy-5α-androstan-17-one (1.33 g, 87%) as a white solid. ¹H NMR (300 MHz, CDCl₃): d 7.79 (dt, *J* = 8.3, 1.9 Hz, 2H), 7.33 (dd, *J* = 8.0, 0.5 Hz, 2H), 4.42 (h, *J* = 5.9 Hz, 1H), 2.44 (s, 3H), 2.38-2.47 (m, 1H), 1.99-2.11 (m, 1H), 1.86-1.95 (m, 1H), 1.78-1.80 (m, 1H), 1.71-1.77 (m, 2H), 1.65-1.69 (m, 1H), 1.56-1.64 (m, 3H), 1.44-1.55 (m, 3H), 1.30-1.31 (m, 1H), 1.28-1.29 (m, 2H), 1.22-1.24 (m, 1H), 1.18-1.20 (m, 1H), 1.04-1.16 (m, 1H), 0.85-1.00 (m, 2H), 0.84 (s, 3H), 0.80 (s, 1H), 0.60-0.69 (m, 1H).

### Example 12

### 3α-Azido-5α-Androstan-17-one

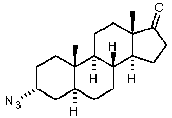

**3α-Azido-5α-Androstan-17-one:** A suspension of tosylate (1.33 g, 2.99 mmol) and NaN₃ (1.94 g, 29.9 mmol) in DMSO (75 mL) was heated to reflux at 90 °C. After approximately 5 h, the reaction mixture was cooled at room temperature before adding H₂O (10 mL). The diluted solution was extracted with Et₂O (3 x 20 mL). All organic layers were collected, dried over MgSO₄, and concentrated under reduced pressure. The solid residue was recrystallized in EtOH to afford 3α-azido-5α-androstan-17-one (0.28 g, 30%). ¹H NMR (300 MHz, CDCl₃): d 3.88 (pentet, *J* = 2.6 Hz, 1H), 2.43 (dd, *J* = 10.3, 9.6 Hz, 1H), 2.00-2.12 (m, 1H), 1.88-1.97 (m, 1H), 1.81-1.83 (m, 1H), 1.76-1.78 (m, 1H), 1.66-1.72 (m, 2H), 1.62-1.65 (m, 1H), 1.51-1.56 (m, 2H), 1.39-1.49 (m, 4H), 1.17-1.34 (m, 7H), 0.94-1.08 (m, 1H), 0.85 (s, 3H), 0.81 (s, 3H).

### Example 13

### 3α-Amino-5α-androstan-17-one hydrochloride

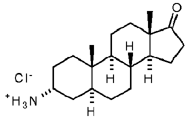

**3α-Amino-5α-androstan-17-one hydrochloride:** In a flame dried flask, azide (0.28 g, 0.89 mmol) and PPh₃ (0.36 g, 1.37 mmol) was dissolved in THF (15 mL). The solution was stirred continuously at room temperature for 18 h. H₂O (3 mL) was added and the solution was heated to reflux at 80 °C. After 1 h, the solution was cooled at room temperature. The organic layer was collected, dried over Na₂SO₄, and concentrated under reduced pressure. The residue was dissolved Et₂O (7 mL) and a solution of HCl (0.89 mL, 2 M) was added which resulted to formation of precipitate. The precipitate was filtered over filter paper, washed over Et₂O, and dried to afford 3α-amino-5α-androstan-17-one hydrochloride (0.22 g, 76%) as white solid. IR (KBr): 3326, 2923, 1737, 1496, 1455, 731 cm⁻¹. ¹H NMR (300 MHz, CDCl₃): δ 8.42 (broad, 3H), 3.61 (broad, 1H), 2.42 (dd, *J* = 11.1, 8.7 Hz, 1 H), 2.00-2.13 (m, 1H), 1.86-1.94 (m, 2H), 1.76-1.83 (m, 3H), 1.44-1.64 (m, 7H), 1.19-1.38 (m, 6H), 0.95-1.13 (m, 2H), 0.84 (s, 3H), 0.81 (s, 3H).

### Example 14

### 3α-Azidocholest-5-ene

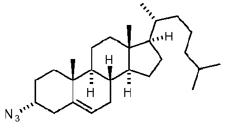

**3α-Azidocholest-5-ene:** Cholesterol (7.76 mmol, 3.0 g) and triphenylphosphine (7.76 mmol, 2.04 g) were dissolved in 77.6 mL of anhydrous tetrahydrofuran. Diisopropyl azodicarboxylate (7.76 mmol, 1.5 mL) was then added dropwise. After stirring the orange mixture for a few minutes, diphenylphosphoryl azide (7.76 mmol, 1.68 mL) was added dropwise. After 24 hours, the pale yellow reaction mixture was concentrated. Purification by silica gel chromatography (100% hexanes) afforded 3α-azidocholest-5-ene (2.14 g, 67%) as a white solid. mp 110-112 °C; TLC *R_{f}* = 0.87 (20% ethyl acetate/hexanes); IR (thin film) 2946, 2914, 2845, 2083 cm⁻¹; ¹H NMR (300 MHz, CDCl₃) δ 5.42-5.40 (m, 1H), 3.89 (t, 1 H, *J* = 2.9 Hz), 2.58-2.49 (m, 1H), 2.23-2.16 (m, 1H), 2.16-1.93 (m, 2H), 1.89-1.05 (m, 24H), 1.02 (s, 3H), 0.93 (d, 3H, *J* = 6.5 Hz), 0.88 (d, 6H, *J* = 6.6 Hz), 0.69 (s, 3H).

### Example 15

### 3α-Aminocholest-5-ene

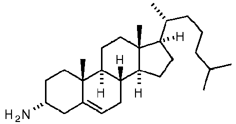

**3α-Aminocholest-5-ene:** 3a-Azidocholest-5-ene (4.62 mmol, 1.9 g) was dissolved in 154 mL of anhydrous diethyl ether. Lithium aluminum hydride (46.2 mmol, 1.75 g) was then added in one portion. After 30 hours, the reaction mixture was cooled to 0 °C. 1.75 mL of deionized water was then added dropwise. After stirring for five minutes, 1.75 mL of 15% aqueous NaOH was added dropwise. After stirring for another five minutes, 5.25 mL of deionized water was added dropwise. The reaction was then stirred until all the salts turned white. Immediately afterwards, the reaction was filtered, dried (Na₂SO₄), and concentrated to afford 3a-Aminocholest-5-ene (1.57 g, 93%) as a white solid. mp 104-106 °C; IR (thin film) 3367, 3343, 2931, 1557 cm^{-1 1}H NMR (300 MHz, CDCl₃) d 5.37-5.34 (m, 1H), 3.15 (t, 1 H, *J* = 3.2 Hz), 2.61-2.54 (m, 1H), 2.04-1.74 (m, 6H), 1.63-1.03 (m, 21H), 1.00 (s, 3H), 0.91 (d, 3H, *J* = 6.5 Hz), 0.86 (d, 6H, *J* = 6.6 Hz), 0.67 (s, 3H).

### Example 16

### 3α-Aminocholest-5-ene Hydrochloride

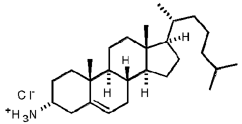

**3α-Aminocholest-5-ene Hydrochloride:** 3α-Aminocholest-5-ene (1.61 mmol, 0.59 g) was dissolved in 2 mL of anhydrous diethyl ether. Hydrogen chloride (2.0 M in diethyl ether) (3.22 mmol, 1.61 mL) was then added. After 3 hours, a white precipitate formed. The reaction was then filtered and the solid was washed with diethyl ether to afford 3α-aminocholest-5-ene hydrochloride (0.31 g, 46%) as a white solid. mp 293-295 °C; IR (thin film) 2947 cm⁻¹; ¹H NMR (300 MHz, CDCl₃) d 8.25 (s, 3H), 5.52 (d, 1H, *J* = 4.3 Hz), 3.58 (s, 1 H), 2.61 (d, 1H, *J* = 14.6 Hz), 2.36 (d, 1H, *J* = 15.0 Hz), 2.02-1.07 (m, 26H), 1.01 (s, 3H), 0.91 (d, 3H, *J* = 6.3 Hz), 0.86 (d, 6H, *J* = 6.6 Hz), 0.67 (s, 3H).

### Example 17

### 3α-Aminocholest-5-ene Citrate

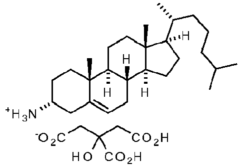

**3α-Aminocholest-5-ene Citrate:** 3α-Aminocholest-5-ene (0.82 mmol, 300 mg) was dissolved in 1.64 ml of tetrahydrofuran. Citric acid (0.82 mmol, 158 mg) was dissolved in 0.82 ml of tetrahydrofuran. The solution of citric acid was added dropwise to the solution of cholesterol amine. The mixture was stirred until the solution became very cloudy (approximately 15 minutes). The solution was vacuum filtered. The resulting white solid was washed with tetrahydofuran, collected, and dried under high vacuum for 12 hours to produce 298 mg of the 3α-aminocholest-5-ene citrate in 63% yield. mp: 172-174 °C; IR (thin film): 3469, 2954, 2247, 1714, 1591 cm⁻¹; ¹H NMR (300 MHz, CD₃OD) δ: 5.53 (d, 1H, *J* = 5.2 Hz), 3.55 (s, 1H), 2.84-2.70 (m, 4H), 2.80-2.70 (m, 1H), 2.19-1.0 (m, 28H), 1.07 (s, 3H), 0.95 (3H, *J* = 6.5 Hz), 0.89 (d, 6H, *J* = 6.6 Hz), 0.73 (s, 3H).

### Example 18

### Synthesis and identification of 3AC derivatives with increased solubility and potency

The solubility of 3AC was assessed by calculating the distribution coefficient (CLogD). This calculation estimates the CLogD for 3AC at 7.17 indicating the molecule is very lipophilic. Thus, we have begun to develop novel 3AC analogs with increased aqueous solubility. One of these compounds, 3A5AS, has a CLogD of 3.33. Lipinski's rules (a common measure of small molecule pharmacokinetics) recommend a ClogD of <5 for *in vivo* applications. The chemical modifications made to derive 3A5AS have not altered its ability to inhibit SHIP1 as it retains equal inhibitory activity in vitro (**Fig. 6A**) and, surprisingly, it is more potent when used on intact cells, as it is substantially more cytotoxic for leukemia cells (**Fig. 6B**). 3A5AS is also more potent at inducing MIR cell numbers *in vivo* as it can induce a comparable MIR cell increase at 15µM as opposed to 60µM 3AC (**Fig. 6C****,D**).

### Example 19

### SHIP Inhibition to Combat Obesity

The role that SHIP1 plays in adipocytes (fat cells) has been observed. For example, mice have been developed that selectively lack SHIP1 expression in MSC/osteoprogenitors, OSXCreSHIPflox/flox mice. In these mice we noted that as the mice aged they lost body fat indicating that accumulation of body fat is disrupted when the SHIP1 gene is ablated in the mesenchymal compartment. It was hypothesized then that treatment of adult or aging mice with a SHIP1 inhibitor might reduce body fat content (i.e., obesity). This was then tested by purchasing older mice that have a larger degree of body fat than their young adult counterparts and treating them three times per week with a SHIP1 selective inhibitor 3AC and then monitoring body fat content by whole body DEXA imaging. These studies confirmed the validity of the hypothesis as the 3AC treatment regimen signifcantly reduced % body fat in aging mice (see Figure 26, p<0.001).

Further, as set forth in **Example 19A** and its associated figures and tables, it was shown that under conditions that drive osteolineage differentiation by MSC, SHIP1 limits MSC proliferation and facilitates osteoblast development by repressing the USP1/Id2 axis. The UPS1/Id2 axis was recently shown to promote 'sternness' of MSC. Remarkably, SHIP1-deficient MSC are not compromised for differentiation to other lineages as shown by the retention of adipogenesis in SHIP-deficient MSC. Our findings identify a novel SHIPl/USPl/Id2 circuit that controls MSC proliferation and lineage commitment and thus link inositol phospholipid signaling to control of MSC self-renewal and multi-lineage potential by USP1/Id2. Our findings also provide cellular and molecular explanations for how SHIP1 influences MSC biology, osteolineage development, adipogenesis and osteoporosis.

Provided is some compelling data that represent the first demonstration of *in vivo* modulation of MSC development and function by small molecule targeting of a cell signaling pathway that has important metabolic implications. The analysis of OSXCreSHIPflox/flox mice indicated that as these mice age they lose both bone mass and body fat, leading to the hypothesis that treatment of aged adult mice with a selective small molecule inhibitor of SHIP1 might achieve the same outcome - reduction of bone mass and body fat. Thus, the **Example 19A** includes a series of studies that rather convincingly demonstrate pharmacologic inhibition of SHIP1 significantly reduces both body fat and bone mass in older mice. When paired with our genetic studies in OSXCreSHIP1flox/flox mice these pharmacological studies demonstrate unequivocally that SHIP1 is a molecular target in both obesity and osteopetrotic diseases. Moreover, they demonstrate for the first time that targeting of cell signaling in the adult MSC compartment can achieve significant metabolic changes that could have translational application in both obesity and osteopetrotic diseases.

The disclosure set forth in **Example 19A** and its associated figures and tables describe profound implications for regulation of mesenchymal stem cell fate, bone biology as well as for treatment of obesity and bone diseases.

### Example 19A

### SHIP1 Limits MSC Proliferation and Promotes Osteolineage Commitment by Repressing the USP1/d2 Axis

### Summary

Here it is shown that SHIP1 is required for the efficient development of osteoblasts from MSC such that growth and bone mass are reduced in mice that lack SHIP1 expression in MSC. SHIP1 controls MSC fate by repressing the expression of USP1 and consequently Id2 to promote osteogenesis. It was also found that mice with myeloid-restricted ablation of SHIP1, including osteoclasts, show no reduction in bone mass, length, mineralization or body size. Hence, diminished bone mass and density in the SHIP 1 -deficient host results from SHIP-deficiency in MSC and osteolineage progenitors. Intriguingly, mice with a SHIP-deficient MSC compartment also exhibit decreased osteoclast numbers. Consistent with this decrease in bone resorptive cells, mice with a SHIP-deficient MSC compartment do not exhibit age-associated bone loss. In addition, we show that treatment of mice with a SHIP1 inhibitor significantly reduces bone and fat composition. These findings demonstrate a novel role for SHIP1 in MSC fate determination and bone growth and the potential for SHIP 1 inhibition to limit bone development in osteopetrotic and sclerotic bone diseases.

The finding that SHIP1 was expressed by primary OB and that OB development is impaired in germline SHIP^{-/-} mice (Hazen et al., 2009) led us to examine a potential role for SHIP1 in MSC/osteoprogenitor function *in vivo.* Here we show that SHIP1 is required for the efficient development of osteoblasts from MSC such that normal body growth, bone formation and mineralization are impaired in mice that lack expression of SHIP1 in MSC/osteoprogenitors. In addition, we show that the absence of SHIP1 expression in MSC results in a bias towards an adipogenic fate. Moreover, SHIP-deficiency promotes a profound expansion of MSC that are selectively hindered for osteolineage commitment due to unrepressed USP1/Id2 expression. In addition, we show that administration of the SHIP1 inhibitor 3AC reduces both bone and fat accumulation. Therefore, this suggests that 3AC may be an effective treatment of osteopetrosis and sclerotic bone disorders, both of which lack therapeutic interventions. Together these findings provide a cellular and molecular basis for SHIP1 maintenance of the pool of MSC within the bone marrow niche and the regulation of MSC osteolineage commitment (see Figure 7).

### Results

### Mice with ablation of SHIP1 expression in MSC exhibit impaired growth, bone development and mineralization.

Our previous findings demonstrating SHIP1 expression in primary OB and defective development in SHIP1-deficient mice (Hazen et al., 2009) led us to question whether SHIP1 plays a functional role in osteolineage development *in vivo* that perhaps could contribute to the osteoporotic phenotype of SHIP 1-deficient mice (Takeshita et al., 2002). To test whether SHIP has a mesenchymal role in OB development and the osteoporotic pathology reported, we developed mice harboring a floxed SHIP locus (Wang et al., 2002) combined with a Cre recombinase transgene under the control of the Osterix promoter that enables selective deletion in pre-osteoblastic mesenchymal stem/progenitors (Rodda and McMahon, 2006). Adult OSXCreSHIP^{flox/flox} mice are viable; however, we observed a significant under-representation of OSXCreSHIP^{flox/flox} weanlings versus their expected Mendelian frequency (p<0.0001, binomial exact test) **(****Figure 22****),** indicating a survival disadvantage for fetuses and/or neonates that lack mesenchymal stem/progenitor expression of SHIP. We confirmed the ablation of SHIP 1 expression in MSC and OB prepared from adult OSXCreSHIP^{flox/flox} mice by immunoblotting whole cell lysates (WCL) prepared from mesenchymal stem cells (MSC) cultured in a primitive, uninduced state as well as following induction of osteogenic differentiation **(****Figure 8A****).** This analysis confirmed that SHIP1 is expressed by MSC and osteolineage cells (*see SHIP*^{*flox*/}*^{flox} control lanes*) and confirmed that SHIP1 protein expression is lost in osteolineage cells derived from OSXCreSHIP^{flox/flox} mice. We also confirmed that OB express the 3' inositol phosphatase, PTEN, that like SHIP1 can also hydrolyze the PI3K product, PI(3,4,5)P₃. PTEN has previously been shown to play a role in OB development and bone formation (Liu et al., 2007). Importantly, we observed no ablation of SHIP1 expression in circulating PBMC **(****Figure 8B****)** or BM derived osteoclasts (OC) obtained from multiple OSXCreSHIP^{flox/flox} mice, **(****Figure 8C****)** confirming that SHIP1 expression in the hematopoietic compartment and myeloid-derived OC remains intact in OSXCreSHIP^{flox/flox} mice.

Despite normal post-weaning viability, the loss of SHIP1 in OSXCreSHIP^{flox/flox} mice resulted in profound effects on their growth. Although male and female OSXCreSHIP^{flox/flox} mice were indistinguishable in size at birth from their SHIP^{flox/flox} counterparts, OSXCreSHIP^{flox/flox} mice were smaller at the time of weaning and remain smaller throughout life. In **Figure 8D** we show images of representative 3 week old male littermates of OSXCreSHI^{flox/flox} and SHIP^{flox/flox}, mice in which the former exhibit distinct growth retardation relative to their SHIP1-competent SHIP^{flox/flox} littermates. This growth retardation was confirmed by measuring body weight weekly **(****Figure 8E** and **Figure 14****)** and tibial lengths at 2, 4, 8 and 16 weeks post-partum **(****Figure 8F****).** These measurements demonstrated significantly reduced body mass at all time points and delayed limb growth at 2, 4 and 8 weeks in OSXCreSHIP^{flox/flox} mice. However, at 16 weeks we observed no significant difference in the limbs lengths of SHIP^{flox/flox} and OSXCreSHIP^{flox/flox} mice. Notably, a logarithmic best-fit analysis of the growth curves, or growth rate, demonstrated that longitudinal growth in OSXCreSHIP^{flox/flox} mice (y=3.33^{∗}ln(x)+7.54; R²=0.99) was increased 41% relative to the SHIP^{flox/flox} control mice (y=2.36^{∗}ln(x)+11.11; R²=0.99). We anticipate that this increased post-partum rate of bone growth in OSXCreSHIP^{flox/flox} mice is due to a compensatory response by chondrocytes in the growth plate. Further, our data on longitudinal growth and body weights are consistent with the seminal observations made by Tanner (Tanner, 1963), in which a disturbance to normal growth leads to an increase in the expression of neuroendocrine factors that subsequently increase growth rate until some intrinsic target-length is achieved.

Direct evidence for compromised OB development was accomplished through the comparison of the internal micro-skeletal architecture of both OSXCreSHIP^{flox/flox} and SHTP^{flox/flox} mice via both dual energy x-ray absorption (DEXA) and three-dimensional micro computed tomography (microCT). Whole body bone mineral density (BMD) in OSXCreSHIP^{flox/flox} mice is significantly reduced relative to SHTP^{flox/flox} controls until 30 weeks of age. This decrease in BMD was consistently found by microCT at 6 weeks through 30 weeks of age **(****Figure 9A****).** Analysis of the microCT images **(****Figure 9B****)** demonstrated that OSXCreSHIP^{flox/flox} mice have diminished mineral content (Bv/Tv, **Figure 9C**) within the proximal tibial metaphysis through 16 weeks of age. OSXCreSHIP^{flox/flox} mice also show reduced trabecular number and thickness early in life, but catch up in these measures of bone formation between 8 and 16 weeks **(****Figures 15B and 15C****).** However, OSXCreSHIP^{flox/flox} did not exhibit age related loss of bone mass as seen in SHIP^{flox/flox} controls **(****Figure 9C****).** Using a histomorphometric analysis of metaphyseal bone that employs unbiased stereology these changes were found to correspond to a significant decrease in the thickness of the metaphysis, the site of initial bone modeling in the proximal tibia **(****Figure 15A****).** BMD at 1 year (40 to 52 weeks) ceased being significantly different **(****Figure 9A****).** At 6 months and 1 year, however, SHIP^{flox/flox} mice appear to lose metaphyseal Bv/Tv in an age related fashion; such that at 6 months there is no difference between the SHIP^{flox/flox} mice and the OSXCreSHIP^{flox/flox} mice and a significant decrease in the metaphyseal Bv/Tv of the OSXCreSHI^{flox/flox} versus SHIP^{flox/flox} controls. Interestingly, while the OSXCreSHIP^{flox/flox} mice reach a peak in Bv/Tv between 8 and 16 weeks their Bv/Tv values do not to decline through 1 year of age. Significantly elevated production of the decoy ligand for RANKL, OPG, was observed in 40 week and older OSXCreSHIP^{flox/flox} relative to SHIP^{flox/flox} controls **(****Figure 9D****).** Increased OPG production by the aging SHIP-deficient osteolineage compartment may further reduce the resorptive capacity and numbers of OC in the metaphyseal compartment in OSXCreSHI^{flox/flox} **(****Figures 10E and 10F****)** during aging and thus provide protection against age-associated loss of bone mass **(****Figures 9C****,** **16C and 16D****).**

We also find that both thickness and the perimeter of cortical, mid-shaft bone in OSXCreSHIP^{flox/flox} mice are significantly decreased relative to SHIP^{flox/flox} controls **(****Figures 16A, 16C and 16D****).** However, at 26 weeks and 52 weeks perimeter and thickness have significantly increased in OSXCreSHIP^{flox/flox} mice in comparison to SHIP^{flox/flox} controls consistent with the Bv/Tv results for the tibial metaphysis **(****Figure 9C****).** A regression analysis demonstrates that the rate at which perimeter and thickness increased were greater in the OSXCreSHIP^{flox/flox} mice. Notably, however, Bv/Tv measured at the mid-shaft of the tibia (cortical bone) was not significantly different between OSXCreSHIP^{flox/flox} mice and SHIP^{flox/flox} counterparts at any age **(****Figure 16B****).** It is particularly intriguing that there is a defect in bone mass accrual in the metaphysis and not in the mid-shaft diaphyseal (cortical) bone, since metaphyseal bone is largely generated through endochondral bone formation and cortical bone through intramembranous bone formation (Karsenty et al., 2009). In this context, it is not surprising that geometric parameters (*e.g.,* cortical thickness/perimeter) begin to catch up following the cessation of endochondral bone growth 6 to 8 weeks post-partum, suggesting that intramembranous bone formation may act *in lieu* of endochondral bone formation to compensate for the overall loss of bone mass in OSXCreSHIP^{flox/flox} mice.

### Osteolineage expression of SHIP1 prevents skewing of mesenchymal progenitors toward an adipogenic fate.

To further confirm that SHIP is required for normal osteoblast development we performed an *ex vivo* analysis of the osteogenic potential of BM MSC in OSXCreSHIP^{flox/flox} mice using the colony forming unit-fibroblastic (CFU-F) assay. We found that the number of MSC-derived alkaline phosphatase (ALP) positive osteoblastic colonies is significantly reduced in OSXCreSHIP^{flox/flox} mice, while formation of ALP negative colonies was unaffected **(****Figure 10A and 10B**). To further define the role of SHIP in OB differentiation and function we tested the ability of MSC cultures prepared from OSXCreSHIP^{flox/flox} mice to become OB and secrete mineralized matrix *ex vivo* following induction. OSXCreSHIP^{flox/flox} OB cultures showed reduced mineralization upon induction of osteogenic differentiation as compared to control SHIP^{flox/flox} OB (Figure 3C). In parallel, we also assessed the ability of MSC cultures derived from OSXCreSHIP^{flox/flox} mice to differentiate into adipocytes *ex vivo.* Intriguingly, we find more adipocytic cells are obtained from OSXCreSHIP^{flox/flox} MSC than SHIP^{flox/flox} MSC **(****Figure 10D****).** Osteoblastic differentiation of MSC to ALP positive colonies remained significantly decreased in the OSXCreSHIP^{flox/flox} mice at 26 and 52 weeks of age, indicating that osteogenic differentiation of SHIP-deficient MSC remains defective throughout life **(****Figure 17A and 17B****).** These results indicate that the SHIP 1-deficient MSC is unable to efficiently commit to an osteoblastic fate.

### Osteoblastic expression of SHIP1 is required for normal osteoclastogenesis.

SHIP-deficiency in one cell type can lead to lineage extrinsic effects on other lineages that alter their homeostasis and function (Collazo et al., 2012; Hazen et al., 2009). Others have reported that OC numbers are significantly increased in germline SHIP^{-/-} mice and that this contributes to their osteoporotic pathology (Takeshita et al., 2002). However, we observed a significant decrease in OC numbers in OSXCreSHIP^{flox/flox} BM **(****Figures 10E and 10F**). This OC decrease was observed consistently at all ages examined between 4 to 52 weeks of age and is consistent with the role that osteolineage cells play in promoting osteoclast differentiation via macrophage colony stimulating factor (M-CSF) and receptor activator of nuclear factor κB ligand (RANKL) production (Karsenty et al., 2009; Yoshida et al., 1990). No difference was observed in circulating monocyte numbers in OSXCreSHIP^{flox/flox} and SHIP^{flox/flox} controls indicating SHIP1 expression by OB selectively impacts OC differentiation rather than exerting a general effect on myelopoiesis *in vivo* **(****Figure 10G**).

### Osteolineage expression of SHIP1 influences both metaphyseal and systemic fat content.

Consistent with the above results showing an adipogenic bias by SHIP 1 -deficient MSC *ex vivo,* we also find that the fat content of bone marrow (metaphyseal fat) is significantly increased in OsxCreSHIP^{flox/flox} mice, confirming MSC lineage commitment is also skewed *in vivo.* Body fat analysis by DEXA demonstrated that there is also a significantly higher percentage of body fat up to 10 weeks of age in OSXCreSHIP^{flox/flox} mice. However, the percentage of total body fat normalized relative to SHIP^{flox/flox} controls by 26 weeks of age **(****Figure 18A****).** Image segmentation analysis of microCT images demonstrated that bone marrow fat content in OSXCreSHIP^{flox/flox} mice at 2 and 4 weeks was 16% and 35% greater than SHIP^{flox/flox} mice while at 16 weeks fat content was 32% less in the OSXCreSHIP^{flox/flox} mice versus the SHIP^{flox/flox} mice **(****Figure 18B****).** A linear regression analysis demonstrated that fat content significantly decreased (p<0.0001) as a function of age in the OSXCreSHI^{flox/flox} mice (R²=0.53). However, there was no age-associated change in bone marrow fat content in the SHIP^{flox/flox} mice (R²=0.0008). Increased bone marrow adiposity was confirmed in the tibias of 4 weeks OSXCreSHIP^{flox/flox} mice stained with the lipophilic fluorescent stain Nile Red **(****Figure 18C****).** The increase in bone marrow adiposity corresponded with the loss of bone mass; an observation that is consistent with our data showing that SHIP1 is required for efficient commitment of MSC to OB and thus loss of SHIP1 expression leads by default to increased adipogenic commitment and thus increased adiposity. These data suggest that SHIP1 promotes osteogenesis and consequently limits adipogenesis *in vivo.*

### SHIP1 expression in osteoclasts does not limit OC differentiation and activity in vivo.

A previous study suggested that germline SHIP1^{-/-} mice were osteoporotic due to increased numbers of OC *in vivo* that were shown to be hyper-resorptive *ex vivo* (Takeshita et al., 2002). Our collaborative study confirmed the hyper-resorptive capacity of SHIP1^{-/-} OC *ex vivo* and attributed this to a failure of SHIP1 to block PI3K recruitment to DAP12:TREM2 complexes expressed by OC (Peng et al.). However, given our findings of osteoporosis in mice where SHIP ablation is confined to the osteolineage we questioned whether the failure of normal bone growth and mineralization in SHIP^{-/-} mice is actually a consequence of hyper-resportive OC *in vivo.* As OC are a terminally differentiated myeloid lineage cell, we developed LysMCreSHIP^{flox/flox} mice where myeloid derived cells are selectively rendered SHIP1-deficient (Collazo et al., 2012). As anticipated, this genetic strategy resulted in robust ablation of SHIP1 expression in OC from LysMCreSHIP^{flox/flox} mice **(****Figure 11A****).** However, no difference was observed in circulating monocyte numbers in LysMCreSHIP^{flox/flox} and SHIP^{flox/flox} controls indicating that myeloid expression of SHIP1 is not necessary to limit the size of the circulating myeloid cell compartment *in vivo* **(****Figure 11I****).** We then performed detailed analysis on internal micro-architecture of 16 week old adult LysMCreSHIP^{flox/flox} mice using DEXA, microCT and the CFU-F assay. DEXA analysis failed to show any negative impact on whole body BMD in adult LysMCreSHIP^{flox/flox} mice relative to SHIP^{flox/flox} controls **(****Figure 11B****).** MicroCT analysis also failed to show any significant difference in trabecular bone Bv/Tv **(****Figure 11D****).** Further, microCT analysis of the tibia **(****Figure 11C****)** revealed no difference in metaphyseal thickness in comparison to age-matched SHIP^{flox/flox} controls demonstrating that a SHIP-deficient OC compartment has no negative impact on bone mass accrual *in vivo.* In addition, OBs are found in normal numbers in LysMCreSHIP^{flox/flox} mice as we observed normal numbers of ALP positive colonies **(****Figure 11F****).** TRAP staining of bone sections indicated there were normal numbers of OC present in the bone of LysMCreSHIP^{flox/flox} as compared to SHIP^{flox/flox} mice **(****Figure 11E**), but, and consistent with previous findings,(Peng et al.; Takeshita et al., 2002) SHIP-deficient OC from LysMCreSHIP^{flox/flox} expand to a greater extent when cultured *ex vivo* in the presence of M-CSF and RANKL relative to OC from SHIP^{flox/flox} controls **(****Figures 11G and 11H****).** Thus, a SHIP-competent OB compartment limits the regulation of OC differentiation and resorptive behavior *in vivo* to prevent the development of osteoporosis. However, SHIP also has an OC cell autonomous role in limiting control of their response to key differentiation-inducing ligands like M-CSF and RANKL. Nonetheless, a SHIP-deficient OC compartment is not sufficient to cause loss of bone mass or density.

### SHIP1 expression in osteolineage cells is not required for bone formation

To further examine the functional role of SHIP1 in osteoblasts, we crossed mice harboring floxed SHIP locus (Wang et al., 2002) with mice carrying the Cre recombinase transgene under the control of the type I collagen (Col1a1) promoter (Dacquin et al., 2002) to generate Col1a1CreSHIP^{flox/flox} mice. The loss of SHIP1 in mature osteoblasts in 4-6 weeks old Col1a1CreSHIP^{flox/flox} mice resulted in no effects on their growth **(****Figure 19A****).** No difference was observed on whole body BMD by DEXA analysis in Col1a1CreSHIP^{flox/flox} mice relative to SHI^{pflox/flox} controls **(****Figures 19B and 19C****).** Unlike OSXCreSHIP^{flox/flox}, Col1a1CreSHIP^{flox/flox} mice showed no difference in limb lengths relative to SHIP^{flox/flox} controls **(****Figure 19D****).** Trabecular bone Bv/Tv **(****Figure 19E****),** trabecular number, thickness and space **(****Figures 19F-19H****)** were not observed to be significantly different in the Col1a1CreSHIP^{flox/flox} mice relative to controls. In addition, we observed normal numbers of ALP positive colonies **(****Figure 20A****),** and serum OPG, RANKL levels in Col1a1CreSHIP^{flox/flox} mice. In comparison to 4-6 week old OSXCreSHIP^{flox/flox} mice that exhibited impaired growth and bone development; Col1a1CreSHIP^{flox/flox} were normal, confirming that the loss of SHIP expression in a mature osteoblast phenotype does not affect osteogenesis. Instead, expression of SHIP1 in MSC is required for normal osteogenesis and bone appositional homeostasis.

### SHIP1 expression represses USP1 and Id2 to facilitate osteolineage commitment and limit proliferation of MSC.

We noted that BM MSC cultures from OSXCreSHI^{flox/flox} mice showed significantly increased total cell numbers both prior to and after induction of osteogenic differentiation despite equal seeding of the two cultures. Consequently we analyzed these cultures by flow cytometry to determine if the increased cellularity reflects a greater degree of proliferation and survival by OSXCreSHIP^{flox/flox} MSC. We quantified the frequency and absolute numbers of MSC **(****Figures 12A-12D****)** in these cultures using the CD29⁺Lin⁻ MSC phenotype described recently by Zhu *et al* (Collazo et al., 2012). The absolute numbers of MSC was significantly higher in the OSXCreSHIP^{flox/flox} cultures relative to SHIP^{flox/flox} MSC cultures indicating SHIP-deficient MSC proliferate vigorously and resist differentiation even under osteogenic culture conditions **(****Figure 12D****).** In fact, the absolute yield of MSC was approximately 10-fold higher for SHIP-deficient MSC suggesting a rather profound impact of SHIP-deficiency on MSC growth and expansion. In addition to having a growth advantage, SHIP-deficient MSC were also found to undergo apoptosis at a significantly lower frequency than SHIP-competent cultures **(****Figures 12E and 12F****).**

We speculated that SHIP1 signaling must limit the expression of factors that promote MSC proliferation, while also blocking their differentiation toward an osteoblast fate. Over-expression of the Id2 transcription factor has been shown to promote MSC proliferation, while selectively blocking osteolineage differentiation by MSC (Peng et al., 2004; Williams et al., 2011). Moreover, coordinate over-expression of the deubiquitinase USP1 is necessary to prevent proteasomal degradation of Id2 and sustain its expression at a level sufficient to promote MSC proliferation and block osteoblast differentiation (Williams et al., 2011). Western blot analysis of both Id2 and USP1 revealed that SHIP1-deficient MSC express higher levels of both proteins under undifferentiated growth, but also importantly under osteogenic conditions **(****Figure 12G****).** In fact, we observe a 25-fold increase in USP1 expression under osteogenic differentiation conditions. These results reveal that activation of SHIP1 expression in MSC limits their proliferation and facilitates their osteolinage differentiation by limiting expression of USP1 and consequently Id2 in response to osteogenic signals. The increased expansion of MSC with hindered osteolineage commitment also accounts for the increase in adipogenesis in OSXCreSHI^{flox/flox} MSC observed both *in vivo* and *in vitro.*

### Administration of a SHIP1 inhibitor reduces bone mass

Based on our findings in the genetic mouse model described above we hypothesized that chemical inhibition of SHIP1 might then be employed to impact both bone and fat composition. A selective, small molecule inhibitor of SHIP1, 3-α-aminocholestane (3AC),(Brooks et al., 2010; Fuhler et al., 2012) has recently been identified. Patients with osteopetrosis and sclerotic bone diseases present with pathologically increased bone mass, for which there are no effective therapies and thus if chemical inhibition of SHIP 1 *in vivo* can reduce bone growth then such compounds might become an effective therapy for such diseases. Moreover, our studies of older OSXCreSHIP1^{flox/flox} mice suggests that fat composition might also be reduced by targeting SHIP1 and thus such an pharmacological approach might have relevance to the control of obesity. To test the feasibility of these hypotheses, we administered the SHIP1 inhibitor 3AC to adult mice three times per week for 1-3 months duration. We find that 3AC treatment significantly reduces whole-body BMD **(****Figure 13A****)** and bone mass **(****Figure 13B****).** We also observe a significant decrease in fat composition of 3AC treated adult mice as measured by percentage of whole-body fat **(****Figure 13C****).** Consistent with our studies in OSXCreSHIP^{flox/flox} of comparable ages to the treated mice, 3AC treatment also significantly increases serum levels of OPG and decreases RANKL **(****Figures 13D and 13E****).** 3AC treatment also resulted in diminished mechanical properties in the treated femurs; with a 15.4% (p<0.01) decrease in the peak load and a 21.6% (p<0.04) decrease in the stiffness **(****Figure 23****).** These changes correspond to significant decreases in cortical thickness in the femurs of 3AC treated mice **(****Figure 23****).** However, the energy to peak and the energy to fracture were not observed to be significantly different **(****Figure 23****),** suggesting that 3AC treated limbs are able to undergo increased deformation prior to failure. Taken together, these data suggest that 3AC effects are primarily through diminished bone apposition, which in an adult mouse would occur at the periosteal and endosteal surfaces and not through turnover of the metaphyseal bone. Nevertheless, it remains unclear if the relatively modest decrease in peak load and stiffness observed in the 3AC treated mice would incur an increase in the incidence of pathologic fracture. This decrease in total body fat is consistent with results obtained in the OSXCreSHIP^{flox/flox} mice. Taken together, these data indicate that SHIP1 inhibition may have the potential in osteopetrotic and other sclerotic bone disorders to repress unrestrained osteoblastogenesis. In addition, significant reductions in adult body fat composition may also be possible via pharmacological targeting of SHIP1.

### Discussion

These findings shed new light on the origin of osteoporotic pathology in SHIP-deficient mice. We confirm the earlier findings of Takeshita *et al* (Takeshita et al., 2002) and Peng *et al* (Peng et al., 2010) that showed a cell autonomous role for SHIP in limiting the response of OC to M-CSF and RANKL *ex vivo.* However, our findings also demonstrate a role for SHIP in osteogenesis and indicate a SHIP-competent OB compartment regulates OC differentiation and resorptive capacity *in vivo* and can prevent SHIP-deficient OC from undergoing dysregulated differentiation and function *in situ.* Our findings also have potential therapeutic implications for Paget's disease, and particularly Paget kindreds where GWAS analysis has implicated a genetic locus at 2q37 - the location of the human SHIP1/INPP5D locus (Hocking et al., 2001). If human SHIP1 deficiency is found to be linked to disease in these kindreds, then our findings suggest that a hematopoietic marrow graft to replace a hyper-resorptive OC compartment may not be beneficial as the disease pathology is likely caused by SHIP 1-deficiency in mesenchymally-derived OB that are not replenished from donor HSC following BMT.

The osteoblastic requirement for SHIP1 that we have identified here represents the first function demonstrated for SHIP1 in a cell type that is not a component of the hematolymphoid compartment. As SHIP1 is not typically expressed in mesenchymal lineages its expression could potentially be controlled by transcription factors active in both hematopoietic and mesenchymal lineages. In this regard, a potential candidate regulator is SMAD4 which is required for OB development (Tan et al., 2007) and known to also induce expression of SHIP1 in myeloid cells (Pan et al., 2010). Induction of SHIP1 by SMAD4 may in turn repress the activity of other SMAD factors that promote sternness and MSC proliferation (*e.g*., USP1, Id2).

The diminished bone mass that was observed in OSXCreSHIP^{flox/flox} mice up to 6 months of age occurred concurrently with dysregulated fat mass accumulation, such that fat content was higher initially in OSXCreSHIP^{flox/flox} mice and then decreased to be less than that observed in SHIP^{flox/flox} mice as the mice aged. OSXCreSHIP^{flox/flox} mice were runted when compared to SHIP^{flox/flox} mice independent of age despite an age-associated acceleration in the rate of longitudinal bone growth. Importantly, we find that MSC from OSXCreSHIP^{flox/flox} mice, independent of donor age, were more adipogenic than SHIP-competent MSCs, which suggests that in OSXCreSHIP^{flox/flox} mice some extrinsic factor suppresses adipogenesis systemically as the mice age. A number of studies have shown that adipocyte proliferation and differentiation can be suppressed through adipokine and neuroendocrine signaling (Fu et al., 2005),(MacDougald and Burant, 2007),(Thomas et al., 1999). In addition, growth plate chondrocyte proliferation and hypertrophy have been shown to be increased by adipokine signaling, which corresponds to our observation of compensatory longitudinal growth (Kume et al., 2002),(Maor et al., 2002). Thus, in addition to our data demonstrating that SHIP1 is required for osteogenesis, SHIP1 appears to participate in the regulation of an unknown extrinsic signaling axis, perhaps adipokine signaling, by limiting adipogenesis. Further, this role for SHIP1 in OB that we have shown is consistent with a number of recent studies that have demonstrated that bone participates in the regulation of insulin signaling and glucose metabolism in parallel with adipose tissue (Lecka-Czernik, 2011),(Kanazawa et al., 2009),(Ferron et al., 2008; Ferron et al., 2010) and also reflects an attempt by OB to maintain mechanical homeostasis. Our data suggests that endochondral bone formation and intramembranous bone formation become de-coupled - endochondral bone formation is impaired whereas intramembranous bone formation remains unaffected. This later observation, that intramembranous bone formation was unaffected by the loss of SHIP1 expression was confirmed in the Col1a1CreSHIP^{flox/flox} mice, which showed no bone phenotype. Since the deletion of SHIP1 in Col1a1CreSHIP^{flox/flox} mice is restricted to mature OB and not MSC, these data further support the observation in the OSXCreSHIP^{flox/flox} mice that SHIP1 expression is required for efficient OB differentiation from MSC.

Our findings implicate SHIP1 at the nexus of a novel molecular pathway that limits Id2 expression, and consequently MSC proliferation, while also promoting commitment of MSC to osteolineage development. *Williams et al* (Williams et al., 2011) identified USP1 and its co-factor WDR48 in MSC and as such are potential targets for SHIP1 regulation in MSC. Our findings demonstrate that SHIP1 expression in MSC limits USP1 expression and consequently Id2 expression. *Park et al* demonstrated that super-physiological expression of Id2 promotes adipocyte differentiation by promoting PPARγ activity in adipocyte progenitors (Park et al., 2008). Thus, increased expression of Id2 in SHIP-deficient MSC corresponds with reduced OB differentiation and increased adipogenesis that we observed *in vivo* in young OSXCreSHIP^{flox/flox} mice and in MSC *in vitro.*

Our findings are the first to show that SHIP 1 can promote lineage commitment in a population of MSC rather than simply acting as an inhibitor of cell survival and function in differentiated cells. Our study identifies a SHIP1 regulated switch that controls the USP1/WDR48/Id2 axis that promotes sternness versus lineage commitment in MSC. The enzymatic activity of SHIP1 has recently been shown to be amenable to modulation *in vivo* and can provide a therapeutic benefit without apparent toxicity to the host (Brooks et al., 2010; Fuhler et al., 2012). Age-associated loss of bone mass is abrogated in OSXCreSHIP^{flox/flox} mice which suggests that there are factors that compensate the loss of SHIP1 during aging, such as increases in OPG in conjunction with decreases in OC numbers.

We administered the SHIP1 inhibitor 3AC to adult mice and found that we could selectively reduce bone mass, without producing a significant loss in mechanical properties or bone morphology. Therefore, we propose that 3AC may be an effective therapy for osteopetrotic syndromes and sclerotic bone diseases. Osteopetrosis is a collection of intractable and incurable syndromes that manifest as a pathologic increase in bone mass (Whyte and Asbmr, 2009). Activating mutations in Lrp5 (high bone mass syndrome) and the two different loss of function mutations in SOST gene expression (Van Buchem Disease and sclerosteosis) result in increased osteogenesis due to decreased osteoblast apoptosis (Whyte and Asbmr, 2009). High bone mass syndrome is a relatively mild condition that may present with cranial nerve palsy and oropharyngeal exostoses (Whyte and Asbmr, 2009). Whereas, Van Buchem disease and sclerosteosis are severe autosomal recessive disorders that result in limb pain, cranial nerve palsy, deafness and optic nerve atrophy (Whyte and Asbmr, 2009). Sclerosteosis patients also exhibit excessive height, syndactyly and a shortened life expectancy (Whyte and Asbmr, 2009). Pathological localized increases in bone mass, or sclerlosis, can be induced through the application of X-radiation therapy (Barth et al., 2011; Damron et al., 2003; Margulies et al., 2003; Wall et al., 1996). X-radiation therapy used to treat metastatic carcinomas in bone, primary pediatric sarcomas or osteomas often leads to sclerosis and pathologic fracture (Barth et al., 2011; Damron et al., 2003; Margulies et al., 2003; Wall et al., 1996). Sclerotic bone diseases are often treated surgically and have a poor long-term prognosis, often leading to fracture and disunion (Wall et al., 1996). In this context 3AC may represent an effective adjuvant chemotherapeutic. Whereas, in the mutations that result in a gain of function in canonical Wnt/□□ catenin signaling and increased osteogenesis, 3AC may represent a novel therapy for these conditions for which there is currently no known pharmacological intervention.

### Material and Methods:

### Mice and genotyping

SHIP^{flox/flox} mice backcrossed to a C57BL6/J background express normal levels of SHIP, but the SHIP proximal promoter and 1st exon are floxed, meaning that SHIP can be deleted when Cre recombinase is expressed (Wang et al., 2002). Osx-Cre transgenic mice have been previously described (Rodda and McMahon, 2006). Genotyping of Cre transgenic mice was performed by PCR using primers detecting the *Cre* sequence (P1, 5'-GTGAAACAGCATTGCTGTCACTT-3'; P2, 5'-GCGGTCTGGCAGTAAAAACTA- 3'). All animal experiments were approved by the SUNY Upstate Medical University Committee for the Humane Use of Animals.

### Derivation of bone-marrow-derived MSC and monocytes

The adherent cell-fraction (MSC) and non-adherent cell-fraction (monocytes) were collected from OSXCreSHI^{flox/flox} mouse bone marrow (BM) and subsequently used to study regulation of the differentiation and metabolic activity of bone cells (osteoblasts, adipocytes and osteoclasts). Mice were euthanized; femurs were removed aseptically and flushed using αMEM (Cellgro, Mediatech) and passed through a 70-µm filter into a collection tube. The cells were then resuspended and plated in growth media: αMEM, 10% FBS (Atlanta Biological), 1% penicillin-streptomycin solution (Cellgro, Mediatech) and 1% L-glutamine (Cellgro, Mediatech) and cultured at 37 °C with 5% CO₂. After 48 hours monocytes were removed and cultured separately for differentiation of osteoclasts. The remaining MSC were expanded through the 3rd passage for 3- 5 weeks and used to assess osteogenic and adipogenic differentiation.

### Analysis of Serum Markers

Osteoprotegerin (OPG) and RANK-ligand levels in mouse serum were measured using ELISA kits (R&D Systems).

### Western blot analysis

Cell lysates were individually treated and prepared from primary BM derived cells from OSXCreSHIP^{flox/flox} and SHIP^{flox/flox} mice. Lysate supernatants were resolved on a 7.5% Bis-Tris gel and transferred to a Hybond-ECL nitrocellulose membrane (GE Healthcare, Little Chalfont, United Kingdom). For chemiluminescence the membranes were incubated with specific primary antibodies against SHIP1 (P1C1,) Actin and Id2 (C-20) (Santa Cruz Biotechnology, SantaCruz, CA). PTEN and USP1 (Cell Signaling Technology Inc., Danvers, MA, USA). The membrane was blocked with 5% BSA in TBS with 0.1%Tween-20 (TBS-T) and probed with specific primary antibody and then by horseradish peroxidase (HRP) conjugated secondary antibody (Cell Signaling Technology Inc., Danvers, MA, USA). Protein was detected using SuperSignal ECL Substrate / Pico Substrate (Pierce).

### Colony Forming Unit Assay

Primary whole BM cells (adherent + non-adherent) were plated in 60-mm plates (triplicates, 3X10⁶ cells per plate) in CFUF growth media: αMEM, Mesencult Serum (Stem Cell Technologies) with 1% penicillin-streptomycin-glutamine. Cells were cultured for approximately 12 days, after which cells were fixed with 70% ethanol and stained for alkaline phosphatase activity using the Leukocyte Alkaline Phosphatase Kit (Sigma) and counter-stained with neutral red (Sigma). ALP⁺ cells/ colonies stain purple while ALP⁻ cells/colonies stain red. Colonies were counted when they contained 25 cells per colony using the Software program "Image J" (National Institutes of Health Research Services Branch (http://rsbweb.nih.gov/ij/)).

### Osteogenic, adipogenic and osteoclastic differentiation

MSC were induced to become osteoblasts using osteogenic induction media (OIM) or adipogenic induction media (AIM). OIM was composed of β-glycerol 2- phosphate (BGP, 4mM; Sigma; Cat: G9891), 2-phospho-1-ascorbic acid (25 µg/mL; Sigma; Cat: A8960) for 14 days, with media changed every 2 days. To assay for formation of mineral nodules, plates were stained with 1% Alizarin red S solution (pH 4.2) (Margulies et al., 2008). These cells were also used to detect SHIP and PTEN expression. For adipocyte differentiation cells were induced with AIM containing 1 µM dexamethasone, 10 µg/ ml insulin, 0.5 mM Methylisobutylxanthanine, 5 mM Troglitazone and 10% FBS in αMEM. Adipocytes were stained with Oil Red O as follows: cells were washed with 60% isopropanol and then stained with a 0.35 g/ml Oil Red O solution in isopropanol. Cells were incubated for 10 min with Oil red working solution and rinsed four times.

Osteoclasts were differentiated from monocytes cultured for 48 hours with 100 ng/mL of recombinant M-CSF (Wyeth). Monocytes expanded through M-CSF treatment were then cultured (1x10⁶ cells per well) with 25 ng/ml recombinant RANK-ligand (R&D Systems) and 25 ng/mL M-CSF for approximately 14 days, with media changes every 2 days. Multinucleate osteoclasts were stained with the tartrate resistant acid phosphatase (TRAP) (Acid Phosphatase, Leukocyte Kit; Sigma, St. Louis, Missouri) (Margulies et al., 2008). Multinucleate, TRAP+ osteoclasts were subsequently counted using a modification of Cavalieri's sampling method and the fractionator to generate an unbiased estimate of the numbers of osteoclasts within each tissue-culture well (Cruz-Orive and Weibel, 1990; Margulies et al., 2006). Osteoclasts were also used to detect SHIP expression.

### MicroCT

High-resolution images of the tibia were acquired by using a desktop microtomographic imaging system (MicroCT40; Scanco Medical, Basserdorf, Switzerland). The tibia was scanned at 45 keV with an isotropic voxel size of 6 µm, and the resulting 2-dimensional cross-sectional images are shown in gray scale. Proximal tibial scans were conducted to capture the same region of the metaphysis independent of gender or age, such that the number of slices selected for scanning began at the most proximal aspect of the tibia and extended a distance equal to 20% of the length of the tibia. In addition, a mid- shaft diaphyseal scan of 50 slices was conducted using the mid-point landmark tool present in the Scanco analysis software. In both cases a series of axial images was produced; however, proximal tibial images were re-sampled to be saggittal for analysis. Metaphyseal Bv/Tv, trabecular number (Tb.N) and trabecular thickness (Tb.Th) was calculated using the Scanco software, within a 50-slice region of interest centered on the central long-axis of the tibia and bounded by the growth plate at the proximal end and an arbitrary 1.5 mm point distal to the growth plate, with cortical bone carefully excluded. Metaphyseal thickness was determined using unbiased stereology, in which a 'thickness' was measured in individual sections spaced 5 µm apart through the primary and second spongiosa (Damron et al., 2003). Mid-shaft cortical Bv/Tv measurements were made using the Scanco software, while cortical thickness and cortical perimeter were determined using the BoneJ plugin for ImageJ.

### Image Segmentation and Nile Red Staining:

MicroCT images acquired at the proximal tibia were used to assay adipose tissue locally within the BM compartment. Adipose tissue derived the visceral fat compartment was scanned and the density values derived from these scans were then used to segment the adipose fraction in the proximal tibial BM compartment using the ImageJ plugin PhaseQuant (Judex et al., 2010). To directly assay the BM fat compartment, tibias were fixed using 10% neutral-buffered formalin, snap frozen and then sectioned at 10 µm. Sections were then stained for adiposity with 12 mg/ml of the lipo-philic fluorescent stain Nile Red (Sigma) and counterstained with 10 µg/ ml 4', 6-diamidino-2-phenylindole (DAPI, Sigma) for morphology.

### Hematological Analysis

Peripheral blood samples were harvested in EDTA coated microtubes (Sarstedt Co., Germany) by submandibular bleeding and analyzed with a HEMAVET 950S Veterinary Hematology Analyzer (Drew Scientific Inc.) using the mouse species program.

### Bone mass measurements by dual energy x-ray absorptiometry.

Total areal-body bone mineral density (BMD, g/cm2) was assessed with dual-energy Xray-absorptometry (DEXA) using a PixiMus scanner (GE Healthcare), according to previously published techniques (Margulies et al., 2003). Briefly, mice were anesthetized using 5% isoflurane and oxygen. BMD was determined using the PixiMus software by identifying a region of interest (excluding the head).

### TRAP staining of proximal tibia sections

Tibia were fixed under refrigeration and then decalcified using an EDTA-sucrose solution before being embedded in OCT compound and stored at -80°C prior to cryo-sectioning. To identify osteoclasts TRAP staining was performed using the Acid Phosphatase, Leukocyte Kit (Sigma, St. Louis, Missouri) and counter- stained using an acidified 1% methyl green.

### MSC immunophenotyping by flow cytometry

For FACS phenotyping of MSC bone marrow derived MSCs were seeded in quadruplet wells of a 60mm plate at 1 X 10⁵ cells/well. When the cells reached 80-90% confluency osteogenesis was induced by the addition of β-glycerol 2- phosphate (BGP, 10 mM; Sigma; Cat: G9891) and 2-phospho-1-ascorbic acid (50 µg/mL; Sigma; Cat: A8960) for 6 days, with osteogenic media changed every other day. Uninduced cells were seeded simultaneously and were used as controls. On day 6, cells were trypsinized and washed twice with staining buffer (3% heat-inactivated FBS, 2.5mM HEPES in IX PBS). 2.5 X 10⁶ cells were treated with CD16/CD32 mouse Fc block (2.4G2) on ice for 15 minutes and then stained with a panel of antibodies. The Lineage (Lin) panel included endothelial and hematopoietic markers on FITC; CD2 (RM2-5), CD3ε (145-2C11), CD4 (GK1.5), CD5 (53-7.3), CD8a (53-6.7), B220 (RA3-6B2), Gr-1 (RB6-8C5), Mac-1 (M1/70), NK1.1 (PK136), Ter119 (TER-119), CD31 (PECAM-1), CD34 (RAM34), CD45 (30-F11), CD86 (B7-2), MHC Class II (I-A/I-E) and CD43 (R2/60). MSC marker CD29 (Integrin beta 1, clone HMb1-1) was used to positively demarcate MSC with in the Lin- cell population. All antibodies were purchased from BD Biosciences and eBioscience (San Diego, CA).

### Annexin V staining

Apoptotic MSCs were evaluated on day 6 of osteogenic induction as described above. 2.5X10⁵ cells were treated with CD16/CD32 mouse Fc block (2.4G2) on ice for 15 minutes and stained with Lin panel and the MSC marker CD29 on ice for 30 mins. Cells were washed once with cold PBS and resuspended in IX binding buffer and stained with APC-labeled Annexin V for 15 mins at room temperature, protected from light. Cells were washed once and resuspended in 200 µL of 1X Binding Buffer. The frequency of Annexin V⁺ within the CD29⁺Lin⁻ MSC populations were detected and quantitated by flow cytometry.

### Use of SHIP1 inhibitor (3AC) for invivo studies

3AC was administered intraperitoneally at 25 mg/kg of body weight as published earlier (Fuhler et al., 2012). Vehicle treated mice received 100µL injection of 0.3% Klucel/H2O solution. 6-12 months old C57BL6/J mice were treated with 3AC or vehicle three times per week for 4, 8 and 16 weeks.

### Bone Mechanical Testing

Mouse femurs were cleaned, wrapped in PBS-soaked gauze and frozen at -20°C. Femurs were rehydrated in a PBS solution at room temperature for 2 hours before testing. We employed a 3-point-bending test to assay the biomechanical properties of femurs treated with the 3AC inhibitor. Femurs were placed into a testing frame (1250 N Load Cell and a Q Test 1/L, MTS Systems Corp, Eden Prairie, MN), pre-loaded with 1 N and then loaded at 1 N/mm until failure. Prior to testing femurs were scanned using microCT to assay the geometric properties.

### Statistical analysis

All statistical analyses were performed using the statistical software Prism (GraphPad, San Diego, CA). Body weight was assayed using ANCOVA while limb lengths, Bv/Tv, metaphyseal thickness, TRAP numbers, CFU-F measurements, BMD, %FAT (assayed with DEXA) and serum analysis of OPG were all analyzed using Student's t-test with a p<0.05. Linear Regression analyses were performed for mid-shaft cortical perimeter, thickness and BM fat content (volume fat). Ex vivo tissue culture differentiation experiments (osteogenesis, adipogenesis and osteoclastigenesis) were performed with three replicates in each experiment with each experiment performed a minimum of three times.

### References Cited in this Example

Citation of a reference herein shall not be construed as an admission that such reference is prior art to the present invention. Below is a listing of references cited herein with reference number indicators:
Barth, H.D., Zimmermann, E.A., Schaible, E., Tang, S.Y., Alliston, T., and Ritchie, R.O. (2011). Characterization of the effects of x-ray irradiation on the hierarchical structure and mechanical properties of human cortical bone. Biomaterials 32, 8892-8904.
Benezra, R., Davis, R.L., Lockshon, D., Turner, D.L., and Weintraub, H. (1990). The protein Id: a negative regulator of helix-loop-helix DNA binding proteins. Cell 61, 49-59.
Brooks, R., Fuhler, G.M., Iyer, S., Smith, M.J., Park, M.Y., Paraiso, K.H., Engelman, R.W., and Kerr, W.G. (2010). SHIP1 inhibition increases immunoregulatory capacity and triggers apoptosis of hematopoietic cancer cells. J Immunol 184, 3582-3589.
Collazo, M.M., Paraiso, K.H.T., Park, M.-Y., Hazen, A.L., and Kerr, W.G. (2012). Lineage extrinsic and intrinsic control of immunoregulatory cell numbers by SHIP. European Journal of Immunology 42, 1785-1795.
Cruz-Orive, L.M., and Weibel, E.R. (1990). Recent stereological methods for cell biology: a brief survey. The American journal of physiology 258, L148-156.
Dacquin, R., Starbuck, M., Schipke, T., and Karsenty, G. (2002). Mouse alphal(I)-collagen promoter is the best known promoter to drive efficient Cre recombinase expression in osteoblast. Dev Dyn 224, 245-251.
Damron, T.A., Margulies, B.S., Strauss, J.A., O'Hara, K., Spadaro, J.A., and Farnum, C.E. (2003). Sequential histomorphometric analysis of the growth plate following irradiation with and without radioprotection. J Bone Joint Surg Am 85-A, 1302-1313.
Desponts, C., Hazen, A.L., Paraiso, K.H., and Ken, W.G. (2006). SHIP deficiency enhances HSC proliferation and survival but compromises homing and repopulation. Blood 107, 4338-4345.
Ferron, M., Hinoi, E., Karsenty, G., and Ducy, P. (2008). Osteocalcin differentially regulates beta cell and adipocyte gene expression and affects the development of metabolic diseases in wild-type mice. Proceedings of the National Academy of Sciences of the United States of America 105, 5266-5270.
Ferron, M., Wei, J., Yoshizawa, T., Del Fattore, A., DePinho, R.A., Teti, A., Ducy, P., and Karsenty, G. (2010). Insulin signaling in osteoblasts integrates bone remodeling and energy metabolism. Cell 142, 296-308.
Franke, T.F., Kaplan, D.R., Cantley, L.C., and Toker, A. (1997). Direct regulation of the Akt proto-oncogene product by phosphatidylinositol-3,4-bisphosphate [see comments]. Science 275, 665-668. Fu, Y., Luo, N., Klein, R.L., and Garvey, W.T. (2005). Adiponectin promotes adipocyte differentiation, insulin sensitivity, and lipid accumulation. Journal of lipid research 46, 1369-1379.
Fuhler, G.M., Brooks, R., Toms, B., Iyer, S., Gengo, E.A., Park, M.Y., Gumbleton, M., Viernes, D.R., Chisholm, J.D., and Kerr, W.G. (2012). Therapeutic potential of SH2 domain-containing inositol-5'-phosphatase 1 (SHIP1) and SHIP2 inhibition in cancer. Mol Med 18, 65-75.
Hadjidakis, D.J., and Androulakis, I. (2006). Bone remodeling. Ann N Y Acad Sci 1092, 385-396.
Hazen, A.L., Smith, M.J., Desponts, C., Winter, O., Moser, K., and Kerr, W.G. (2009). SHIP is required for a functional hematopoietic stem cell niche. Blood 113, 2924-2933.
Helgason, C.D., Antonchuk, J., Bodner, C., and Humphries, R.K. (2003). Homeostasis and regeneration of the hematopoietic stem cell pool is altered in SHIP-deficient mice. Blood.
Hocking, L.J., Herbert, C.A., Nicholls, R.K., Williams, F., Bennett, S.T., Cundy, T., Nicholson, G.C., Wuyts, W., Van Hul, W., and Ralston, S.H. (2001). Genomewide search in familial Paget disease of bone shows evidence of genetic heterogeneity with candidate loci on chromosomes 2q36, 10p13, and 5q35. Am J Hum Genet 69, 1055-1061.
Jogi, A., Persson, P., Grynfeld, A., Pahlman, S., and Axelson, H. (2002). Modulation of basic helix-loop-helix transcription complex formation by Id proteins during neuronal differentiation. J Biol Chem 277, 9118-9126.
Judex, S., Luu, Y.K., Ozcivici, E., Adler, B., Lublinsky, S., and Rubin, C.T. (2010). Quantification of adiposity in small rodents using micro-CT. Methods 50, 14-19.
Kanazawa, I., Yamaguchi, T., Yamamoto, M., Yamauchi, M., Kurioka, S., Yano, S., and Sugimoto, T. (2009). Serum osteocalcin level is associated with glucose metabolism and atherosclerosis parameters in type 2 diabetes mellitus. The Journal of clinical endocrinology and metabolism 94, 45-49.
Karsenty, G., Kronenberg, H.M., and Settembre, C. (2009). Genetic control of bone formation. Annu Rev Cell Dev Biol 25, 629-648.
Kume, K., Satomura, K., Nishisho, S., Kitaoka, E., Yamanouchi, K., Tobiume, S., and Nagayama, M. (2002). Potential role of leptin in endochondral ossification. The journal of histochemistry and cytochemistry : official journal of the Histochemistry Society 50, 159-169.
Lasorella, A., Uo, T., and Iavarone, A. (2001). Id proteins at the cross-road of development and cancer. Oncogene 20, 8326-8333.
Lecka-Czernik, B. (2011). Marrow fat metabolism is linked to the systemic energy metabolism. Bone.
Liu, X., Bruxvoort, K.J., Zylstra, C.R., Liu, J., Cichowski, R., Faugere, M.C., Bouxsein, M.L., Wan, C., Williams, B.O., and Clemens, T.L. (2007). Lifelong accumulation of bone in mice lacking Pten in osteoblasts. Proc Natl Acad Sci U S A 104, 2259-2264.
Ma, K., Cheung, S.M., Marshall, A.J., and Duronio, V. (2008). PI(3,4,5)P3 and PI(3,4)P2 levels correlate with PKB/akt phosphorylation at Thr308 and Ser473, respectively; PI(3,4)P2 levels determine PKB activity. Cell Signal 20, 684-694.
MacDougald, O.A., and Burant, C.F. (2007). The rapidly expanding family of adipokines. Cell metabolism 6, 159-161.
Maor, G., Rochwerger, M., Segev, Y., and Phillip, M. (2002). Leptin acts as a growth factor on the chondrocytes of skeletal growth centers. Journal of bone and mineral research : the official journal of the American Society for Bone and Mineral Research 17, 1034-1043.
Margulies, B., Morgan, H., Allen, M., Strauss, J., Spadaro, J., and Damron, T. (2003). Transiently increased bone density after irradiation and the radioprotectant drug amifostine in a rat model. American journal of clinical oncology 26, e 106-114.
Margulies, B.S., Damron, T.A., and Allen, M.J. (2008). The differential effects of the radioprotectant drugs amifostine and sodium selenite treatment in combination with radiation therapy on constituent bone cells, Ewing's sarcoma of bone tumor cells, and rhabdomyosarcoma tumor cells in vitro. J Orthop Res 26, 1512-1519.
Margulies, B.S., Horton, J.A., Wang, Y., Damron, T.A., and Allen, M.J. (2006). Effects of radiation therapy on chondrocytes in vitro. Calcified tissue international 78, 302-313.
Massari, M.E., and Murre, C. (2000). Helix-loop-helix proteins: regulators of transcription in eucaryotic organisms. Mol Cell Biol 20, 429-440.
O'Toole, P.J., Inoue, T., Emerson, L., Morrison, I.E., Mackie, A.R., Cherry, R.J., and Norton, J.D. (2003). Id proteins negatively regulate basic helix-loop-helix transcription factor function by disrupting subnuclear compartmentalization. J Biol Chem 278, 45770-45776.
Pan, H., Ding, E., Hu, M., Lagoo, A.S., Datto, M.B., and Lagoo-Deenadayalan, S.A. (2010). SMAD4 is required for development of maximal endotoxin tolerance. J Immunol 184, 5502-5509.
Park, K.W., Waki, H., Villanueva, C.J., Monticelli, L.A., Hong, C., Kang, S., MacDougald, O.A., Goldrath, A.W., and Tontonoz, P. (2008). Inhibitor of DNA binding 2 is a small molecule-inducible modulator of peroxisome proliferator-activated receptor-gamma expression and adipocyte differentiation. Mol Endocrinol 22, 2038-2048.
Peng, Q., Malhotra, S., Torchia, J.A., Kerr, W.G., Coggeshall, K.M., and Humphrey, M.B. (2010). TREM2- and DAP12-dependent activation of PI3K requires DAP10 and is inhibited by SHIP1. Sci Signal 3, ra38.
Peng, Y., Kang, Q., Luo, Q., Jiang, W., Si, W., Liu, B.A., Luu, H.H., Park, J.K., Li, X., Luo, J., et al. (2004). Inhibitor of DNA Binding/Differentiation Helix-Loop-Helix Proteins Mediate Bone Morphogenetic Protein-induced Osteoblast Differentiation of Mesenchymal Stem Cells. Journal of Biological Chemistry 279, 32941-32949.
Rodda, S.J., and McMahon, A.P. (2006). Distinct roles for Hedgehog and canonical Wnt signaling in specification, differentiation and maintenance of osteoblast progenitors. Development 133, 3231-3244.
Takeshita, S., Namba, N., Zhao, J.J., Jiang, Y., Genant, H.K., Silva, M.J., Brodt, M.D., Helgason, C.D., Kalesnikoff, J., Rauh, M.J., et al. (2002). SHIP-deficient mice are severely osteoporotic due to increased numbers of hyper-resorptive osteoclasts. Nat Med 8, 943-949.
Tan, X., Weng, T., Zhang, J., Wang, J., Li, W., Wan, H., Lan, Y., Cheng, X., Hou, N., Liu, H., et al. (2007). Smad4 is required for maintaining normal murine postnatal bone homeostasis. J Cell Sci 120, 2162-2170.
Tanner, J.M. (1963). Regulation of Growth in Size in Mammals. Nature 199, 845-850.
Thomas, T., Gori, F., Khosla, S., Jensen, M.D., Burguera, B., and Riggs, B.L. (1999). Leptin acts on human marrow stromal cells to enhance differentiation to osteoblasts and to inhibit differentiation to adipocytes. Endocrinology 140, 1630-1638.
Wall, J.E., Kaste, S.C., Greenwald, C.A., Jenkins, J.J., Douglass, E.C., and Pratt, C.B. (1996). Fractures in children treated with radiotherapy for soft tissue sarcoma. Orthopedics 19, 657-664.
Wang, J.W., Howson, J.M., Ghansah, T., Desponts, C., Ninos, J.M., May, S.L., Nguyen, K.H., Toyama-Sorimachi, N., and Kerr, W.G. (2002). Influence of SHIP on the NK repertoire and allogeneic bone marrow transplantation. Science 295, 2094-2097.
Whyte, M.P., and Asbmr (2009). Chapter 88. Sclerosing Bone Disorders. In Primer on the Metabolic Bone Diseases and Disorders of Mineral Metabolism (John Wiley & Sons, Inc.), pp. 411-423.
Williams, S.A., Maecker, H.L., French, D.M., Liu, J., Gregg, A., Silverstein, L.B., Cao, T.C., Carano, R.A., and Dixit, V.M. (2011). USP1 deubiquitinates ID proteins to preserve a mesenchymal stem cell program in osteosarcoma. Cell 146, 918-930.
Yoshida, H., Hayashi, S., Kunisada, T., Ogawa, M., Nishikawa, S., Okamura, H., Sudo, T., and Shultz, L.D. (1990). The murine mutation osteopetrosis is in the coding region of the macrophage colony stimulating factor gene. Nature 345, 442-444.
Zebedee, Z., and Hara, E. (2001). Id proteins in cell cycle control and cellular senescence. Oncogene 20, 8317-8325.

### Example 20

### Synthesis of K118

This example describes the synthesis of the K118 compound.

### 3α-Aminocholestane hydrochloride (3AC):

This compound was synthesized from β-cholestanol following the method of Sugandhi (Sugandhi, E.W.; Slebodnick, C.; Falkinham, J.O.; Gandour, R.D. Synthesis and antimicrobial evaluation of water-soluble, dendritic derivatives of epimeric 5α-cholestan-3-amines and 5α-cholestan-3-yl aminoethanoates. Steroids, 2007, 72(8), 615-626). The hydrochloride salt was prepared from the free amine by dissolving the free amine in diethyl ether and bubbling HCl gas through the solution. The solid 3AC precipitated, was isolated by filtration and further purified by recrystallization from ethanol/water. Identity of the final product and all intermediates was established using ¹H NMR. Purity of the final product was verified using ¹H NMR, melting point and combustion analysis.

### 3β-Hydroxy-5α-androstane (K189):

This molecule was prepared from 3β-hydroxy-5α-androstan-17-one following the method of Norden (Norden, S.; Bender, M.; Rullkoetter, J.; Christoffers, J. Androstanes with Modified Carbon Skeletons. Eur. J. Org. Chem., 2011, 2011(24), 4543-4550). Identity of the final product was established using ¹H NMR. Purity of the final product was verified using ¹H NMR, melting point and combustion analysis.

### 3β-Amino-5α-androstane hydrochloride (K118):

K118 was prepared through the sequence shown in Scheme 1 below. First, K189 was converted to the 3α-iodide **1** using a Mitsunobu reaction. Displacement with sodium azide then proceeded with inversion to provide the β-azide **2.** Staudinger reduction of the azide followed by formation of the HCl salt then gave K118. Detailed experimental procedures follow. Identity of the intermediates and the final product was established using ¹H NMR. Purity of the final product was verified using ¹H NMR, ¹³C NMR and combustion analysis.

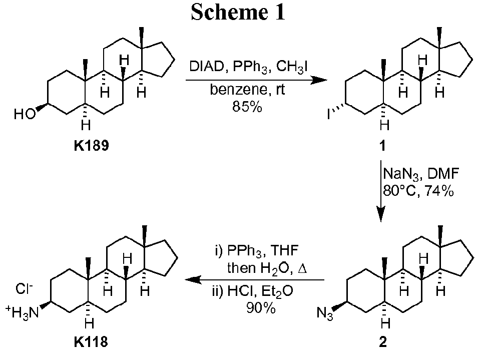

### 3α-Iodo-5α-androstane (1):

In a flame dried round bottom flask, **K189** (1.00 g, 3.62 mmol) and triphenylphosphine (1.138 g, 4.34 mmol) were dissolved in dry benzene (20 mL). A solution of DIAD (0.86 mL, 4.34 mmol) in dry benzene (8 mL) was added dropwise over several minutes followed by a solution of iodomethane (0.27 mL, 4.34 mmol) in dry benzene (8 mL). The resulting milky yellow solution was stirred continuously at rt. After approximately 24 h, the reaction mixture was concentrated and the residue was purified using flash column chromatography eluting with hexane, which afforded iodide **1** (1.194 g, 85%) as a white solid.

**1.** ¹H NMR (300 MHz, CDCl₃) δ 4.94 (p, *J* = 2.6 Hz, 1H), 1.91 (pd, *J* = 15.4, 3.3 Hz, 1H), 1.76-0.82 (m, 23H), 0.79 (s, 3H), 0.69 (s, 3H).

### 3β-Azido-5α-androstane (2):

In a flame dried flask, iodide **1** (1.483 g, 3.84 mmol) and sodium azide (2.496 g, 38.4 mmol) were suspended in dry DMF (20 mL). The suspension was heated to 80 °C. After 5 hours, the solution was allowed to cool to rt before quenching the reaction by adding water (200 mL). The quenched reaction mixture was then extracted with diethyl ether (3 x 200 mL). The organic layers were collected, combined, dried over magnesium sulfate, filtered, and concentrated under reduced pressure. Purification of the residue using silica gel column chromatography with hexane as the eluent afforded azide **2** (0.859 g, 74%) as white solid.

**2.** TLC *R_{f}* = 0.38 (hexanes); ¹H NMR (300 MHz, CDCl₃) δ 3.25 (dt, *J* = 12.9, 4.5 Hz, 1H), 1.87-0.83 (m, 23H), 0.80 (s, 3H), 0.70-0.61 (m, 1H), 0.68 (s, 3H).

### 3β-Amino-5α-androstane hydrochloride (K118):

In a flame dried flask, azide **2** (1.694 g, 5.62 mmol) and triphenylphosphine (2.938 g, 11.2 mmol) were dissolved in dry THF (26 mL). The solution was stirred continuously at rt. After approximately 2 hours, water (7 mL) was added and the solution was heated to reflux overnight. The reaction mixture was then cooled at rt. The organic layer was separated, dried over sodium sulfate, and concentrated under reduced pressure. Silica gel column chromatography (90:9:1 DCM: MeOH: NH₄OH as eluent) on the residue provided the free amine as a clear oil (1.456 g, 94%, ¹H NMR (300 MHz, CDCl₃) δ 2.74-2.61 (m, 1H), 1.79 (bs, 2H), 1.75-0.82 (m, 23H), 0.79 (s, 3H), 0.70-0.60 (m, 1H), 0.68 (s, 3H). The **β**-amine (1.456 g, 5.29 mmol) was dissolved in diethyl ether (10 mL). Anhydrous hydrogen chloride gas, generated by the reaction of sulfuric acid with sodium chloride in a separate flask, was bubbled into the diethyl ether solution, which resulted in precipitate formation. The suspension was filtered. The precipitate was collected and dried under vacuum to afford **K118** (1.589 g, 90%) as a white solid.

**K118.** m.p. = 276 °C (dec.); IR (KBr) 3449, 2928, 2361, 1451, 1377 cm⁻¹; ¹H NMR (300 MHz, CDCl₃) δ 8.29 (bs, 3H), 3.13 (bs, 1H), 1.99 (app d, *J* = 12.3 Hz, 1H), 1.83-0.81 (m, 22H), 0.84 (s, 3H) 0.68 (s, 3H), 0.72-0.61 (m, 1H). ¹³C NMR (75 MHz, CDCl₃) δ 54.7, 54.5, 51.5, 45.3, 41.0, 40.6, 39.0, 36.9, 35.9, 35.7, 33.3, 32.4, 28.5, 27.1, 25.7, 21.3, 20.7, 17.8, 12.5. Anal. calcd for C₁₉H₃₄ClN: C, 73.16; H, 10.99; N, 4.49. Found: C, 73.10; H, 10.59. N, 4.46.

### Example 21

### K118 as SHIP Inhibitor to Prevent or Reduce Obesity

Studies have been conducted that show compound K118, which is water-soluble, as reducing the accumulation of body fat without reducing bone density in aging mice. Studies have also been conducted that show compound K118 as preventing an increase in body fat content and weight in adult mice placed on a high caloric diet and that it does so without reducing bone density.

Conditional mutation of SHIP1 in mesenchymal stem cell compartment leads to decreased body fat as mice age. We had previously developed OsterixCreSHIP^{flox/flox} mice that early in life exhibit increased body fat due to bias of mesenchymal stem cell development toward adipogenesis (Iyer et al submitted). However, as these mice age % body fat goes through an inflection, such that % body fat returns to normal (∼8-10 weeks), and thereafter % body fat declines and is significantly lower than WT mice that actually accumulate increased body fat with age **(****Fig. 24****).** These genetic findings unequivocally implicate SHIP1 in control of adiposity and specifically in the accumulation of excess body fat.

We had previously described a small molecule inhibitor of SHIP1, 3AC **(****Fig. 25A****),** that we demonstrated could effectively target SHIP1 activity *in vivo.* ^{1,2} We have recently developed a water-soluble derivative of 3AC, K118 **(****Fig. 25B****),** that has comparable SHIP1 inhibitory activity as measured by phosphate release assay (Malachite Green). However, significant SHIP1 inhibitory activity is lost when the amine group (-NH2) of K118 is removed and replaced with a hydroxyl group to yield K189 **(****Fig. 25C****).** Importantly K118 is a water-soluble and thus represents a more attractive candidate for pharmacological targeting of SHIP1.

***SHIPi prevents or reverses accumulation of excess body fat during aging.*** We hypothesized then that treatment of adult or aging mice with a SHIP1 inhibitor, 3AC, might reduce body fat content. This was then tested by purchasing older mice that have begun to accumulate excessbody fat than their young adult counterparts and treating them three time per week with a SHIP1 selective inhibitor 3AC and then monitoring body fat content by whole body DEXA imaging. **(****Fig. 26****)** This analysis confirmed that pharmacological targetting of SHIP1 in vivo was a feasible approach to reverse the accumulation of excess body fat.

***K118 reduces the accumulation of body fat without reducing bone density in aging mice.*** As mice age they accumulate excess weight and body fat. This is particularly true in C57BL6 mice that are prone to obesity and diabetes. We hypothesized that like 3AC the aminosteroid K118, which also has significant SHIP1 inhibitory activity, might decrease age-associated body fat. Thus, we purchased 9-12 month old mice and began treating them two times per week with K118 (10mg/kg) by intraperitoneal injection for 4 weeks. In parallel, we treated mice with Vehicle (95% H2O: 5% DMSO) or a very similar steroid K189 that lacks the amine group. K189 does not have significant SHIP1 inhibitory activity. After one month we monitored body weight, % body fat and bone mineral density (BMD) in all groups. This analysis revealed that K118 was quite effective at reducing body fat and consequently body mass (weight) of aged mice **(****Fig. 27A, B****).** However, unlike our previous studies with 3AC we found that K118 does not significantly reduce BMD **(****Fig. 27C****).** As BMD is correlated with bone strength a reduction in BMD might reflect an adverse impact on bone integrity that could lead to spontaneous fractures. Thus, we conclude that K118 can prevent or reduce age-associated weight gain and obesity without reducing bone density.

***K118 prevents an increase in body fat content and weight in adult mice placed on a high caloric diet and does so without reducing bone density.*** To further test the ability as a potential therapeutic we wanted to determine if K118 could prevent or reverse obesity and weight gain in young adult C57BL6 mice placed on a *high fat diet* (60% fat diet) called "DIO mice" (Jackson laboratories). DIO mice are fed a diet that includes both high quantities of fat as well as sugar. It is known to induce both obesity and diabetes in C57BL6 mice. DIO mice were purchased from Jackson labs at 12 weeks of age. Mice were maintained on the high fat diet during treatment with K118 (2X/week, at the same dose as described above). After 1 month on the high fat diet we examined % body fat, **(****Fig. 28****)** weight, **(****Fig. 29****)** and BMD **(****Fig. 30****).** We found that K118 treated mice had significantly lower % body fat as compared to pre-treatment mice **(****Fig. 28A****)** while K189 and vehicle treated mice did not show a significant reduction of % body fat **(****Fig. 28B, C****)** as compared to pre-treatment mice. In addition, K118 treated mice did not significantly gain weight **(****Fig. 29A****),** while vehicle treated mice did have a significant weight gain over the 1 month treatment **(****Fig. 29C****).** Importantly, as we saw in the aged mouse study K118 did not adversely impact BMD of young mice on a high fat diet **(****Fig. 30****).** Analysis of weight **(****Fig. 31A****)** and % body fat **(****Fig. 31B**) in individual mice in the K118 treatment group showed that 3 out of 4 mice lost weight while all K118 treated mice on this diet exhibited reduced body fat during the one month course of treatment.

**Dosing and Delivery Information:** Suitable dosing and delivery information for embodiments of aging and DIO K118 obesity studies are described below. For example, dosing and delivery can be as follows: Two times per week by intraperitoneal injection for 4 weeks of K118 (10mg/kg), K189 (10mg/kg) and Vehicle (95% H2O: 5% DMSO). Please note that, in certain studies, K189 is soluble only in 95% H2O: 5% DMSO, unlike K118 that is water soluble. For this study, we prepared K118 and K189 in 95% H2O: 5% DMSO and hence used 95% H2O: 5% DMSO as the Vehicle control. Please note that, in certain embodiments, K118 could have been given in straight water but that in certain embodiments its inactive alcohol analog (K189) needed to be solubilized in 95% H20: 5% DMSO. K189 is not soluble in 100% H2O. In certain experiments, we felt it was necessary that, in order to fairly compare K118 to its inactive analog K189, we should make the delivery solutions identical for both.

### Example 22

### Accumulation of Excess Body Fat During Aging is Prevented by SHIP1 Deficiency or SHIPi

Studies have been conducted that show that SHIP inhibitors can be used in various methods, including, without limitation, the following: (i) a method to treat or prevent diabetes; (ii) a method to reduce glucose intolerance or insulin resistance; and (iii) a method to lower cholesterol.

In a particular study, compound K118 was found to reduce the accumulation of body fat without reducing bone density in aging mice. In another study, K118-mediated SHIPi was found to prevent diet-induced obesity and diabetes. In yet another study, K118 treatment was found to improve glucose tolerance in DIO mice. Data was also found to support the use of SHIP inhibitors to lower cholesterol.

We had previously developed OSXCreSHIP^{flox/flox} mice that early in life exhibit impaired bone development due to impaired mesenchymal stem cell development toward osteoblasts.¹ We noted also that as OSXCreSHIP^{flox/flox} mice age (3-12 months) their percent body fat declines and remains significantly lower than SHIP1-competent SHIPflox/flox controls that like typical C57BL/6 mice accumulate increased body fat with age **(****Fig. 32A****).** These genetic findings revealed an unanticipated and pivotal role for SHIP 1 in promoting accumulation of excess body fat during aging. We noted that mutation of the SHIP1 paralog, SHIP2, also promotes resistance to diet-induced obesity and diabetes.² We hypothesized then that treatment of adult or aging mice with a pan-SHIP1/2 inhibitor, 3AC^{3,4}, (SHIPi) might reduce body fat content. This was then tested by pulsatile SHIPi treatment of aged C57BL/6 mice that have begun to accumulate excess body fat and then assessing their body fat content via whole body DEXA imaging. **(****Fig. 32B****)** After 1 month treatment with 3AC we examined fasting blood glucose,**(****Fig. 32C****)** and insulin,**(****Fig**. **32D****),** we observed significant decrease in both blood glucose and insulin levels post treatment. This analysis confirmed that small molecule targetting of SHIP1/2 *in vivo* is a feasible approach to reverse the accumulation of excess body fat and glucose homeostais during aging.

### K118 reduces the accumulation of body fat without reducing bone density in aging mice.

We recently developed a 3AC analog, K118 **(****Fig. 33A****),** that is moderately SHIP1-selective but that targets SHIP2 comparable potency determined by a fluorescent polarization (FP) assay that measures SHIP1³ or SHIP2⁵ enzymatic activity **(****Fig. 33B****).** Importantly, K118 is water-soluble and thus represents a more attractive candidate for pharmacological targeting of SHIP1 vs. 3AC which has poor aqueous solubility.³ As C57BL/6 mice age they are prone to obesity and development of diabetes^{6,7}. We hypothesized that like 3AC,K118 might decrease age-associated weight and body fat increases in C57BL/6 mice. Thus, we performed pulsatile K118 treatment (2X/week; 10mg/kg) of aged male and female C57BL/6 mice (9-12m) for 1 month. During the treatment we monitored body weight and food intake and after treatment we then measured percent body fat, BMD and bone mineral content (BMC) in vehicle and K118 treated groups. This analysis revealed that K118 was quite effective at reducing body fat and consequently body mass (weight) in aging C57BL/6 mice of both sexes **(****Fig. 34A-F****).** Importantly, we observed no significant difference in food intake between the treatment groups in either gender **(****Fig. 34G-H****)** indicating K118 does not mediate this effect via appetite suppression. However, unlike our previous studies with 3AC we found that K118 does not significantly reduce BMD **(****Figure 37A****).** There are three major pathologies that limit the viability and health of germline SHIP1 mutant mice and adult MxCreSHIP^{flox/flox} mice with induced SHIP1 deficiency that include severe inflammation of the lungs^{3,8} and small intestine^{9,10} as well as osteoporosis.¹¹ We find that pulsatile K118 treatment for a one month period did not lead to any of these pathologies as K118-treated mice had normal bone density and mineral content **(****Fig. 38****)** and lacked any evidence of inflammation in the lungs or small intestine **(****Fig. 39****).** Moreover, no significant morbidity or mortality was observed in K118 treated mice. Thus, K118 can prevent or reduce age-associated weight gain and obesity without adversely impacting the health and viability of the host.

### K118-mediated SHIPi prevents diet-induced obesity and diabetes

To further test the ability as a potential therapeutic we wanted to determine if K118 could prevent or reverse obesity and weight gain in young adult C57BL6 mice (12-16 week old) placed on a *high caloric diet (HCD) that consists of* 60% fat/XX% sugar; henceforth, DIO mice.¹² C57BL6 mice maintained on a HCD uniformly develop both obesity and diabetes.^{12,13} DIO mice (3-4 months of age) that were maintained on a HCD since weaning were purchased from Jackson Laboratories and maintained on the HCD for the duration of the study. Hence study mice were obese prior to initiation of K118 treatment (2X/week, 10mg/kg). As before K118 treatment was performed for one month. After K118 or vehicle treatment we examined weight,**(****Fig. 35A-C****)** food intake,**(****Fig. 35D****)** gross comparison of fat depots,**(****Fig. 35E****)** % body fat,**(****Fig. 35F****)** BMD and BMC **(****Fig. 38A,B****).** We found that K118 treated mice lost a significant amount of body weight relative to both their pre-treatment weight **(****Fig. 35A, B****)** and the post-treatment vehicle group.**(****Fig. 35A,B****)** The vehicle group retained or increased their body mass during the one month study **(****Fig. 35C****).** The weight difference between the K118- and vehicle-treated mice was readily apparent upon visual examination of the mice **(****Fig. 35C****).** The weight loss could not be attributed to appetite suppression as there was no significant difference in food intake between the K118 and vehicle groups **(****Fig. 35D****).** K118-treatment also reduced leptin production in HCD mice and therefore the *leptin resistance* that occurs in HCD mice was also averted by K118 treatment¹⁴ **(****Figure 40****).** In addition, K118 treated mice had visibly less fat depot storage **(****Fig. 35E****)** significantly lower percent body fat as compared to pre-treatment and vehicle mice **(****Fig. 35F****)** while vehicle treated mice show a significant increase in percent body fat **(****Fig. 35F****)** as compared to pre-treatment mice, consistent with the well-documented response C57BL/6 mice to a HCD. Consistent with the reduced body fat we observed reduced total cholesterol levels in the serum of K118-treated HCD mice **(****Figure 40****).** Importantly, K118 did not adversely impact BMD or BMC in C57BL6 mice on a HCD.**(****Fig. 38A,B****)** Taken together, these data indicate that pan-SHIP1/2 inhibition by K118 is a potent anti-obesity agent that reverses body fat and weight gain while retaining consumption of a high caloric diet.

### K118 treatment improves glucose tolerance in DIO mice

Obesity is a complex metabolic disorder that predisposes individuals to several co-morbidities, such as type 2 diabetes mellitus. DIO mice are a well-studied model to of diet-related obesity and its leading co-morbidity, type 2 diabetes^{12,13}. In addition, DIO mice demonstrate insulin resistance, characterized by high plasma levels of insulin and glucose.^{12,13} Because K118 treatment substantially reduces fat accumulation and weight gain in DIO mice, we further investigated whether K118 treatment improves impaired glucose control that is observed in DIO mice. We measured fasting and *ad libitum* blood glucose and insulin levels and performed the intraperitoneal glucose tolerance test (IGTT) on fasted DIO mice. As described DIO mice were maintained on HCD during treatment with K118 (2X/week, 10mg/kg). After 1 month we examined fasting and *ad libitum* blood glucose,**(****Fig**. **36A-B****)** fasting and *ad libitum* insulin,**(****Fig. 36C-D****)** the fasting and *ad libitum* insulin:glucose ratio,**(****Fig**. **36E****)** and IGTT on fasted DIO mice **(****Fig. 36F****).** We found that K118 treated DIO mice significantly reduced blood glucose levels in comparison to pre-treatment and vehicle after one-month of K118 treatment. **(****Fig. 36A-B****).** In addition, K118 treated mice had significantly lower serum insulin levels as compared to vehicle mice **(****Fig. 36C-D****),** consistent with DIO phenotype. The insulin:glucose ratio is a clinical measure of insulin resistance, and studies by Winzell *et al* demonstrated a progressive increase in the insulin:glucose ratio in HCD fed C57BL/6 mice with age.¹³ We also observed a significant decrease the insulin:glucose ratio in K118 treated DIO mice in comparison to vehicle mice **(****Fig. 36E****),** suggesting that K118 treated DIO mice have greater insulin sensitivity. Importantly, we observed improved glucose tolerance in K118 treated mice in comparison to vehicle mice, as demonstrated by their response in the glucose tolerance test **(****Fig. 36F****).** These data indicate that pan-SHIP1/2 inhibition by K118 can significantly improve control of blood sugar and improve insulin sensitivity in obese individuals in spite of continued consumption of a high caloric diet.

### Example 23

### Oral Administration of K118

Studies have shown that K118 is bioavailable when delivered orally. In preliminary studies, K118 was shown to induce increases in immunoregulatory cells when delivered orally at 10mg/kg (see **Figure 41**)**.** A significant increase was observed in the frequency of myeloid derived suppressor cells (MDSC) expressing both Gr1 and Mac1 cell markers. This was observed in the spleen of treated mice. Significant increases were also observed in the frequency of "natural" Tregulatory cells (nTreg), characterized by expression of CD4⁺CD25⁺FoxP3⁺, in both the spleen and in the mesenteric lymph node (mLN). Finally, we observed a trend for increased neutrophil numbers, as is observed with intraperitonial injection of SHIP1 inhibitor 3AC.

### Example 24

### SHIP Inhibition Activity of Various Compounds

Various compounds were tested for SHIP inhibition activity in triplicate using standard procedures as described herein. Some of the compounds that were tested are as follows:

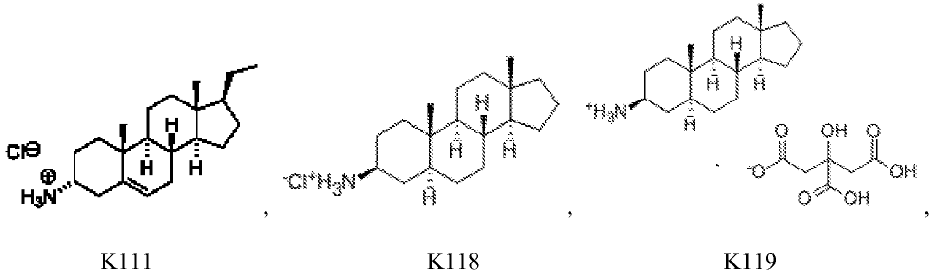

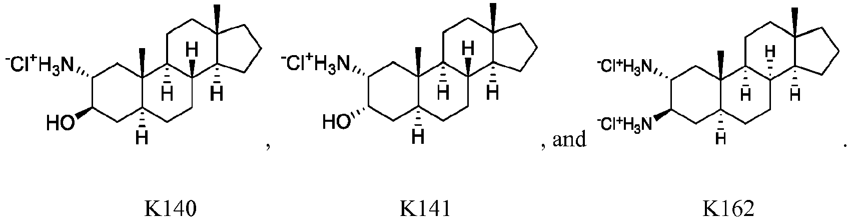

The standard protocol for measuring SHIP inhibition activity was conducted as described in the materials section of Brooks, R., Fuhler, G.M., Iyer, S., Smith, M.J., Park, M.Y., Paraiso, K.H., Engelman, R.W., and Kerr, W.G., "SHIP1 inhibition increases immunoregulatory capacity and triggers apoptosis of hematopoietic cancer cells," J Immunol 184:3582-3589 (2010), which protocol is described in pertinent part as follows: Malachite Green Assay was obtained from Echelon Biosciences. Malachite Green forms a colored complex with free phosphate liberated by the SHIP1, SHIP2, or PTEN enzyme reactions. The assay was performed according to manufacturer's protocol. The 3a-aminocholestane (3AC) was dissolved in 100% ethanol and used at a final concentration of 1 mM in the Malachite Green assay.

**Results:** K111 exhibited 67% inhibition of SHIP1 at ImM. K118 exhibited 59% inhibition of SHIP1 at ImM. K119 exhibited 61% inhibition of SHIP1 at 1mM. K140 exhibited 61% inhibition of SHIP1 at ImM. K141 exhibited 69% inhibition of SHIP1 at 1mM. K162 exhibited 56% inhibiton of SHIP1 at ImM.

### REFERENCES CITED

Citation of a reference herein shall not be construed as an admission that such reference is prior art to the present invention. Below is a listing of references cited herein with reference number indicators:
1. Wang JW, et al. (2002) Influence of SHIP on the NK repertoire and allogeneic bone marrow transplantation. (Translated from eng) Science 295(5562):2094-2097 (in eng).
2. Ghansah T, et al. (2004) Expansion of myeloid suppressor cells in SHIP-deficient mice represses allogeneic T cell responses. (Translated from eng) J Immunol 173(12):7324-7330 (in eng).
3. Wahle JA, et al. (2006) Cutting edge: dominance by an MHC-independent inhibitory receptor compromises NK killing of complex targets. (Translated from eng) J Immunol 176(12):7165-7169 (in eng).
4. Paraiso KH, Ghansah T, Costello A, Engelman RW, & Kerr WG (2007) Induced SHIP deficiency expands myeloid regulatory cells and abrogates graft-versus-host disease. (Translated from eng) J Immunol 178(5):2893-2900 (in eng).
5. Kerr WG (2008) A role for SHIP in stem cell biology and transplantation. Curr Stem Cell Res Ther 3(2):99-106.
6. Helgason CD, et al. (1998) Targeted disruption of SHIP leads to hemopoietic perturbations, lung pathology, and a shortened life span. Genes & Development 12(11):1610-1620.
7. Rauh MJ, et al. (2005) SHIP represses the generation of alternatively activated macrophages. Immunity 23(4):361-374.
8. Takeshita S, et al. (2002) SHIP-deficient mice are severely osteoporotic due to increased numbers of hyper-resorptive osteoclasts. Nat Med 8(9):943-949.
9. Franke TF, Kaplan DR, Cantley LC, & Toker A (1997) Direct regulation of the Akt proto-oncogene product by phosphatidylinositol-3,4-bisphosphate [see comments]. Science 275(5300):665-668.
10. Jain SK, et al. (1996) PI 3-kinase activation in BCR/abl-transformed hematopoietic cells does not require interaction of p85 SH2 domains with p210 BCR/abl. (Translated from eng) Blood 88(5):1542-1550 (in eng).
11.Ivetac I, et al. (2009) Regulation of PI(3)K/Akt signalling and cellular transformation by inositol polyphosphate 4-phosphatase-1. (Translated from eng) EMBO Rep 10(5):487-493 (in eng).

Although preferred embodiments have been depicted and described in detail herein, it will be apparent to those skilled in the relevant art that various modifications, additions, substitutions, and the like can be made within the scope of the invention as defined in the claims which follow.

## Claims

1. A pan-SHIP1/2 inhibitor for use in a method to treat obesity or to reduce body fat in an obese subject,
wherein the pan-SHIP1/2 inhibitor has the structure of: 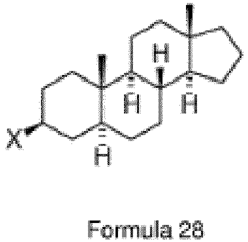 or is a
pharmaceutically acceptable salt thereof,
wherein X = NR₂, NRCONR₂, OR, SR, OCOR, OCONR₂, or NHCNHNH₂, preferably X = NH₂ or NH₃Cl, and wherein R = H, alkyl, cycloalkyl, aryl or benzyl, and
wherein the inhibitor reduces body fat in the obese subject.

2. A pan-SHIP1/2 inhibitor for use in treatment to reduce glucose intolerance or to decrease insulin resistance in a subject,
wherein the pan-SHIP1/2 inhibitor has the structure of: 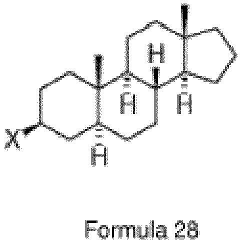 or is a
pharmaceutically acceptable salt thereof,
wherein X = NR₂, NRCONR₂, OR, SR, OCOR, OCONR₂, or NHCNHNH₂, preferably X = NH₂ or NH₃Cl, and wherein R = H, alkyl, cycloalkyl, aryl or benzyl, and
wherein the inhibitor reduces glucose intolerance or decreases insulin resistance when administered to the subject.

3. The pan-SHIP1/2 inhibitor for use according to claim 2,
wherein the pan-SHIP1/2 inhibitor reduces glucose intolerance
when administered to the subject.

4. The pan-SHIP1/2 inhibitor for use according to claim 2,
wherein the pan-SHIP1/2 inhibitor reduces insulin resistance
when administered to the subject.

5. The pan-SHIP1/2 inhibitor for use according to claim 1, wherein the pan-SHIP1/2 inhibitor does not negatively impact bone density when administered to the subject.

6. The pan-SHIP1/2 inhibitor for use according to any one of the preceding claims, wherein the pan-SHIP1/2 inhibitor has the structure of:

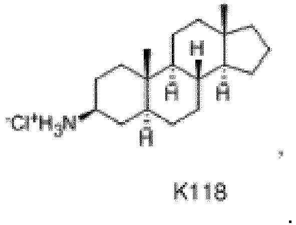

7. The pan-SHIP1/2 inhibitor for use according to any one of the preceding claims, wherein said pan-SHIP1/2 inhibitor is administered in a pulsatile manner.

8. The pan-SHIP1/2 inhibitor for use according to any of claims 1-5, wherein the inhibitor is administered in a continuous manner.

9. The pan-SHIP1/2 inhibitor for use according to any of claims 1-5, wherein the inhibitor is injected intraperitoneally preferably at about 25 mg/kg of body weight. or is administered orally to the subject.

10. The pan-SHIP1/2 inhibitor for use according to any of the preceding claims, wherein said pan-SHIP1/2 inhibitor compound or pharmaceutically acceptable salt thereof is comprised in a pharmaceutical composition.

## Patentansprüche

1. Ein Pan-SHIP-1/2-Hemmer zur Verwendung in einem Verfahren zum Behandeln von Fettleibigkeit oder zum Reduzieren von Körperfett bei einem fettleibigen Individuum, wobei der Pan-SHIP-1/2-Hemmer die folgende Struktur aufweist: 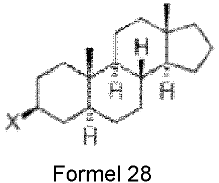 oder ein pharmazeutisch annehmbares Salz davon ist,
wobei X = NR₂, NRCONR₂, OR, SR, OCOR, OCONR₂ oder NHCNHNH₂, vorzugsweise X = NH₂ oder NH₃Cl und wobei R = H, Alkyl, Cycloalkyl, Aryl oder Benzyl und
wobei der Hemmer bei dem fettleibigen Individuum Körperfett reduziert.

2. Ein Pan-SHIP-1/2-Hemmer zur Verwendung bei der Behandlung zum Reduzieren von Glukoseintoleranz oder zum Vermindern von Insulinresistenz bei einem Individuum, wobei der Pan-SHIP-1/2-Hemmer die folgende Struktur aufweist: 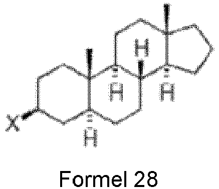 oder ein pharmazeutisch annehmbares Salz davon ist,
wobei X = NR₂, NRCONR₂, OR, SR, OCOR, OCONR₂ oder NHCNHNH₂, vorzugsweise X = NH₂ oder NH₃Cl und wobei R = H, Alkyl, Cycloalkyl, Aryl oder Benzyl und
wobei der Hemmer bei Verabreichen an das Individuum Glukoseintoleranz reduziert oder Insulinresistenz vermindert.

3. Pan-SHIP-1/2-Hemmer zur Verwendung gemäß Anspruch 2, wobei der Pan-SHIP-1/2-Hemmer bei Verabreichen an das Individuum Glukoseintoleranz reduziert.

4. Pan-SHIP-1/2-Hemmer zur Verwendung gemäß Anspruch 2, wobei der Pan-SHIP-1/2-Hemmer bei Verabreichen an das Individuum Insulinresistenz reduziert.

5. Pan-SHIP-1/2-Hemmer zur Verwendung gemäß Anspruch 1, wobei der Pan-SHIP-1/2-Hemmer bei Verabreichen an das Individuum die Knochendichte nicht negativ beeinflusst.

6. Pan-SHIP-1/2-Hemmer zur Verwendung gemäß einem der vorhergehenden Ansprüche, wobei der Pan-SHIP-1/2-Hemmer die folgende Struktur aufweist:

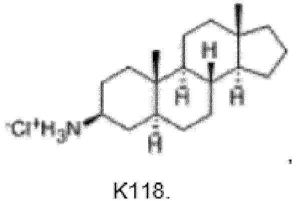

7. Pan-SHIP-1/2-Hemmer zur Verwendung gemäß einem der vorhergehenden Ansprüche, wobei der Pan-SHIP-1/2-Hemmer auf eine pulsierende Weise verabreicht wird.

8. Pan-SHIP-1/2-Hemmer zur Verwendung gemäß einem der Ansprüche 1-5, wobei der Hemmer auf eine kontinuierliche Weise verabreicht wird.

9. Pan-SHIP-1/2-Hemmer zur Verwendung gemäß einem der Ansprüche 1-5, wobei der Hemmer intraperitoneal injiziert wird, vorzugsweise zu ungefähr 25 mg/kg Körpergewicht, oder dem Individuum oral verabreicht wird.

10. Pan-SHIP-1/2-Hemmer zur Verwendung gemäß einem der vorhergehenden Ansprüche, wobei die Pan-SHIP-1/2-Hemmer-Verbindung oder das pharmazeutisch annehmbare Salz davon in einer pharmazeutischen Zusammensetzung beinhaltet ist.

## Revendications

1. Un inhibiteur de pan-SHIP1/2 pour une utilisation dans une méthode pour traiter l'obésité ou réduire la masse grasse corporelle chez un sujet obèse,
l'inhibiteur de pan-SHIP1/2 ayant la structure suivante : 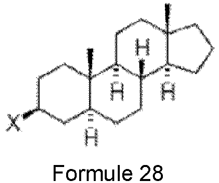 ou étant un sel pharmaceutiquement acceptable de celui-ci,
où X = NR₂, NRCONR₂, OR, SR, OCOR, OCONR₂, ou NHCNHNH₂, préférablement X = NH₂ ou NH₃Cl, et où R = H, un alkyle, un cycloalkyle, un aryle ou un benzyle, et l'inhibiteur réduisant la masse grasse corporelle de l'organisme chez le sujet obèse.

2. Un inhibiteur de pan-SHIP1/2 pour une utilisation dans un traitement pour réduire l'intolérance au glucose ou diminuer l'insulinorésistance chez un sujet,
l'inhibiteur de pan-SHIP1/2 ayant la structure suivante : 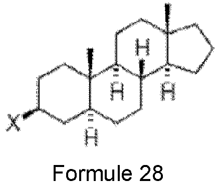 ou étant un sel pharmaceutiquement acceptable de celui-ci,
où X = NR₂, NRCONR₂, OR, SR, OCOR, OCONR₂, ou NHCNHNH₂, préférablement X = NH₂ ou NH₃Cl, et où R = H, un alkyle, un cycloalkyle, un aryle ou un benzyle, et l'inhibiteur réduisant l'intolérance au glucose ou diminuant l'insulinorésistance lorsqu'il est administré au sujet.

3. L'inhibiteur de pan-SHIP1/2 pour une utilisation selon la revendication 2, l'inhibiteur de pan-SHIP1/2 réduisant l'intolérance au glucose lorsqu'il est administré au sujet.

4. L'inhibiteur de pan-SHIP1/2 pour une utilisation selon la revendication 2, l'inhibiteur de pan-SHIP1/2 réduisant l'insulinorésistance lorsqu'il est administré au sujet.

5. L'inhibiteur de pan-SHIP1/2 pour une utilisation selon la revendication 1, l'inhibiteur de pan-SHIP1/2 n'ayant pas d'impact négatif sur la densité osseuse lorsqu'il est administré au sujet.

6. L'inhibiteur de pan-SHIP1/2 pour une utilisation selon l'une quelconque des revendications précédentes, l'inhibiteur de pan-SHIP1/2 ayant la structure de :

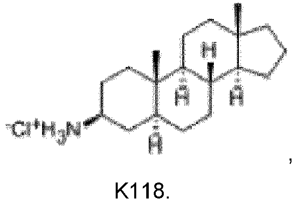

7. L'inhibiteur de pan-SHIP1/2 pour une utilisation selon l'une quelconque des revendications précédentes, ledit inhibiteur de pan-SHIP1/2 étant administré d'une manière pulsatile.

8. L'inhibiteur de pan-SHIP1/2 pour une utilisation selon n'importe lesquelles des revendications 1 à 5, l'inhibiteur étant administré d'une manière continue.

9. L'inhibiteur de pan-SHIP1/2 pour une utilisation selon n'importe lesquelles des revendications 1 à 5, l'inhibiteur étant injecté de façon intrapéritonéale préférablement à raison d'environ 25 mg/kg de poids corporel ou étant administré par voie orale au sujet.

10. L'inhibiteur de pan-SHIP1/2 pour une utilisation selon n'importe lesquelles des revendications précédentes, ledit composé inhibiteur de pan-SHIP1/2 ou sel pharmaceutiquement acceptable de celui-ci étant compris dans une composition pharmaceutique.
